# EUROPEAN PATENT APPLICATION

(11) **EP 2 774 532 A1**
(43) Date of publication of application: **10.09.2014**
(21) Application number: 14157589.4
(22) Date of filing: 04.03.2014
(51) Int. Cl.: A61B 5/00, A61B 5/02, A61B 5/021, A61B 5/11

(54) **Biological information detecting device, heart rate meter, and computer program**

(30) Priority: 06.03.2013 JP 2013044293; 06.03.2013 JP 2013044294; 28.03.2013 JP 2013068292
(71) Applicant: Seiko Epson Corporation, Shinjuku-ku, Tokyo 163-0811 (JP)
(72) Inventor: Kuroda, Masao, Nagano, 392-8502 (JP); Nagamine, Toshiyuki, Nagano, 392-8502 (JP); Miyoshi, Kazuhiro, Nagano, 392-8502 (JP); Yamazaki, Tatsuto, Nagano, 392-8502 (JP)
(74) Representative: HOFFMANN EITLE

(57) **Abstract**

A biological information detecting device includes a pulse-wave-information detecting section configured to detect pulse wave information of a test subject, a display section, and a processing section configured to perform measurement processing for the pulse wave information and discrimination processing for a posture state of the test subject. The display section displays a posture state notification image that dynamically changes according to a change in the posture state. The processing section performs processing for determining whether the posture state of the test subject is appropriate as a posture for performing the measurement processing for the pulse wave information, when determining that the posture state is appropriate, performs the measurement processing for the pulse wave information.

## Description

### BACKGROUND

### 1. Technical Field

The present invention relates to a biological information detecting device, a heart rate meter, a computer program, and the like.

### 2. Related Art

Electronic devices such as a biological information detecting device (in a narrow sense, a heart rate meter) have been widely used. The heart rate meter is a device for detecting a pulsation deriving from a heat beat of a human body. For example, the heart rate meter is a device for detecting a signal deriving from a heat beat on the basis of a signal output from a pulse wave sensor amounted on an arm, a palm, a finger, or the like.

For example, a photoelectric sensor is used as the pulse wave sensor. In this case, for example, a method of detecting, with the photoelectric sensor, reflected light or transmitted light of light irradiated on a living organism is conceivable. Since an absorption amount and a reflection amount of the irradiated light are different according to a blood flow in a blood vessel, sensor information (a pulse wave sensor signal) detected by the photoelectric sensor is a signal corresponding to the blood flow rate or the like. It is possible to acquire information concerning a pulsation by analyzing the signal.

For example, JP-A-2008-54890 (Patent Literature 1) presents a structure that can measure a contact pressure of a biological-information detecting section for detecting biological information such as a heart rate meter. Patent Literature 1 also presents a method of determining whether the contact pressure is a proper pressing force and displays the pressure as a graph to inform the user of the pressure. This is because it is known that the amplitude of a pulse signal is different according to a pressing force and, since the pulse signal decreases with an excessively large pressing force or an excessively small pressing force, it is necessary to set an appropriate pressing force in order to accurately perform processing based on the pulse signal (e.g., calculation of pulsation information).

However, it is known that a vital sign such as a pulsation changes according to a posture state of a user, who is a measurement target. For example, in the example of the pressing force, in order to obtain a proper pressing force state, not only an external pressure applied to a living organism from the outside but also an internal pressure, which is pressure inside a blood vessel, has to be taken into account. As the internal pressure, a water head pressure has large influence. When it is determined whether a pressing force is the proper pressing force, it is necessary to set the water head pressure in an appropriate state (an example of a specific state is explained below). That is, it is necessary to set the posture state of the user in an appropriate state. However, in Patent Literature 1, it is not taken into account that, for example, the appropriate posture state is clearly presented to the user.

This is not a problem limited to the pressing state. For example, when a posture changes, the water head pressure changes according to the posture change, an inertial force is generated by the swing of an arm or the like and the internal pressure fluctuates, and an active state of the heart fluctuates because an exercise load is applied to the user. That is, pulse wave information to be detected fluctuates according to the posture state. It is necessary to appropriately set the posture state in order to accurately detect the pulse wave information.

### SUMMARY

An advantage of some aspects of the invention is to provide a biological information detecting device, a computer program, and the like for accurately performing measurement processing for pulse wave information by displaying a posture state notification image.

An aspect of the invention is directed to a biological information detecting device including: a pulse-wave-information detecting section configured to detect pulse wave information of a test subject; a display section; and a processing section configured to perform measurement processing for the pulse wave information and discrimination processing for a posture state of the test subject. The display section displays a posture state notification image that dynamically changes according to a change in the posture state. The processing section performs processing for determining whether the posture state of the test subject is appropriate as a posture for performing the measurement processing for the pulse wave information and, when determining that the posture state is appropriate, performs the measurement processing for the pulse wave information.

In the aspect of the invention, the posture state notification image is displayed on the display section. When it is determined that the posture state is appropriate, the measurement processing for the pulse wave information is performed. Therefore, the present posture state is notified to a user in an intuitively understandable form. It is possible for the user to take the proper posture state with ease and further to improve accuracy of processing for performing the measurement processing in the appropriate posture state, for example.

In the aspect of the invention, the display section may display, as the posture state notification image, an image in which a display position of an object representing the posture state changes according to the posture state.

With this configuration, it is possible to, for example, display the posture state in association with the display position of the object.

In the aspect of the invention, when the processing section determines that the posture state is appropriate, the display section may display, as the posture state notification image, an image in which the object is displayed in a first region in the image.

With this configuration, it is possible to, for example, notify whether the posture state is appropriate according to whether the display position of the object is present in the first region. The first region is a region where the object is displayed when the posture state of the test subject is appropriate.

In the aspect of the invention, when the biological information detecting device is mounted on the test subject, a direction from the forearm to the hand of the test subject is represented as X axis, a direction orthogonal to the X axis and orthogonal to a screen of the display section is represented as Z axis, an axis orthogonal to the X axis and the Z axis is represented as Y axis, an angle representing rotation of the X axis around the Y axis during the discrimination processing with respect to a horizontal plane orthogonal to the gravity direction is represented as first angle θx, and an angle representing rotation of the Y axis around the X axis during the discrimination processing with respect to the horizontal plane is represented as second angle θy, the processing section may determine that the posture state is appropriate when it is determined that the first angle θx is within a first angle range and the second angle θy is within a second angle range.

With this configuration, it is possible to perform determination processing for the posture state on the basis of a rotation state of a given axis.

In the aspect of the invention, concerning the second angle θy, when an angle representing rotation on the gravity direction side with respect to the horizontal plane is set as a positive value and an angle representing rotation on the opposite side of the gravity direction is set as a negative value, the processing section may determine that the posture state is appropriate when it is determined that the second angle θy is within the second angle range in which a median is a negative value.

With this configuration, it is possible to, for example, improve possibility that the rotation at the second angle θy is in a negative direction and suppress a twist of the arm.

In the aspect of the invention, when the processing section determines that the posture state is appropriate, the display section may continuously display, as the posture state notification image, for a given wait time, an image in which the object is displayed in the first region.

With this configuration, it is possible to, for example, clearly notify the user that the present posture state is appropriate.

In the aspect of the invention, the display section may display, after the elapse of the wait time, a measurement state notification image representing that the processing section is performing the measurement processing for the pulse wave information.

With this configuration, it is possible to, for example, display an image corresponding to the measurement processing during the measurement processing.

In the aspect of the invention, when the processing section determines that the posture state is inappropriate during the display of the measurement state notification image after the elapse of the wait time, the processing section may end the measurement processing for the pulse wave information, and the display section may end the display of the measurement state notification image and perform the display of the posture state notification image.

With this configuration, it is possible to, for example, continue the determination processing for the posture state even after the start of the measurement processing and perform the determination processing for the posture state again when the posture state is inappropriate.

In the aspect of the invention, when the processing section determines that the posture state is inappropriate, the display section may display, as the posture state notification image, an image in which the object is displayed in a second region different from the first region in the image and display, as the posture state notification image, an image in which a display form of the object is different when the object is displayed in the first region and when the object is displayed in the second region.

With this configuration, it is possible to, for example, notify whether the posture state is appropriate according to a difference in a display region of the object and a difference in a display form of the object.

In the aspect of the invention, the biological information detecting device may include a load mechanism configured to generate a pressing force for pressing the pulse-wave-information detecting section against the test subject. A load state of the load mechanism may be set to any one of first to Nth (N is an integer equal to or larger than 2) load states in which states of the pressing force are different. The processing section may perform the discrimination processing for the posture state in an ith (i is an integer satisfying 1≤i≤N) load state of the load mechanism. The display section may display the posture state notification image that dynamically changes according to a change in the posture state in the ith load state.

With this configuration, it is possible to, for example, perform the discrimination processing for the posture state and the display of the posture state notification image in each of a plurality of load states.

In the aspect of the invention, when the processing section determines that the posture state is appropriate as a result of the discrimination processing, the processing section may perform the measurement processing for the pulse wave information in the ith load state, and the display section may display, after the end of the measurement processing in the processing section, an instruction image for performing an instruction to change the load mechanism to a jth (j is an integer satisfying 1≤j≤N, i:#j) load state different from the ith load state.

With this configuration, it is possible to, for example, urge a shift to another load state after the end of the measurement processing in a given load state and acquire a measurement results in the plurality of load states.

In the aspect of the invention, the processing section may perform, on the basis of results of the measurement processing for the pulse wave information acquired in at least two load states among the first to Nth load states, proper pressing force information acquisition processing for calculating proper pressing force information representing a proper value of the pressing force on the test subject.

With this configuration, it is possible to, for example, calculate a proper pressing force having a large individual difference from a measurement result of the pulse wave information.

Another aspect of the invention is directed to a biological information detecting device including: a pulse-wave-information detecting section configured to detect pulse wave information of a test subject; a presenting section configured to present information; and a processing section configured to perform measurement processing for the pulse wave information and discrimination processing for a posture state of the test subject. The presenting section presents the information concerning the posture state by taking a different presentation form according to a change in the posture state. The processing section performs processing for determining whether the posture state of the test subject is appropriate as a posture for performing the measurement processing for the pulse wave information and, when determining that the posture state is appropriate, performs the measurement processing for the pulse wave information.

Still another aspect of the invention is directed to a computer program for causing a computer to function as: a pulse-wave-information detecting section configured to acquire pulse wave information of a test subject; a processing section configured to perform measurement processing for the pulse wave information and discrimination processing for a posture state of the test subject; and a display control section configured to perform control for displaying, on a display section, a posture state notification image that dynamically changes according to a change in the posture state. The processing section performs processing for determining whether the posture state of the test subject is appropriate as a posture for performing the measurement processing for the pulse wave information and, when determining that the posture state is appropriate, performs the measurement processing for the pulse wave information.

Yet another aspect of the invention is directed to a biological information detecting device including: a pulse-wave-information detecting section configured to detect pulse wave information of a test subject; a processing section configured to perform measurement processing for a level of the pulse wave information at the time when a pressing force of the pulse-wave-information detecting section on the test subject is in each of a first pressing state to an Nth (N is an integer equal to or larger than 2); and a display section configured to display a first measurement result to an Mth (M is an integer satisfying M≤N) measurement result among results of the measurement processing for the level of the pulse wave information in the first pressing state to the Nth pressing state.

In the aspect of the invention, a plurality of measurement results corresponding to a plurality of pressing states are displayed. Therefore, when the pressing states are switched according to various causes such as determination of a proper pressing force, it is possible to, for example, present results of the switching of the pressing states to the user in a format in which the results can be easily viewed while comparing the results.

In the aspect of the invention, the display section may display the first measurement result to the Mth measurement result subjected to normalization processing for the level of the pulse wave information.

With this configuration, since the normalization processing for the level is performed, it is possible to, for example, appropriately display a displayable range and an actual measurement result in association with each other.

In the aspect of the invention, the display section may display, with a maximum value of the level among the first measurement result to the Mth measurement result, the first measurement result to the Mth measurement result subjected to the normalization processing.

With this configuration, it is possible to, for example, suppress all of a plurality of measurement results to be displayed from exceeding a displayable upper limit value and appropriately display the measurement results.

In the aspect of the invention, when results of the measurement processing for the level of the pulse wave information in the first pressing state to an ith (i is an integer satisfying 2≤i≤N) pressing state are acquired and results of the measurement processing for the level of the pulse wave information in an i+1th pressing state to the Nth pressing state are not acquired yet, the display section may perform the normalization processing with a maximum value of the level among the first measurement result to a jth (j is an integer satisfying j≤i) measurement result included in the results of the measurement processing in the first pressing state to the ith pressing state and display the first measurement result to the jth measurement result subjected to the normalization processing.

With this configuration, even when a measurement result is acquired only in a part of a plurality of pressing states, it is possible to, for example, appropriately display the acquired measurement result.

In the aspect of the invention, the display section may display an instruction screen for performing an instruction to change a pressing state to the i+1th pressing state and, when the processing section starts the measurement processing for the level of the pulse wave information in the i+1th pressing state, display a provisional measurement result of the level of the pulse wave information in the i+1th pressing state during the measurement processing together with the first measurement result to the jth measurement result.

With this configuration, it is possible to, for example, display a provisional value of pulse wave information currently being measured in addition to the acquired measurement result.

In the aspect of the invention, the display section may perform, as the display of the provisional measurement result, animation display that fluctuates in response to fluctuation in the pulse wave information in the i+1th pressing state.

With this configuration, it is possible to, for example, perform display corresponding to actually acquired pulse wave information as the display of the provisional measurement result.

In the aspect of the invention, the processing section may perform, in each of the first pressing state to the Nth pressing state, discrimination processing for determining whether the posture state of the test subject is appropriate as a posture for performing the measurement processing for the pulse wave information. The display section may display, in each of the first pressing state to the Nth pressing state, the posture state notification image that dynamically changes according to a change in the posture state.

With this configuration, the present posture state is notified to the user in an intuitively understandable form. Therefore, it is possible to, for example, cause the user to easily take an appropriate posture state and improve accuracy of processing for performing the measurement processing in the appropriate posture state.

In the aspect of the invention, the display section may display, as the posture state notification image, an image in which a display position of an object representing the posture state changes according to the posture state.

With this configuration, it is possible to, for example, display the posture state in association with the display position of the object.

In the aspect of the invention, when results of the measurement processing in the first pressing state to an ith (i is an integer satisfying 2≤i≤N) pressing state are acquired, results of the measurement processing for the level of the pulse wave information in an i+1th pressing state to the Nth pressing state are not acquired yet, and the processing section determines that the posture state is appropriate in the i+1th pressing state, the processing section may start the measurement processing for the level of the pulse wave information in the i+1th pressing state, and the display section may display the first measurement result to the jth measurement result included in results of the measurement processing for the level of the pulse wave information in the first pressing state to the ith pressing state and a provisional measurement result of the level of the pulse wave information in the i+1th pressing state during the measurement processing.

With this configuration, it is possible to, for example, perform the measurement processing after determining whether the posture state is appropriate in a given pressing state.

In the aspect of the invention, when the processing section determines that the posture state is appropriate in the i+1th pressing state, the display section may continuously display, as the posture state notification image, for a given wait time, an image in which the object is displayed in a first region in the image corresponding to a state in which the posture state is appropriate.

With this configuration, it is possible to, for example, notify the user whether the posture state is appropriate according to whether the display position of the object is present in the first region and, by setting the wait time, clearly notify the user that the present posture state is appropriate.

In the aspect of the invention, the display section may display, after the elapse of the wait time, the first measurement result to the jth measurement result and the provisional measurement result in the i+1th pressing state.

With this configuration, it is possible to, for example, display an image corresponding to the measurement processing during the measurement processing.

In the aspect of the invention, when the processing section acquires a result of the measurement processing in the i+1th pressing state, the display section may display an instruction screen for performing an instruction to change the pressing state to an i+2th pressing state. When the pressing state is changed to the i+2th pressing state according to the instruction screen, the display section may display the posture state notification image in the i+2th pressing state.

With this configuration, it is possible to, for example, urge a shift to another pressing state after the end of the measurement processing in a given pressing state and acquire measurement results in the plurality of pressing states. Further, it is possible to, for example, present determination processing results of the posture state in the pressing states to the user using the posture state notification image.

In the aspect of the invention, the processing section may perform, on the basis of results of the measurement processing of the pulse wave information acquired in at least two pressing states among the first pressing state to the Nth pressing state, proper pressing force information acquisition processing for calculating proper pressing force information representing a proper value of the pressing force on the test subject.

With this configuration, it is possible to, for example, calculate a proper pressing force having a large individual difference from a measurement result of the pulse wave information.

In the aspect of the invention, the first measurement result to the Mth measurement result may include at least a measurement result in which the level is the maximum among results of the measurement processing for the level of the pulse wave information in the first pressing state to the Nth pressing state.

With this configuration, it is possible to, for example, set a measurement result in which the level is the maximum among a plurality of measurement results as a display target.

Still yet another aspect of the invention is directed to a computer program for causing a computer to function as: a pulse-wave-information detecting section configured to acquire pulse wave information of a test subject; a processing section configured to perform measurement processing for a level of the pulse wave information at the time when a pressing force of the pulse-wave-information detecting section on the test subject is in each of a first pressing state to an Nth (N is an integer equal to or larger than 2) pressing state; and a display control section configured to perform control for displaying, on a display section, a first measurement result to a Mth (M is an integer satisfying M≤N) measurement result among results of the measurement processing for the level of the pulse wave information in the first pressing state to the Nth pressing state.

Further another aspect of the invention is directed to a heart rate meter including: a pulse-wave detecting section including a pulse wave sensor configured to output a pulse wave sensor signal; a processing section configured to calculate pulsation information on the basis of the signal output from the pulse-wave detecting section; a display section configured to display a processing result in the processing section; a storing section configured to store individual information of a test subject and the processing result in the processing section; and a holding mechanism for holding the heart rate meter on the test subject. The processing section estimates a holding state of the holding mechanism optimum for the test subject on the basis of the individual information of the test subject stored in the storing section and determines whether a pressing force on the test subject in the pulse-wave detecting section is a proper pressing force. The storing section stores holding state specifying information for specifying a holding state of the holding mechanism at the time when the processing section determines that the pressing force on the test subject in the pulse-wave detecting section is the proper pressing force. The processing section performs control for displaying the holding state specifying information on the display section.

In the aspect of the invention, a holding state of the holding mechanism optimum for the test subject is estimated. It is determined whether the pressing force on the test subject is the proper pressing force. Information for specifying the holding state of the holding mechanism at the time when the pressing force is the proper pressing force is stored and displayed as the holding state specifying information. Therefore, it is possible to determine the proper pressing force with the number of times of determination smaller than the number of times of determination for determining concerning all holding states whether the pressing force is the proper pressing force. The proper pressing force can be stored and displayed as the holding state rather than physical information such as a pressure value. Therefore, it is possible to present information concerning the proper pressing force in a form easy to understand for a user. Further, it is easy to, for example, reproduce the holding state for realizing the proper pressing force.

In the aspect of the invention, the processing section may determine whether the pressing force is the proper pressing force on the basis of the pulse wave sensor signal.

With this configuration, it is possible to perform proper pressing force determination and the like taking into account an individual difference of each of users.

In the aspect of the invention, the processing section may determine whether the pressing force is the proper pressing force on the basis of at least one of an AC component signal corresponding to an AC component of the pulse wave sensor signal and a DC component signal corresponding to a DC component of the pulse wave sensor signal.

With this configuration, it is possible to perform proper pressing force determination and the like using at least one of the AC component signal and the DC component signal.

Still further another aspect of the invention is directed to a heart rate meter including: a pulse-wave detecting section including a pulse wave sensor; a processing section configured to determine whether a pressing force on a test subject in the pulse-wave detecting section is a proper pressing force and calculate pulsation information of the test subject on the basis of a signal output from the pulse-wave detecting section; a display section configured to display a processing result in the processing section; and a storing section configured to store individual information of the test subject and the processing result in the processing section. The processing section estimates a holding state of a holding mechanism optimum for the test subject on the basis of the individual information of the test subject stored in the storing section and performs control for displaying, on the display section, an instruction screen for performing a setting instruction for an environment for determining whether the pressing force is the proper pressing force.

In the aspect of the invention, when the proper pressing force determination is performed, the instruction screen for performing the setting instruction for the environment for determination is displayed on the display section. Therefore, since it can be expected that an environment suitable for the proper pressing force determination is set, it is possible to, for example, improve determination accuracy.

Yet further another aspect of the invention is directed to a computer program for causing a computer to function as the sections explained above.

### BRIEF DESCRIPTION OF THE DRAWINGS

The invention will be described with reference to the accompanying drawings, wherein like numbers reference like elements.
Fig. 1 is a diagram showing a basic configuration example of a biological information detecting device in a first embodiment.
Fig. 2 is a diagram showing a configuration example of a body-motion-noise reducing section including an adaptive filter.
Figs. 3A to 3C are diagrams showing examples of a pulse wave detection signal, a body motion detection signal, a waveform of a signal after body motion noise reduction processing based on the pulse wave detection signal and the body motion detection signal, and a frequency spectrum.
Figs. 4A and 4B are diagrams showing examples of the biological information detecting device.
Fig. 5 is a diagram showing a detailed configuration example of the biological information detecting device in the first embodiment.
Fig. 6 is a relation diagram between a pressing force and a pulse wave detection signal (an AC component signal).
Fig. 7 is a diagram showing a structure example of the biological information detecting device.
Fig. 8 is a plan view showing an expanded state of an expandable section.
Fig. 9 is a plan view showing a twisted state of the expandable section.
Fig. 10 is a perspective view showing a coupling member.
Fig. 11 is a perspective view showing the coupling member.
Fig. 12 is an exploded perspective view showing the coupling member.
Fig. 13 is another perspective view of the coupling member.
Figs. 14A and 14B are diagrams showing a structure example and a moving state of positioning dies.
Figs. 15A and 15B are diagrams showing a structure example of a pulse-wave-information detecting section.
Fig. 16 is a diagram showing a system configuration example in which processing is performed in another electronic device or the like.
Figs. 17A and 17B are diagrams showing examples of a posture state notification image.
Figs. 18A to 18C are explanatory diagrams of a coordinate system and angles used in discrimination processing for a posture state.
Figs. 19A and 19B are diagrams for explaining an appropriate posture state.
Figs. 20A to 20E are diagrams showing examples of a normalized graph (a measurement state notification image).
Fig. 21 is a diagram showing an example of a measurement state notification image including a provisional result.
Fig. 22 is a diagram showing an example of an instruction screen for instructing a change in a load state.
Fig. 23 is a diagram showing an example of an image for notifying a result of measurement processing.
Fig. 24 is a diagram showing a screen transition example of a display image on a display section.
Figs. 25A and 25B are relation diagrams between a pressing force and an AC component signal.
Fig. 26A is a diagram showing an example of a signal value of the AC component signal at a given pressing force.
Fig. 26B is a diagram showing an example of an amplitude value of the AC component signal at the given pressing force.
Fig. 27 is a flowchart for explaining processing in the first embodiment.
Fig. 28 is a flowchart for explaining calculation processing for a pulse amplitude value.
Fig. 29 is a diagram showing a configuration example of a band, which is a holding mechanism.
Fig. 30 is a relation diagram between a pressing force and a DC component signal.
Fig. 31 is a diagram showing an example of a signal value of the DC component signal at a given pressing force.
Fig. 32 is a flowchart for explaining proper pressing force determination processing in which both of the AC component signal and the DC component signal are used.
Fig. 33 is a flowchart for explaining processing in the embodiment including display control and the like.
Figs. 34A to 34C are diagrams showing screen examples displayed on the display section.
Figs. 35A and 35B are diagrams showing examples of acceleration detection values used for stability determination for a body motion.
Fig. 35C is a diagram showing a setting example of axes of an acceleration sensor.
Fig. 36 is a flowchart for explaining stability determination processing for a body motion.
Figs. 37A and 37B are diagrams showing examples of acceleration detection values used for posture determination.
Fig. 38 is a flowchart for explaining posture determination processing.
Figs. 39A to 39C are diagrams for explaining a difference between change characteristics of the AC component signal and the DC component signal in a pressurization process and a depressurization process.
Fig. 40 is a diagram showing a correlation between personal information (BMI, sex, and age) of a user and band hole positions.
Fig. 41 is a diagram showing an example of deviation (dispersion) between estimated band hole positions and actual measurement.
Fig. 42 is a flowchart for explaining processing of initial holding state estimation.

### DESCRIPTION OF EXEMPLARY EMBODIMENTS

A first embodiment is explained below. The first embodiment explained below does not unduly limit contents of the invention described in the appended claims. Not all components explained in the first embodiment are always essential constituent features of the invention.

### First Embodiment

### 1. Configuration example of a biological information detecting device

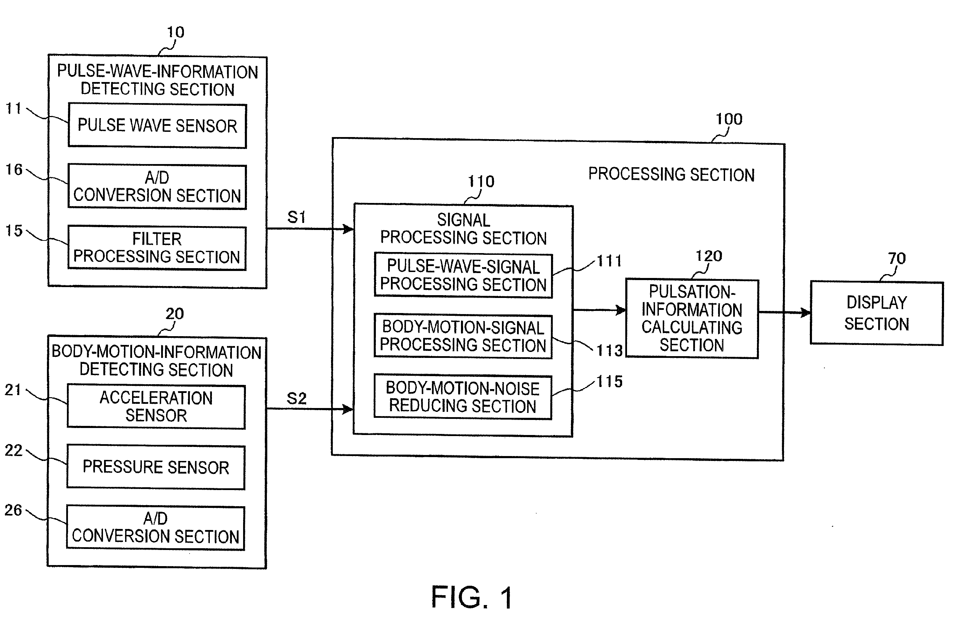

First, a basic configuration example of a biological information detecting device (in a broad sense, an electronic device) in a first embodiment is explained with reference to Fig. 1. Fig. 1 shows an example of the biological information detecting device. Components included in the biological information detecting device in the first embodiment are sometimes simplified or omitted. Components not essential in the biological information detecting device in the first embodiment are sometimes included.

As shown in Fig. 1, the biological information detecting device in the first embodiment includes a pulse-wave-information detecting section 10, a body-motion-information detecting section 20, a processing section 100, and a display section 70. However, the biological information detecting device is not limited to the configuration shown in Fig. 1. Various modifications are possible to, for example, omit or change a part of the components and add other components.

The pulse-wave-information detecting section 10 outputs a signal on the basis of sensor information (a pulse wave sensor signal and pulse wave information) of a pulse wave sensor 11. The pulse-wave-information detecting section 10 can include, for example, the pulse wave sensor 11, a filter processing section 15, and an A/D conversion section 16. However, the pulse-wave-information detecting section 10 is not limited to the configuration shown in Fig. 1. Various modifications are possible to, for example, omit a part of the components and add other components (e.g., an amplifying section configured to amplify a signal).

The pulse wave sensor 11 is a sensor for detecting a pulse wave signal. For example, a photoelectric sensor is conceivable as the pulse wave sensor 11. When the photoelectric sensor is used as the pulse wave sensor 11, a sensor configured to cut a signal component of external light such as the sunlight may be used. This can be realized by, for example, a configuration for providing a plurality of photodiodes and calculating difference information in feedback processing using signals of the photodiodes.

The pulse wave sensor 11 is not limited to the photoelectric sensor and may be a sensor that uses ultrasound. In this case, the pulse wave sensor 11 includes two piezoelectric elements. The pulse wave sensor 11 excites one piezoelectric element to transmit ultrasound into a living organism and receives, with the other piezoelectric element, the ultrasound reflected by a blood flow of the living organism. A frequency change occurs between the transmitted ultrasound and the received ultrasound according to the Doppler effect of the blood flow. Therefore, in this case, it is possible to acquire a signal corresponding to a blood flow rate and estimate pulsation information. Other sensors may be used as the pulse wave sensor 11.

The filter processing section 15 applies high-pass filter processing to sensor information output from the pulse wave sensor 11. A cutoff frequency of a high-pass filter may be calculated from a typical pulse count. For example, it is extremely rare that the pulse count of an ordinary person is smaller than 30 per minute. That is, it is rare that the frequency of a signal deriving from a heartbeat is equal to or lower than 0.5 Hz. Therefore, even if information concerning a frequency band in this range is cut, an adverse effect on a signal desired to be acquired should be small. Therefore, about 0.5 Hz may be set as the cutoff frequency. Depending on a situation, a different cutoff frequency such as 1 Hz may be set. Further, it is also possible to assume a typical upper limit value depending on the pulse count of a person. Therefore, the filter processing section 15 may perform band-pass filter processing rather than the high-pass filter processing. A cutoff frequency on the high-frequency side can also be set freely to some extent. However, a value such as 4 Hz only has to be used.

The A/D conversion section 16 performs A/D conversion processing and outputs a digital signal. The processing in the filter processing section 15 may be analog filter processing performed before the A/D conversion processing or may be digital filter processing performed after the A/D conversion processing.

The body-motion-information detecting section 20 outputs a signal (a body motion detection signal) corresponding to a body motion on the basis of sensor information of various sensors. The body-motion-information detecting section 20 can include, for example, an acceleration sensor 21, a pressure sensor 22, and an A/D conversion section 26. However, the body-motion-information detecting section 20 may include other sensors (e.g., a gyro sensor) and an amplifying section configured to amplify a signal. It is unnecessary to provide a plurality of kinds of sensors. The body-motion-information detecting section 20 may include one kind of a sensor.

The processing section 100 includes a signal processing section 110 and a pulsation-information calculating section 120. However, the processing section 100 is not limited to the configuration shown in Fig. 1. Various modifications are possible to, for example, omit a part of the components and add other components. The signal processing section 110 applies signal processing to an output signal output from the pulse-wave-information detecting section 10 and an output signal output from the body-motion-information detecting section 20.

The signal processing section 110 can include the pulse-wave-signal processing section 111, a body-motion-signal processing section 113, and a body-motion-noise reducing section 115.

The pulse-wave-signal processing section 111 applies some signal processing to a signal output from the pulse-wave-information detecting section 10. As an output from the pulse-wave-information detecting section 10 indicated by S1 in Fig. 1, various signals based on a pulse wave sensor signal are conceivable. For example, calculation of pulsation information explained below is often performed on the basis of a pulse wave sensor signal after DC component cut (hereinafter also referred to as pulse wave detection signal; in the following explanation, a signal equivalent to the signal is represented as AC component signal). Therefore, it is surmised that a pulse wave sensor signal after the high-pass filter processing is included in S1. However, a signal not subjected to the high-pass filter may be output. In some case, a pulse wave sensor signal after the low-pass filter processing may be output. When a plurality of signals (e.g., the pulse wave sensor signal before the high-pass filter processing and the pulse wave sensor signal after the processing) are included in S1, the processing in the pulse-wave-signal processing section 111 may be applied to all the signals included in S1 or may be applied to a part of the signals. Various processing contents are also conceivable. For example, the processing may be equalizer processing for a pulse wave detection signal or may be other processing.

The body-motion-signal processing section 113 applies various kinds of signal processing to a body motion detection signal output from the body-motion-information detecting section 20. Like S1, various signals are conceivable as an output of the body-motion-information detecting section 20 indicated by S2. For example, in the example shown in Fig. 1, the body-motion-information detecting section 20 includes the acceleration sensor 21 and the pressure sensor 22. Therefore, the body motion detection signal of S2 includes an acceleration signal and a pressure signal. As the sensor for body motion detection, other sensors such as a gyro sensor can also be used. Therefore, an output signal of a type corresponding to a type of a sensor is included in S2. The processing in the body-motion-signal processing section 113 may be applied to all the signals included in S2 or may be applied to a part of the signals. For example, processing for performing comparison processing for the signals included in S2 and determining a signal used in noise reduction processing in the body-motion-noise reducing section 115 may be performed.

In the processing in the pulse-wave-signal processing section 111, the body motion detection signal may be used together with the signal output from the pulse-wave-information detecting section 10. Similarly, in the processing in the body-motion-signal processing section 113, the signal output from the pulse-wave-information detecting section 10 may be used together with the body motion detection signal.

A signal obtained by applying, in the pulse-wave-signal processing section 111, given processing to the output signal output from the pulse-wave-information detecting section 10 may be used for the processing in the body-motion-signal processing section 113 or vice versa.

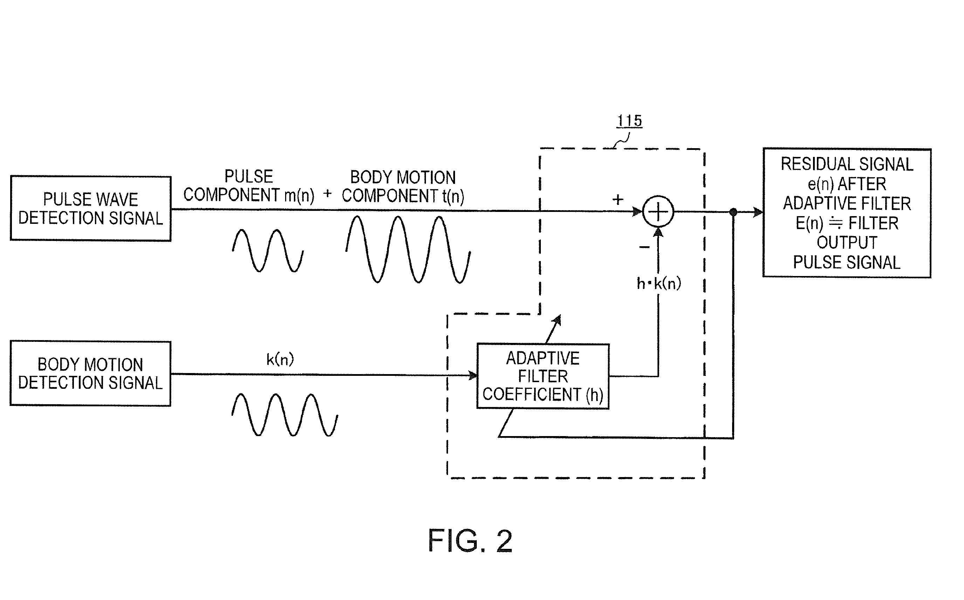

The body-motion-noise reducing section 115 performs, using the body motion detection signal, processing for reducing noise due to a body motion (body motion noise) from the pulse wave detection signal. A specific example of the noise reduction processing performed using an adaptive filter is shown in Fig. 2. The pulse wave sensor signal acquired from the pulse wave sensor 11 includes a component due to a body motion besides a component due to a heartbeat. This is the same in the pulse wave detection signal (the pulse wave sensor signal after the DC component cut) used for calculation of pulsation information. The component due to the heartbeat is useful for the calculation of pulsation information. The component due to the body motion hinders the calculation. Therefore, the signal due to the body motion (the body motion detection signal) is acquired using a body motion sensor and a signal component (referred to as estimated body motion noise component) having a correlation with the body motion detection signal is removed from the pulse wave detection signal to reduce body motion noise included in the pulse wave detection signal. However, even if both of the body motion noise in the pulse wave detection signal and the body motion detection signal output from the body motion sensor are signals due to the same body motion, signal levels of the signals are not always the same. Therefore, filter processing, a filter coefficient of which is adaptively determined, is applied to the body motion detection signal to calculate an estimated body motion noise component and calculate a difference between the pulse wave detection signal and the estimated body motion noise component.

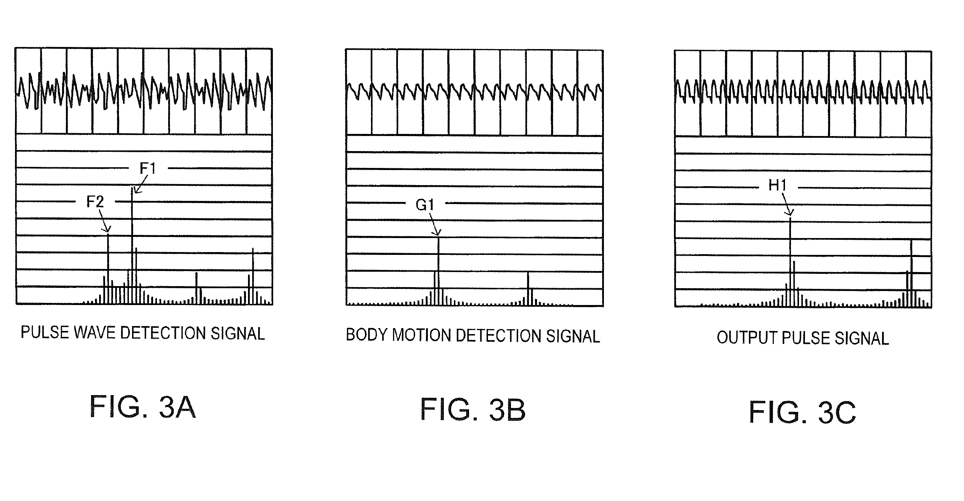

The processing explained above is explained in Figs. 3A to 3C using a frequency spectrum. In Fig. 3A and the like, a temporal change waveform of a signal is shown in an upper part and a frequency spectrum of the temporal change waveform is shown in a lower part. Fig. 3A represents the pulse wave detection signal before the body motion noise reduction. As indicated by F1 and F2, two frequencies having large values appear in the spectrum. One of the frequencies is due to the heartbeat and the other is due to the body motion. Although some frequencies higher than F1 have large values, since the frequencies are high-frequency components equivalent to integer times of F1 and F2, the frequencies are not taken into account. Although high-frequency components are also seen in Figs. 3B and 3C, the high-frequency components are not taken into account either.

On the other hand, Fig. 3B represents the body motion detection signal. If only one kind of a body motion is a cause of the body motion detection signal, one frequency having a large value as indicated by G1 appears. The frequency of G1 corresponds to F2 shown in Fig. 3A. In such a case, a signal shown in Fig. 3C is obtained by calculating a difference between the pulse wave detection signal and the estimated body motion noise component using the method shown in Fig. 2. As it is evident from the figure, the estimated body motion noise component having the peak G1 due to the body motion is subtracted from the pulse wave detection signal having the two peaks F1 and F2 due to the heartbeat and the body motion, whereby a body motion component (corresponding to F2) in the pulse wave detection signal is removed. As a result, a peak H1 (the frequency of which corresponds to F1) due to the heartbeat remains.

In a situation in which it is guaranteed that, for example, the body noise included in the pulse wave detection signal and the body motion detection signal correspond to each other and a signal component adversely affecting the noise reduction processing is not included in the body motion detection signal, it is unnecessary to perform a frequency analysis in the body-motion-noise reducing section 115. Therefore, frequency spectra shown in lower parts of Figs. 3A and 3B do not have to be taken into account. However, depending on a type or the like of a sensor used for acquisition of the body motion detection signal, the condition explained above is not satisfied in some case. In that case, for example, the body-motion-signal processing section 113 may process the body motion detection signal to satisfy the condition or exclude the body motion detection signal not satisfying the condition from the output to the body-motion-noise reducing section 115 or the like. Various methods are conceivable as a method of determining whether the body motion detection signal satisfies the condition. However, the frequency spectra shown in the lower parts of Figs. 3A and 3B obtain by, for example, a frequency analysis may be used.

The pulsation-information calculating section 120 calculates pulsation information on the basis of the input signal. The pulsation information may be, for example, a value of a pulse count. For example, the pulsation-information calculating section 120 may perform processing for applying a frequency analysis such as FFT to the pulse wave detection signal after the noise reduction processing in the body-motion-noise reducing section 115 to calculate a spectrum and setting a representative frequency in the calculated spectrum as the frequency of a heartbeat. In that case, a value obtained by multiplying the calculated frequency by 60 is a pulse count (a heart rate) used in general.

The pulsation information is not limited to the pulse count and may be, for example, information representing the pulse count (the frequency, the cycle, or the like of the heartbeat). The pulsation information may be information representing a state of a pulsation. For example, a value representing a blood flow rate itself (or fluctuation in the blood flow rate) may be set as the pulsation information. However, there is an individual difference of each of the users in a relation between the blood flow rate and a signal value of the pulse wave sensor signal. Therefore, when the blood flow rate or the like is set as the pulsation information, it is desirable to perform correction processing for dealing with the individual difference.

Timing when a given value (an upper peak, a lower peak, a value equal to or larger than a given threshold, etc.) may be detected on a temporal change waveform of the input pulse wave detection signal. The cycle of the heartbeat may be calculated from time equivalent to an interval of the timing to calculate the pulsation information. Alternatively, the pulsation information can also be calculated by transforming the waveform of the pulse wave detection signal into a rectangular wave and using a rising edge or the like of the rectangular wave. This method is advantageous in terms of computational complexity and power consumption because the frequency analysis does not have to be performed. However, in this method, since the signal value is directly used without being converted into a frequency axis, a waveform needs to be in good order to some extent. Therefore, it is desirable to perform the frequency analysis, for example, in a situation with much noise.

The display section 70 (in a broad sense, an output section) is a section for displaying various display screens used for presentation of the calculated pulsation information or the like. The display section 70 can be realized by, for example, a liquid crystal display or an organic EL display.

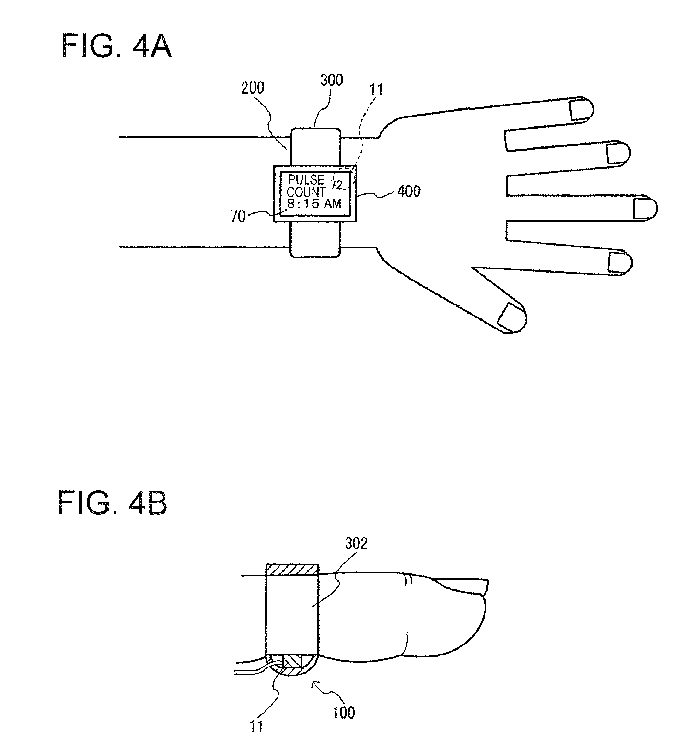

Specific examples of the biological information detecting device are shown in Figs. 4A and 4B. Fig. 4A is an example of a heart rate meter of a watch type. A base section 400 including the pulse wave sensor 11 and the display section 70 is mounted on a left wrist 200 of a test subject (the user) by a loading mechanism (a holding mechanism) 300 (e.g., a band). Fig. 4B is an example of a finger mounted type. The pulse wave sensor 11 is provided in the bottom of a ring-like guide 302 for inserting in the fingertip of the test subject. However, in the biological information detecting device shown in Fig. 4B, since there is no spatial margin for providing the display section 70, it is assumed that the display section 70 is provided (and a portion equivalent to the processing section 100 is provided according to necessity), for example, on the other end side of a wire cable connected to the pulse wave sensor 11. A detailed configuration example including the load mechanism (the band) 300 is explained below. In this embodiment, a force for fixing a device main body 2 to the body of the user is referred to as "load" or "pressing force". In a strict sense, the "load" means a force applied to the device main body 2 by the load mechanism (the band) 300 and the "pressing force" means a force generated in a portion where the sensor and the skin are actually in contact. However, both of the "load" and the "pressing force" may be interpreted as being intended to indicate the same force.

### 2. Method in the first embodiment

A method in the first embodiment is explained below. In the following explanation, processing for calculating a proper pressing force is explained as an example. However, the method in the first embodiment (e.g., display of a posture state notification image) can be applied to processing other than the determination of the proper pressing force.

By using the pulse wave sensor 11 such as the photoelectric sensor as explained above, it is possible to acquire a pulse wave sensor signal corresponding to a blood circulation state (e.g., a blood flow rate). However, as it is easily understood from the fact that, when the arm or the like is strongly compressed, the blood flow rate decreases in a region further on the distal side than the compressed portion, the blood flow rate changes according to an external pressure on a living organism (in a narrow sense, a blood vessel). That is, if the external pressure is excessively strong, a signal value of the pulse wave sensor signal is reduced and the influence of noise relatively increases (an SN state is deteriorated). Therefore, subsequent processing is hindered (e.g., accuracy of pulsation information based on the pulse wave sensor signal) is deteriorated.

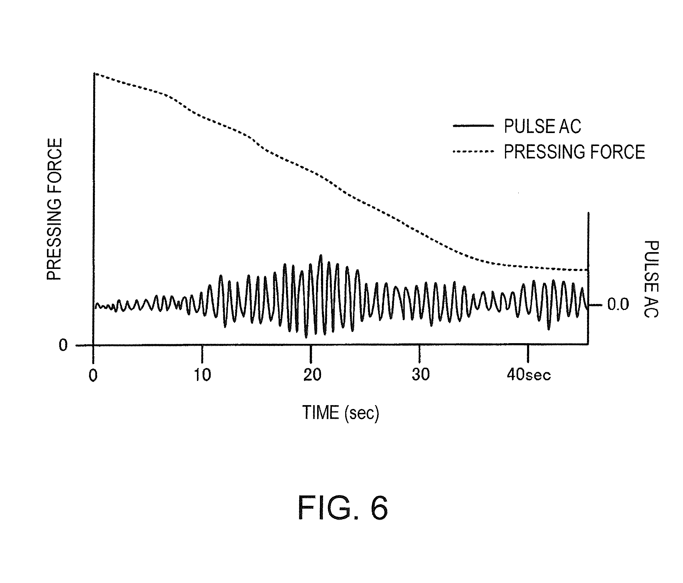

An excessively small external pressure is undesirable as well because the signal value of the pulsation sensor signal decreases. As one of causes of the decrease in the signal value, the influence of a component due to a vein is conceivable. The pulse wave sensor 11 acquires both of a component due to an artery and a component due to a vein. A method widely in use is a method of performing, for example, calculation of pulsation information on the basis of the artery component. On the contrary, the vein component causes an adverse effect such as an increase in noise of the pulse wave sensor signal. That is, the excessively small external pressure is considered to be undesirable because the vein component affects and an S/N ratio of the pulse wave sensor signal decreases. A change characteristic example of the AC component signal (the pulse wave detection signal) with respect to the external pressure is shown in Fig. 6. As it is evident from Fig. 6, it is seen that the signal value decreases when the pressing force is excessively large or excessively small.

The blood flow decreases in both the artery and the vein when the external pressure is applied thereto. From characteristics of the living organism, it is known that the blood flow sufficiently decreases in the vein with the external pressure smaller than the external pressure applied to the artery. That is, when pressure at a point where the blood pressure sufficiently decreases (in a narrow sense, vanishes) in the vein (hereinafter referred to as vein vanishing point) is represented as V1 and pressure at a point where the blood flow sufficiently decreases in the artery (hereinafter referred to as artery vanishing point) is represented as V2, a relation V1<V2 holds. In this case, a situation in which the external pressure is larger than V2 is equivalent to the situation in which the external pressure is excessively strong. Even an artery component originally desired to be acquired vanishes and a sufficient signal value cannot be obtained. On the other hand, a situation in which the external pressure is smaller than V1 is equivalent to the situation in which the external pressure is excessively small. A signal value is affected by a vein component and a sufficient signal value is not obtained either.

That is, by applying an external pressure V satisfying V1<V<V2 to the test subject, it is possible to sufficiently suppress the influence of the vein component and prevent the blood flow of the artery component from decreasing more than necessary. In the first embodiment, V satisfying the condition is set as the proper pressing force. The proper pressing force does not have to be the entire V satisfying the condition and may be a pressing force indicating a range of a part of V, a specific pressure value, or the like.

For example, a method has been disclosed to measure, using a pressure sensor or the like, a contact pressure in a portion where biological information is detected and present, on the basis of comparison processing for the contact pressure and a given reference value, an indication whether present pressure is the proper pressing force to the user as graph representation. However, a vital sign such as a pulsation has an extremely large individual different. The proper pressing force takes a different value (range) for each of the users. Therefore, whereas the individual difference cannot be dealt with unless a reference value to be compared with the measured contact pressure is determined for each of the users, in the method in the past, processing based on physical information of simple pressure is merely described. It is unlikely that a signal due to the individual difference is superimposed on the physical information of the pressure. Further, for example, a setting method for a reference value corresponding to the individual difference is not disclosed either. This means that a method for dealing with the individual difference is not disclosed in the method in the past.

Therefore, even if adjustment of the magnitude of the external pressure is instructed to the user through the graph representation or the like in the method in the past, since the proper pressing force set as a reference cannot deal with the individual difference, it is unknown whether a result obtained by adjusting the magnitude of the external pressure according to the instruction is an appropriate state. This is considered to be a first problem.

A second problem of the method in the past is that the influence of a water head pressure is not sufficiently taken into account. It is known that a blood circulation state depends on not only the external pressure but also an internal pressure, which is pressure inside a blood vessel. The water head pressure is conceivable as one cause for determining the internal pressure. Since the magnitude of the water head pressure changes according to a position (i.e., height) in the vertical direction, the water head pressure takes a value that varies according to the posture of the user (in a narrow sense, relative positions in the height direction of a mounting position of the biological information detecting device and the heart of the user). In the first embodiment, as explained below, since pulse wave signal information in different pressing states is detected and pressing force determination is performed on the basis of the pulse wave signal information, at least values of the water head pressure in the pressing states should not substantially fluctuate. When time of use of the biological information detecting device can be actually assumed, by determining the proper pressing force in a state same as a state of the water head pressure during the use, it is possible to improve detection accuracy of the pulse wave signal information during the use. For example, if it is assumed that the user wears the device on the wrist and uses the device during running, it is desirable to perform the determination of the proper pressing force in a posture in which a wrist position during the running is reproduced as much as possible. However, these points are not taken into account in the method in the past. Further, between walking and running, a ratio of superimposition of body motion noise is larger in the running because the user swings the arms more quickly. Difficulty in detecting pulse wave signal information tends to be higher during the running. Therefore, it is desirable to set an optimum pressing force assuming an exercise state such as the running rather than the walking.

Therefore, the applicant proposes a determination method for the proper pressing force based on a pulse wave sensor signal. A characteristic of the pulse wave sensor signal appears for each of the users. Therefore, it is possible to deal with an individual difference if determination based on the pulse wave sensor signal is performed. Furthermore, although a range of the proper pressing force could fluctuate according to a change in a physical condition or the like even in the same user, the method in the first embodiment can deal with such a change.

However, since a signal value of the pulse wave sensor signal is different for each of the users as explained above, a signal value at the proper pressing force is relatively large for some users and is relatively small for other users. Therefore, even if a signal value in a certain pressing force is independently used, it is difficult to determine whether the pressing force is the proper pressing force. Therefore, in the first embodiment, it is assumed to acquire the pulse wave sensor signal while changing a pressing force and determine whether the pressing force is the proper pressing force using a change characteristic of the pulse wave sensor signal with respect to the pressing force.

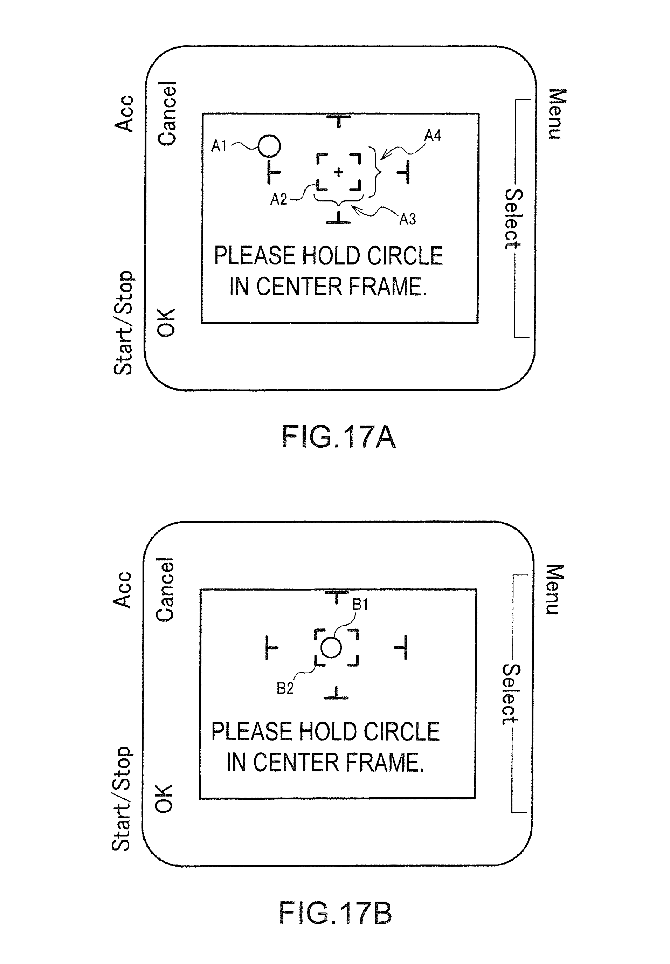

Further, before measurement processing in a given pressing state, a posture state notification image (e.g., Figs. 17A and 17B referred to below) is displayed to urge the user to take a posture state suitable for the measurement processing. By instructing an appropriate posture to the user using the posture state notification image, it is possible to solve the problem concerning the water head pressure. It is known that pulse wave signal information fluctuates according to an exercise state of the user. In the proper pressing force determination, the fluctuation is a cause of accuracy deterioration. Therefore, it may be instructed in the posture state notification image to set the water head pressure to a proper pressure and set a posture in a rest state.

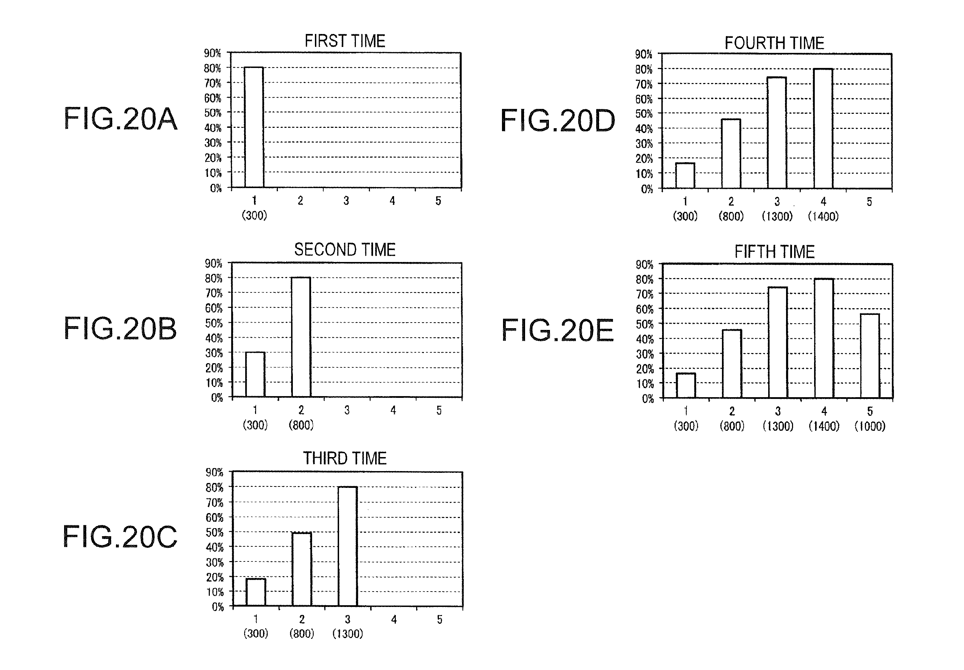

When results of the measurement processing in the pressing states are displayed, the individual difference is taken into account. Since an absolute value of the pulse wave information has a large individual difference as explained above, if the graph representation or the like is performed directly using the absolute value, depending on a user, the absolute value cannot be displayed because a scale of an axis is small for the absolute value or all values of the pulse wave information are too small compared with the scale and a difference cannot be recognized. Therefore, in the first embodiment, after normalization processing is performed, a value of measured pulse wave information is displayed. A specific normalization method or the like is explained below with reference to Figs. 20A to 20E. However, if the normalization processing is performed, it is possible to absorb the individual difference of the absolute value of the pulse wave information and present a result display screen that the user can easily recognize (in a narrow sense, with which the user can easily recognize fluctuation in a value of pulse wave information corresponding to a pressing state).

In the first embodiment, when it is determined whether the pressing force is the proper pressing force, information representing a state of a load applied when it is determined that the pressing force is the proper pressing force is presented to the user. This is, for example, information indicating which band hole should be used to mount the device to enable the proper pressing force to be applied in the case of this user. Consequently, a proper mounting state can be specified by, for example, a state of tightening of the band rather than a value of a pressing force. This method is intuitive and clear for the user. If the information is stored and displayed when the biological information detecting device is mounted again, the user can easily reproduce a holding state for realizing the proper pressing force. That is, adjustment or the like is unnecessary when the biological information detecting device is mounted again. This is advantageous from the viewpoint of convenience and the like.

In the method in the past, a method of automatically performing pressurization and depressurization using a pump or the like is also disclosed. This method is preferable in that manual mounting state adjustment by the user is unnecessary. However, when it is assumed that the biological information detecting device is used in a daily life and during an exercise like a wrist-mounted device, this method is unrealistic if a size, power consumption, and the like are considered. Therefore, such a method is not taken into account. That is, in the first embodiment, the pressing force is changed in the determination of the proper pressing force as explained above. It is assumed that the pressing force is manually changed by the user. Therefore, in order to instruct the user to appropriately change the pressing force, it is important to perform interaction with the user using an interface such as the display section.

In the following explanation, a specific system configuration example of the biological information detecting device is explained and then an example of an image displayed on the display section is explained. Thereafter, determination processing for the proper pressing force is explained as a specific example of processing in which, for example, display of a posture state notification image and a normalized graph is performed.

### 3. Configuration example of the biological information detecting device

A configuration example of the biological information detecting device is explained. Specifically, a system configuration example of the biological information detecting device is explained and then the structure of the load mechanism and the like are explained.

### 3. 1 Specific system configuration example

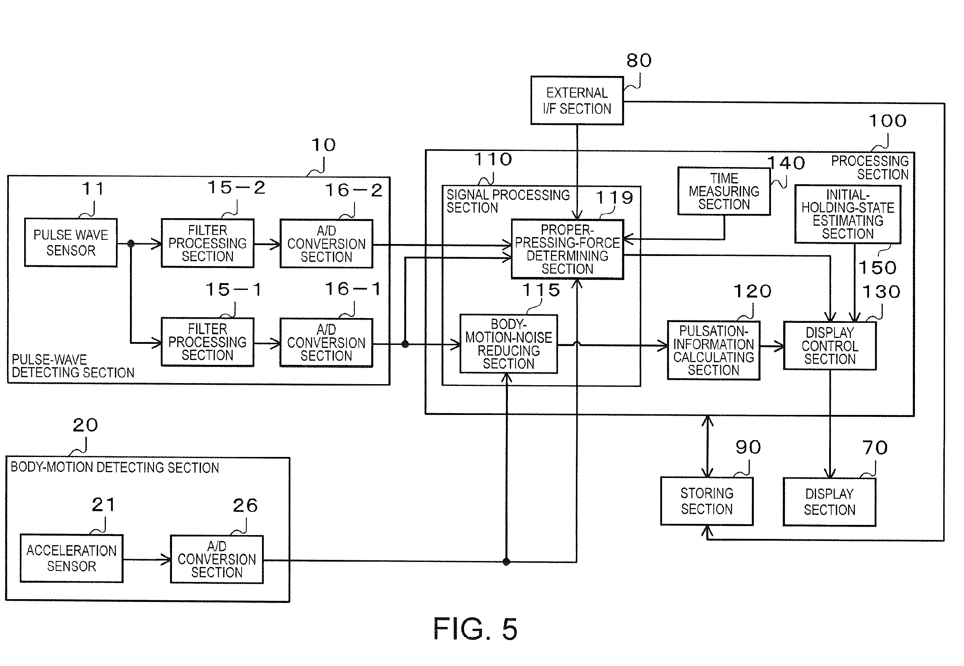

A specific system configuration example of the biological information detecting device in the first embodiment is shown in Fig. 5. The biological information detecting device includes the pulse-wave-information detecting section 10, the body-motion-information detecting section 20, the processing section 100, the display section 70, an external I/F section 80, and a storing section 90. However, the biological information detecting device is not limited to the configuration shown in Fig. 5. Various modifications are possible to, for example, omit a part of the components and add other components. For example, in the first embodiment, the reduction in body motion noise is not essential. The body-motion-noise reducing section 115 and the like of the processing section 100 may be omitted.

The pulse-wave-information detecting section 10 includes the pulse wave sensor 11, filter processing sections 15-1 and 15-2, and A/D conversion sections 16-1 and 16-2. However, the pulse-wave-information detecting section 10 is not limited to the configuration shown in Fig. 5. Various modifications are possible to, for example, omit a part of the components and add other components.

As the pulse wave sensor 11, the photoelectric sensor or the like is used as explained above with reference to Fig. 1. In the first embodiment, the filter processing section 15-1 is realized by a high-pass filter configured to perform high-pass filter processing. The filter processing section 15-2 is realized by a low-pass filter configured to perform low-pass filter processing. That is, an output of the filter processing section 15-1 is an AC component signal, which is a high-frequency component of a pulse wave sensor signal. An output of the filter processing section 15-2 is a DC component signal, which is a low-frequency component of the pulse wave sensor signal. In the first embodiment, the pulse-wave-information detecting section 10 includes the A/D conversion section 16-1 and the A/D conversion section 16-2, which respectively convert input analog signals into digital signals and output the digital signals.

As shown in Fig. 5, the pulse wave sensor 11 is connected to the filter processing section 15-1 and the filter processing section 15-2. The filter processing section 15-1 is connected to the A/D conversion section 16-1. The A/D conversion section 16-1 is connected to the body-motion-noise reducing section 115 and a below-mentioned proper-pressing-force determining section 119. The filter processing section 15-2 is connected to the A/D conversion section 16-2. The A/D conversion section 16-2 is connected to the proper-pressing-force determining section 119.

In the pulse-wave-information detecting section 10, the filter processing section 15-2 may be omitted. In that case, an output of the A/D conversion section 16-2 is a signal including both of the high-frequency component and the low-frequency component of the pulse wave sensor signal. Besides, various modifications are possible concerning connection of the sections included in the pulse-wave-information detecting section 10.

The body-motion-information detecting section 20 includes the acceleration sensor 21 and the A/D conversion section 26. The acceleration sensor 21 is connected to the A/D conversion section 26. The A/D conversion section 26 is connected to the body-motion-noise reducing section 115 and the proper-pressing-force determining section 119. The body-motion-information detecting section 20 only has to include a sensor configured to detect a body motion. The acceleration sensor 21 may be changed to another sensor. The body-motion-information detecting section 20 may include a plurality of sensors.

The processing section 100 includes the signal processing section 110, the pulsation-information calculating section 120, a display control section 130, a time measuring section 140, and an initial-holding-state estimating section 150. The signal processing section 110 includes the body-motion-noise reducing section 115 and the proper-pressing-force determining section 119. However, the processing section 100 and the signal processing section 110 are not limited to the configuration shown in Fig. 5. Various modifications are possible to, for example, omit a part of the components (e.g., the body-motion-noise reducing section 115) and add other components.

The proper-pressing-force determining section 119 determines, based on at least one of the AC component signal output from the A/D conversion section 16-1 and the DC component signal output from the A/D conversion section 16-2, whether a pressing force corresponding to acquisition timing of the signals is the proper pressing force. In the determination, the proper-pressing-force determining section 119 may use a body motion detection signal output from the body-motion-information detecting section 20, time measurement information output from the time measuring section 140, and the like. The proper-pressing-force determining section 119 acquires holding state specifying information for specifying a holding state of the load mechanism 300 at the time when it is determined that the pressing force is the proper pressing force on the basis of, for example, information output from the external I/F section 80 and outputs the acquired holding state specifying information to the storing section 90 and the display control section 130. Details of the processing in the proper-pressing-force determining section 119 are explained below.

The body-motion-noise reducing section 115 applies body-motion-noise reduction processing to the AC component signal output from the A/D conversion section 16-1 on the basis of the body motion detection signal output from the body-motion-information detecting section 20. Processing contents of the body-motion-noise reducing section 115 are the same as the processing contents explained above with reference to Fig. 2 and the like. Therefore, detailed explanation of the processing contents is omitted. Processing in the pulsation-information calculating section 120 is also as explained above.

The display control section 130 performs control for display on the display section 70. For example, in the determination in the proper-pressing-force determining section 119, it is necessary to change the pressing force. The display control section 130 performs control for displaying an instruction screen for instructing the user to appropriately change the pressing force. The display control section 130 may perform control for displaying an instruction screen for instructing the user to set an environment for determining whether the pressing force is the proper pressing force. Besides, the display control section 130 performs, for example, display control for pulsation information calculated by the pulsation-information calculating section 120. Details are explained below.

The time measuring section 140 performs time measurement processing. For example, a time measuring section is conceivable that includes a timer configured to acquire time information such as a time stamp at a given interval and measures time from, for example, a difference of the acquired time information.

The initial-holding-state estimating section 150 performs, on the basis of personal information of the user stored in the storing section 90, processing for estimating a holding state of the load mechanism 300 optimum for the user. For example, it is conceivable to perform a statistical survey or the like beforehand to calculate a correlation between personal information (BMI, sex, age, etc.) of the user and an optimum band hole position, perform polynomial approximation to thereby obtain a correlation expression, and substitute the personal information of the user in the correlation expression to estimate the optimum band hole position.

The display section 70 displays various kinds of information according to control contents in the display control section 130. The external I/F section 80 functions as an interface with the outside. In a narrow sense, the external I/F section 80 may be an operation section including various buttons and a GUI for the user to perform various kinds of operation of the biological information detecting device. The storing section 90 functions as a work region of the processing section 100 and the like. A function of the storing section 90 can be realized by a memory such as a RAM, a HDD (hard disk drive), or the like. The storing section 90 stores various kinds of information. In particular, the storing section 90 stores the holding state specifying information acquired in the proper-pressing-force determining section 119.

### 3.2 Configuration example of the load mechanism and the like

Next, the structures of the load mechanism 300 and the pulse-wave-information detecting section 10 (especially a contact portion with a living organism) will be explained.

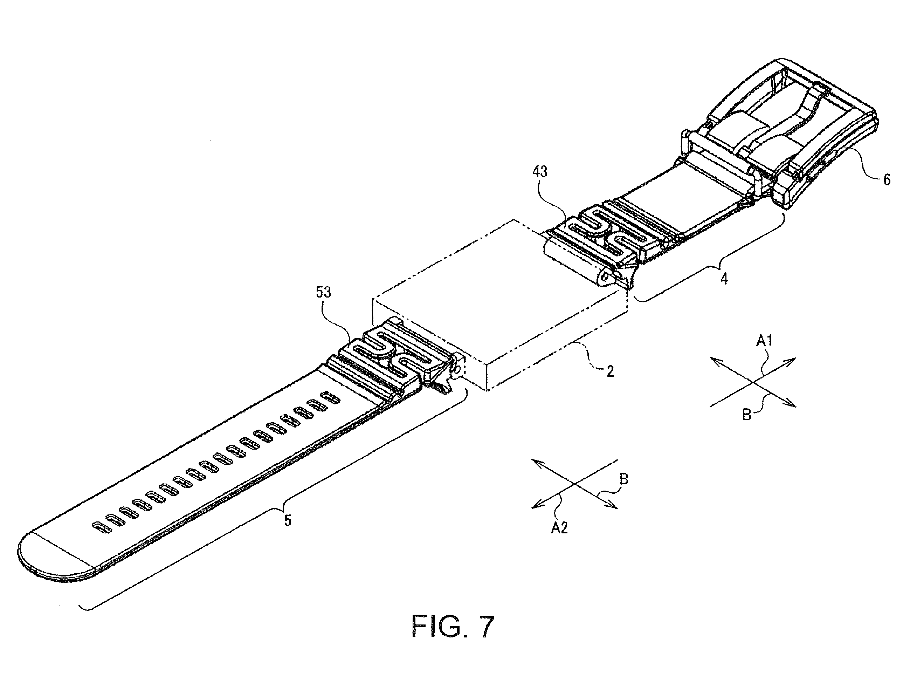

The biological information detecting device includes, as shown in Fig. 7, the device main body 2 attached closely to the human body and configured to measure the biological information and a band (in a broad sense, a load mechanism) 3 attached to the device main body 2. The band 3 includes a first band member 4 and a second band member 5. It is assumed that the pulse-wave-information detecting section 10, the processing section 100, and the display section 70 explained above are included in the device main body 2. The band 3 includes expandable sections 43 and 53 shown in Fig. 7.

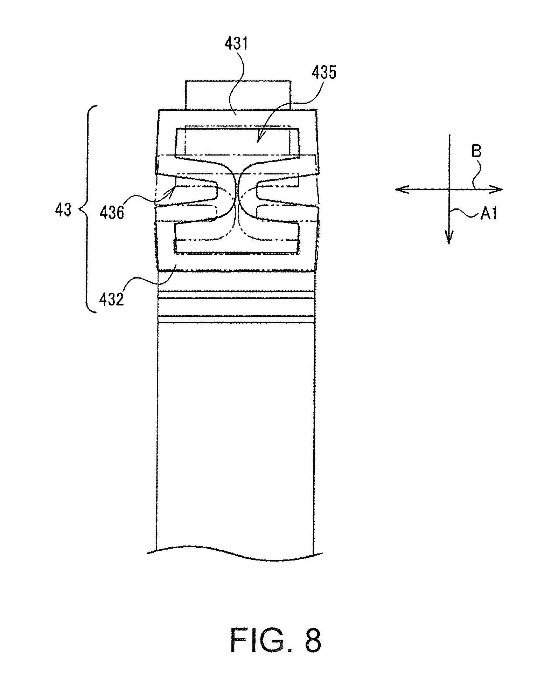

Fig. 8 is a plan view showing an expanded state of the expandable section 43. The expandable section 43 includes a first slit 435 and a second slit 436. Therefore, as shown in Fig. 8, the expandable section 43 is expandable along an A1 direction according to a tensile force of a wearer. Since the expandable section 43 has flexibility, a restoring force for returning to an original state acts when the expandable section 43 is expanded. Therefore, when the tensile force of the wearer is cancelled, the expandable section 43 contracts in the opposite direction of the A1 direction with the restoring force and returns to the original state.

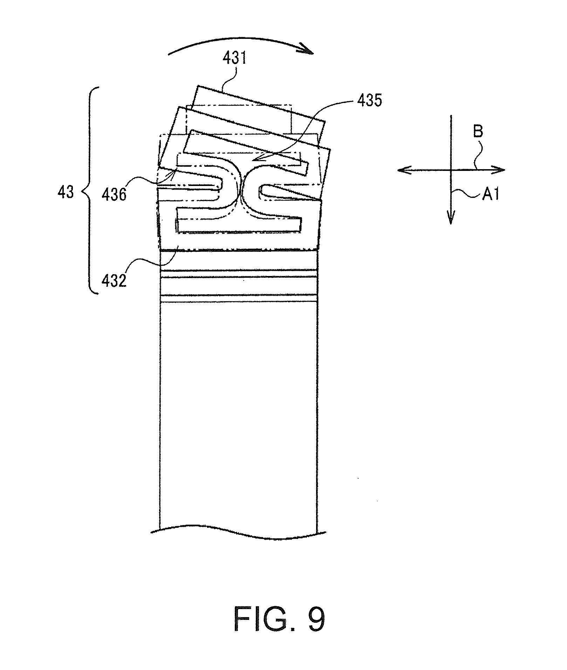

Fig. 9 is a plan view showing a twisted state of the expandable section 43. The expandable section 43 includes a first slit 435 and a second slit 436. Therefore, as shown in Fig. 9, the expandable section 43 is twistable. Since the expandable section 43 is configured to be twistable in this way, when the biological information detecting device is mounted on the wrist or the like, a twist (a tilt) between the device main body 2 and the first band member 4 is allowed. The device main body 2 can be appropriately brought into press contact with the wrist.

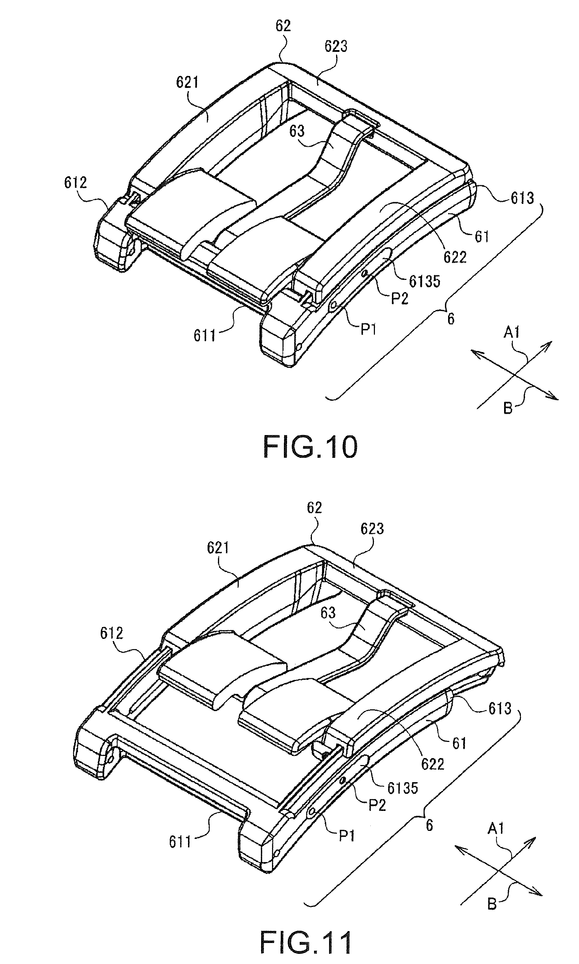

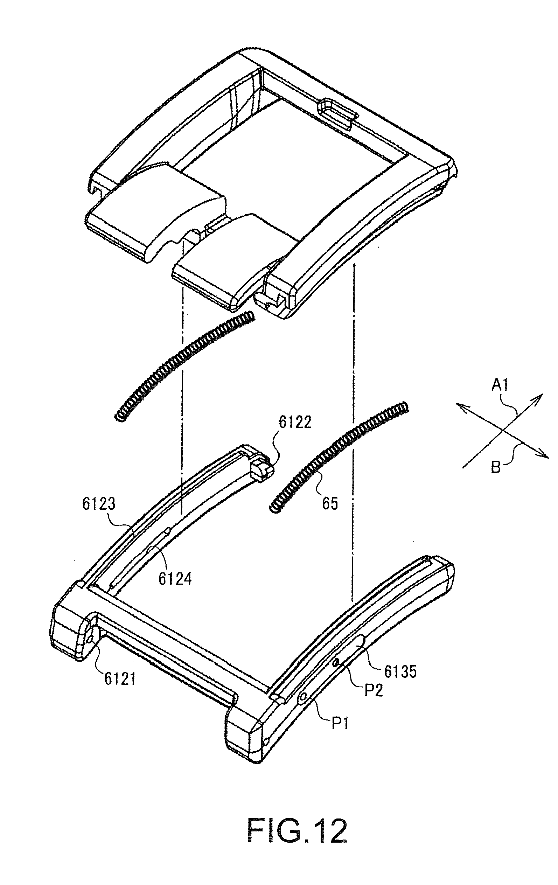

The biological information detecting device includes a coupling member 6 shown in Fig. 7. Figs. 10 and 11 are perspective views showing the coupling member 6. Fig. 10 shows the coupling member 6 before a slide of a slide member 62. Fig. 11 is the coupling member 6 after the slide of the slide member 62. Fig. 12 is an exploded perspective view showing the coupling member 6.

The coupling member 6 is a member made of metal or synthetic resin functioning as a buckle configured to couple the first band member 4 and the second band member 5. As shown in Figs. 10 to 12, to lie along the wrist, the coupling member 6 is formed in an arcuate shape such that a cross section extending along the A1 direction has a predetermined curvature.

The coupling member 6 includes a fixing member 61 (Figs. 10 to 12), a slide member 62 (Figs. 10 to 12) configured to slide on the fixing member 61 along the A1 direction, a projecting bar 63 (Figs. 10 and 11), and coil springs 65 (Fig. 12).

As shown in Figs. 10 to 12, the fixing member 61 is a frame-like body configured to slidably support the slide member 62. The fixing member 61 includes a base 611 extending along a B direction and a pair of extending sections 612 and 613 connected by the base 611 and extending along the A1 direction.

The extending section 612 located on the left side in Fig. 12 includes an insert-through hole 6121, a locking section 6122, a convex section 6123, a long hole 6124, and a display section (not shown in the figure).

The locking section 6122 is formed to project from an end on the opposite side of the first band member 4 side in the extending section 612 toward the other extending section 613. The locking section 6122 locks one end of the coil spring 65.

The convex section 6123 is formed along the A1 direction on an upper surface opposed to the slide member 62 in the extending section 612.

The long hole 6124 is formed along the A1 direction on a side surface opposed to the extending section 613 in the extending direction 612. An end of a below-mentioned spring bar 64 is inserted into the long hole 6124.

A display section 6135 is formed along the A1 direction on a side surface on the opposite side of the extending section 612 in the extending section 613. A scale indicating a proper slide range of the slide member 62 is added to the display section 6135. Specifically, in the first embodiment, two points P1 and P2 indicating the proper slide range are added to the display section 6135. If the end on the first band member 4 side of the slide member 62 is located within the range indicated by the two points P1 and P2, a proper tensile force is caused to act by the band 3.

As shown in Figs. 10 and 11, the slide member 62 slides relatively to the fixing member 61 along the A1 direction and adjusts the length dimension of the band 3 and pressure applied to the human body by the device main body 2. That is, the slide member 62 has a function of causing a tensile force to act on the band 3 and bringing the device main body 2 into close contact with the human body.

The slide member 62 includes, as shown in Figs. 10 to 12, a pair of first side sections 621 and 622 extending along the A1 direction and second side sections 623 and 624 configured to respectively connect ends of the first side sections 621 and 622 along the B direction. The slide member 62 is formed in a substantially rectangular frame shape as a whole by the side sections. The second side section 624 is desirably present for improvement of the strength of the slide member 62. However, the function of the slide member 62 is satisfied even if the second side section 624 is absent.

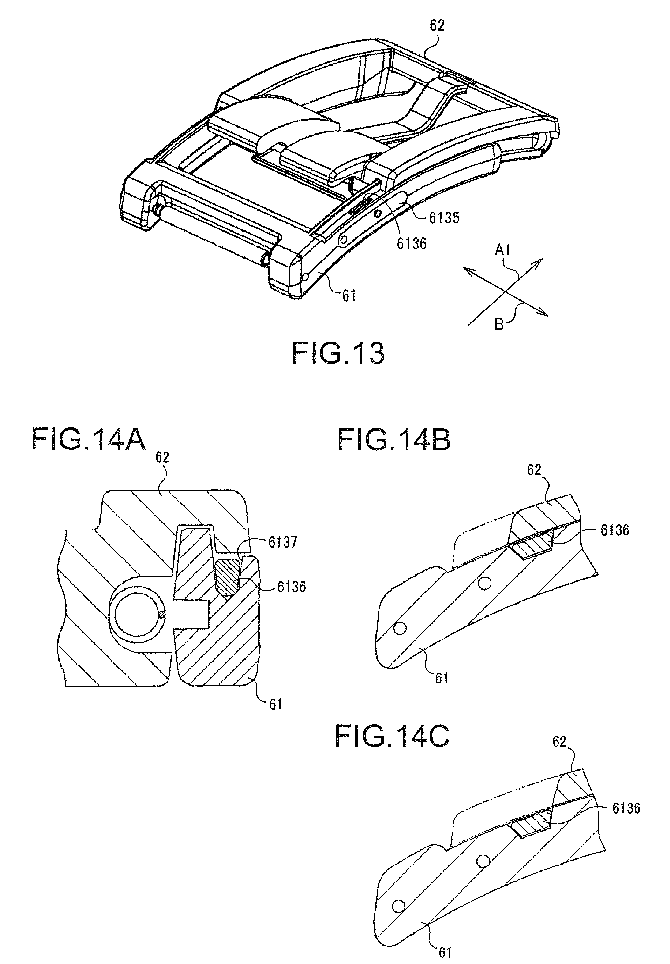

Positioning dies 6136 functioning as second display sections may be provided separately from the display section 6135. As shown in Figs. 13 to 14C, holes 6137 are formed on the surfaces of the extending sections 612 and 613 of the fixing member 61 and the positioning dies 6136 are welded and embedded in the holes 6137. For example, the fixing member 61 and the positioning dies 6136 are formed of synthetic resin (e.g., polyacetal: POM) of the same quality and colored in different colors. In the first embodiment, the positioning dies 6136 are molded of synthetic resin colored in yellow.

When the slide member 62 is slid, a position where the positioning die 6136 starts to be seen as shown in Fig. 14B is set as a minimum movement of the slide member 62. A position where the entire positioning die 6136 is completely seen as shown in Fig. 14C is set as a maximum movement of the slide member 62. The user can easily grasp an appropriate pulling position by moving the slide member 62 to be present within this range.

By adopting the configuration including the expandable sections 43 and 53, it is possible to stably mount the biological information detecting device horizontally with respect to the wrist. By providing the display section 6135 and the positioning dies 6136, it is possible to present a state of an applied pressing force to the user. However, as explained above, since the proper pressing force has a large individual difference of each of the users, determination processing for the proper pressing force is performed using the pulse wave information. That is, the display section 6135 and the like indicate a standard of a general proper pressing force range and are used for a purpose of, for example, narrowing down a search range of a pressing state to some extent with reference to the standard. Concerning actual proper pressing force determination processing, it is assumed that a below-mentioned method is used.

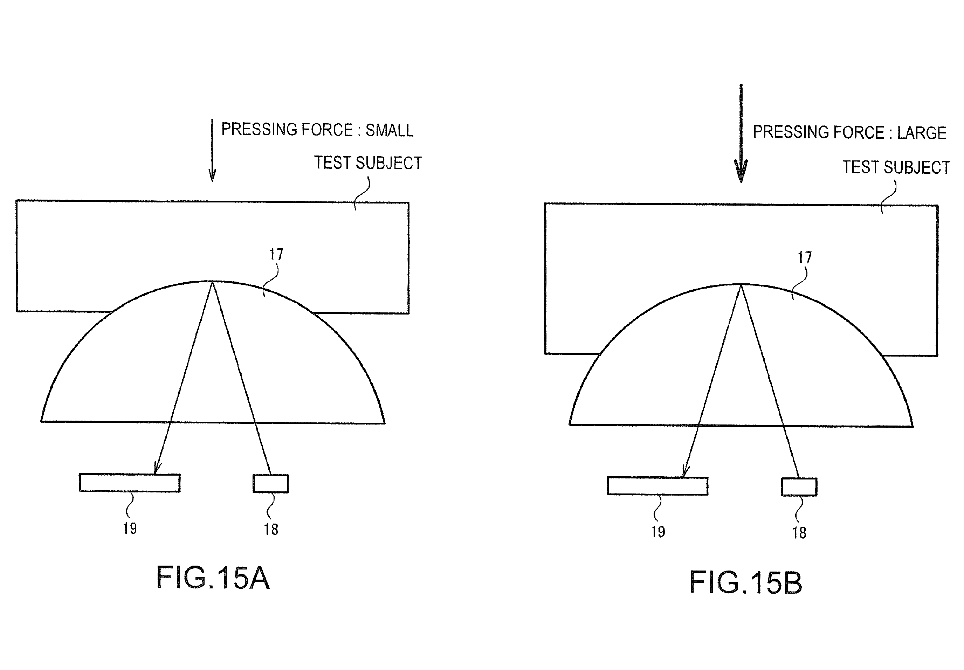

A configuration example of a contact portion with the wrist surface of the user in the pulse-wave-information detecting section 10 is explained. Figs. 15A and 15B are enlarged schematic diagrams of a part of the rear surface (on the wrist side) of the device main body 2. Specifically, Fig. 15A is an enlarged diagram of a region where the pulse-wave-information detecting section 10 is provided in the device main body 2. As shown in Fig. 15A, the pulse-wave-information detecting section 10 includes an LED 18 configured to irradiate light, a photodiode (PD) 19 configured to receive reflected light of the irradiated light reflected by the living organism, and a convex section 17 functioning as a contact portion with the living organism. The biological information detecting device in the first embodiment includes the convex section shown in Fig. 15A to efficiently apply pressure to the living organism. The pressure applied to the living organism in the convex section is the pressing force explained above.

As shown in Figs. 15A and 15B, the pressing force changes as a load state changes. As a result, a contact area between the convex section and the living organism also changes. For example, as shown in Figs. 15A and 15B, if the reflected light is received in a region that is in contact with the living organism irrespective of the load state, i.e., a region where the pressing force can be properly applied, it is possible to accurately measure pulse wave information on the basis of the received reflected light. A structure example of the contact portion of the wrist surface of the user is shown as a convex shape. However, the shape of the contact portion is not limited to this.

### 3.3 Configuration example of external processing

In the example explained above, the processing section 100 configured to perform the measurement processing for the pulse wave information and the determination processing for the proper pressing force and the display section 70 configured to perform the display of the posture state notification image are explained as being included in the biological information detecting device. However, the first embodiment is not limited to this. The processing and the display may be performed in other electronic devices such as a smart phone.

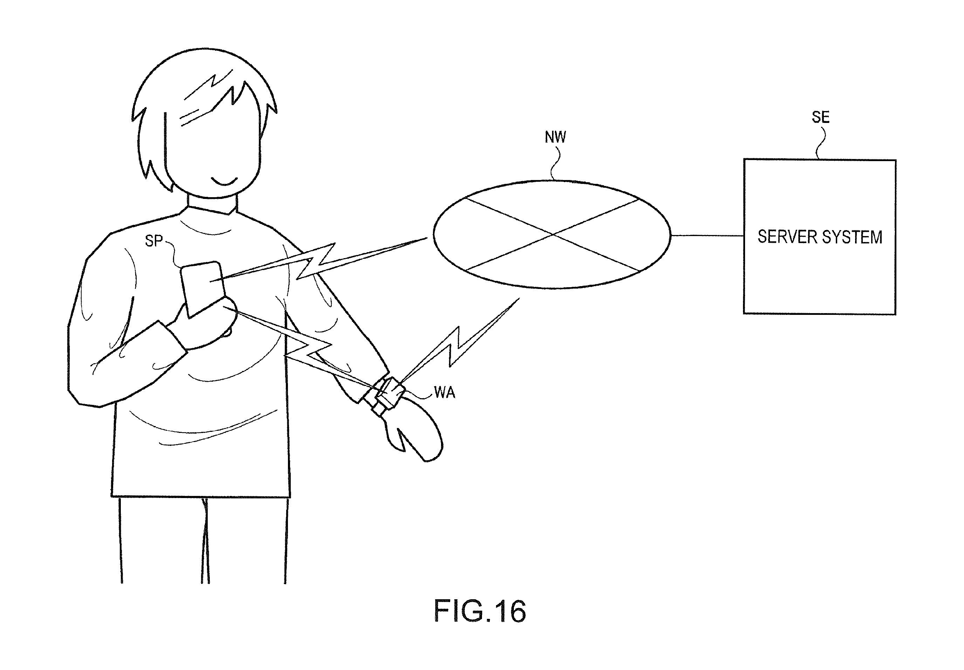

A specific example is shown in Fig. 16. In the example shown in Fig. 16, a device WA that the user wears on the wrist or the like includes the pulse wave sensor 11 configured to detect pulse wave information, the body motion sensor (the acceleration sensor 21) configured to detect body motion information, and a communication section configured to transmit the pulse wave information and the body motion information to another electronic device. In this case, the processing in the processing section 100 explained above is not performed in the device WA.

The communication section of the device WA transmits the pulse wave information and the body motion information to an electronic device SP. The electronic device SP acquires the pulse wave information and the body motion information and performs processing. Specifically, the electronic device SP includes a processing section and a display section. The processing section of the electronic device SP performs processing same as the processing by the processing section 100. The display section of the electronic device SP performs processing same as the processing by the display section 70.

Usually, as the biological information detecting device, a small biological information detecting device is used taking into account that, for example, the biological information detecting device does not hinder an exercise or the like of the user and is easily mounted for a long time. Therefore, there are limits in processing performance of the processing section 100, the size of a display region of the display section 70, and the like. In this regard, if the device WA is used for acquisition and transmission of sensor information and the other electronic device SP performs actual processing and display, it is possible to perform high-speed processing, display on a relatively large screen, and the like. In particular, a portable terminal such as a smart phone can be easily carried even during detection of biological information (e.g., during walk or running). Therefore, the device WA can be used as a device for display.

Various modifications are possible for the configuration of the device for performing the processing and the configuration of the device for performing the display. For example, the smart phone may process pulse wave information or the like, generate a display image (or information corresponding to the display image), and transmit the display image to the device WA. The display section 70 of the device WA may display the display image. This configuration is effective when processing with a large processing load is assumed.

The device for performing the processing is not limited to a device provided in a position close to the user (e.g. a device that the user wears and carries). For example, if the device WA is configured to be capable of communicating with a network NW such as the Internet, the device may transmit pulse wave information or the like acquired from a sensor to a server system SE connected via the network NW. The server system SE may perform processing corresponding to the processing section 100 and transmit a processing result to a device for performing display of the processing result (which may be the device WA, the electronic device SP, or other electronic devices).

As it is seen from the above, the method in the first embodiment can be realized by various electronic devices.

### 4. Specific example of a display screen

An example of a display screen displayed on the display section 70 in the method in the first embodiment and an example of screen transition are explained.

### 4. 1 Posture state notification image

Figs. 17A and 17B are example of a posture state notification image. As shown in Fig. 17A, an object A1 is displayed in a position corresponding to the present posture state of the user in all or a part (in the example shown in Fig. 17A, a part) of the display region of the display section 70. When the object A1 is located in a first region A2 in the display region, a posture state is appropriate. When the object A1 is located in a region other than the first region A2, the posture state is inappropriate.

The display section 70 updates the position of the object A1 on a real time basis according to the posture state of the user. For the user wearing the biological information detecting device, the position of the object A1 changes according to the posture state of the user. Therefore, the user adjusts the posture of the user to set the object A1 in the first region A2 (the state shown in Fig. 17B) while viewing the posture state notification image.

The posture state of the user is determined by determination processing in the processing section 100. For example, the processing section 100 may acquire acceleration information from the acceleration sensor 21, which is a type of the body motion sensor, and determine the posture state using the acquired acceleration information. A specific example is shown below. However, the determination processing for the posture state in the processing section 100 may be realized by other methods.

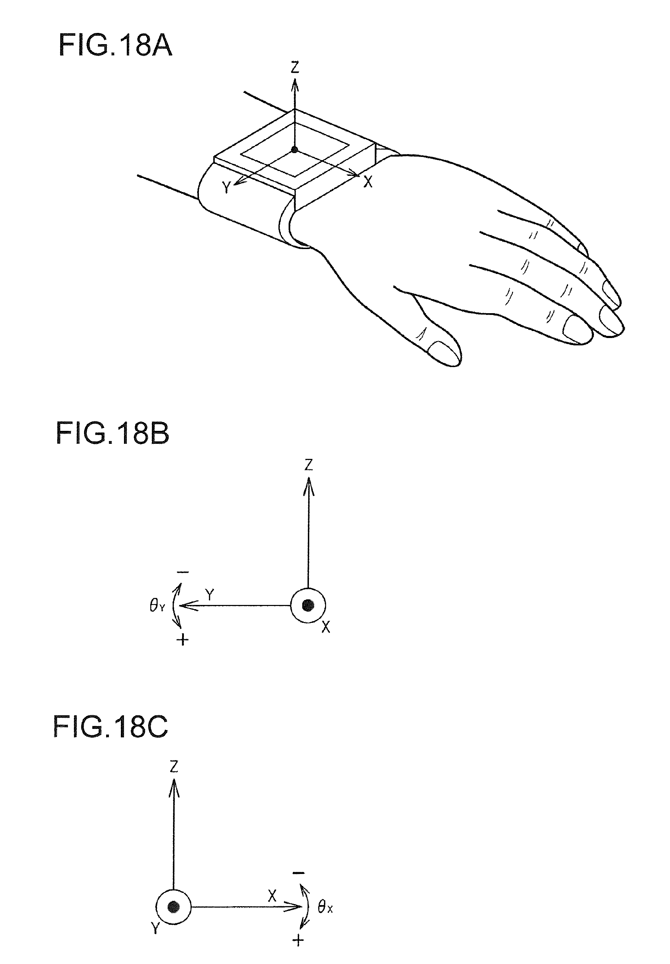

As coordinate axes set in the biological information detecting device, coordinate axes shown in Fig. 18A are examined. A direction from the forearm to the hand of the user is represented as X axis. A direction orthogonal to the display section 70 and directed from the wrist to the display section 70 side is represented as Z axis. An axis orthogonal to the X axis and the Z axis is represented as Y axis. As shown in Fig. 18A, the X axis, the Y axis, and the Z axis are assumed to be the axes in the left-hand system. The acceleration sensor 21 in the first embodiment detects acceleration values in the axes as sensor information. For example, when the user is in a rest state (an acceleration value due to an exercise is not detected), signals or the like of an X-axis component, a Y-axis component, and a Z-axis component of gravitational acceleration acquired as values of the X, Y, and Z axes are the sensor information.

That is, if shifts of a posture allowed in measurement of values of X, Y, and Z in an appropriate posture and pulse wave information (fluctuation values allowed in the X, Y, and Z axes) are determined, numerical value ranges of X, Y, and Z corresponding to the appropriate posture are set. It is possible to determine whether the present posture state is appropriate according to comparison processing for acceleration values in the present posture state and the numerical value ranges.

As explained above, the posture suitable for measurement of pulse wave information represents a posture in which a water head pressure is in the same degree as a water head pressure in a posture assumed during measurement. This is because, if the proper pressing force is determined in such a posture, states of water head pressures during proper pressing force determination and during actual measurement are close to each other and improvement of detection accuracy of the pulse wave information can be expected by using the proper pressing force, which is a determination result. The posture assumed during the measurement could change according to the shape, the mounting position, and the use of the biological information detecting device. In this explanation, a posture during an exercise such as walking or running is examined.

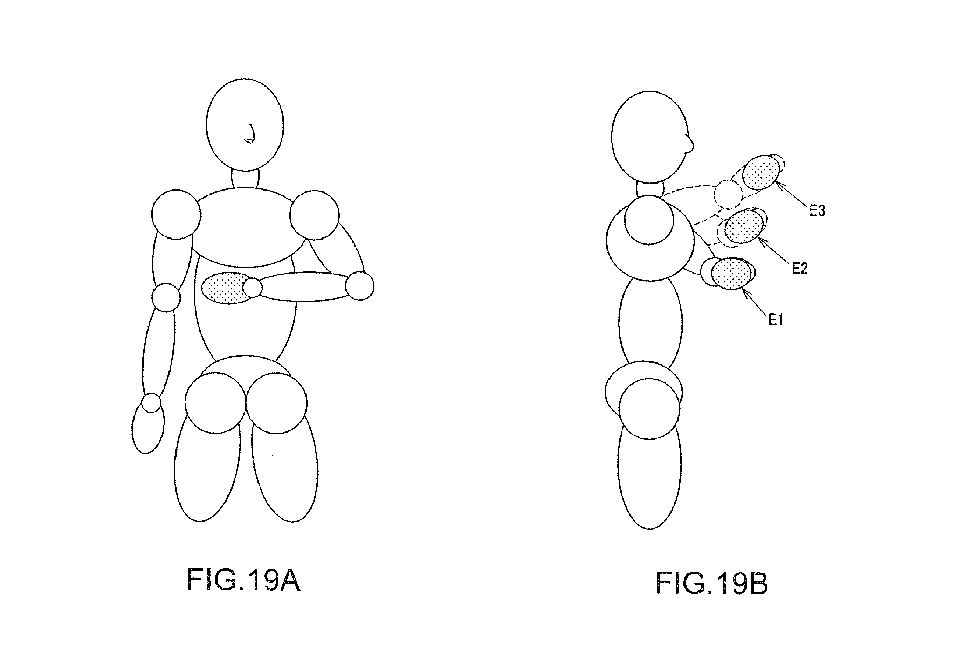

Since the arm is swung during the walking or the running, the position (the height) in the vertical direction of the biological information detecting device changes. As indicated by Fig. 19A and E1 in 19B, a state in which the elbow is bent and the arm is placed close to the trunk is set as an appropriate posture. Fig. 19A is a diagram of the appropriate posture viewed from the front. Fig. 19B is a diagram of the appropriate posture viewed from a side.

Numerical value ranges of X, Y, and Z allowed when the posture shown in Fig. 19A (El in Fig. 19B) is set as a reference are examined. A direction of the X axis in the state of the posture shown in Fig. 19A, i.e., in a state in which the Z-axis direction coincides with the opposite direction of the gravity direction is represented as X-axis reference direction. A direction of the Y axis in the state is represented as Y-axis reference direction. As shown in Figs. 18B and 18C, a rotation (rotation around the Y axis) angle of the X axis with respect to the X-axis reference direction is represented as θx and a rotation (rotation around the X axis) angle of the Y axis with respect to the Y-axis reference direction is represented as θy. An allowed posture change is determined from θx·θy.

θx and θy are explained assuming that rotation on the gravity direction side is a positive value and rotation on the opposite side of the gravity direction is a negative value.

What should be examined first is rotation in a plus direction of θy. As it is seen from Fig. 18B, Fig. 19A, and Fig. 19B, the rotation in the plus direction of θy is a motion for turning the display section 70 to the face direction of the user and is a motion that could be usually performed. However, as it is seen when such a rotating action in the plus direction and a rotating action in a minus direction, which is the opposite direction, are actually executed, ergonomically, the rotation in the plus direction is an unnatural motion with a narrow movable range. Such an unnatural motion applies a large twisting force to the arm. When the arm is twisted, pressure fluctuation is caused by the influence of the twist. A value of pulse wave information also fluctuates compared with a value of pulse wave information obtained when the arm is not twisted. As explained above, in the determination process for the proper pressing force, a state close to a state during actual measurement is desirable. Such a twist has to be suppressed from being applied.

Further, the rotation in the plus direction of θy is a posture unnatural for the user. Therefore, continuation of the posture for a long time is a load on the user. As explained below, considering that a certain degree of time is required to measure pulse wave information in a given load state, it is undesirable to set such a posture as a proper posture.

A posture with θy set to plus is an ergonomically comfortable state if the arm is lifted as indicated by E2 and E3 in Fig. 19B. That is, if the rotation in the plus direction of θy is allowed, it is highly likely that the user lifts the arm in an attempt to cancel the unnatural posture. As a result, a state of a water head pressure deviates from a state assumed during measurement.

If the deterioration in the accuracy of the pulse wave information due to the twist, the load on the user due to the unnatural state, the likelihood of the lift of the arm for cancelling the unnatural posture, and the like are taken into account, it is considered desirable to secure a wide allowable range of θy in the minus direction compared with the plus direction. Therefore, a range in which a median is a negative value as an angle range of θy such as -18 degrees<θy<+ 6 degrees is set.

θx is examined. Concerning θx, both of the rotation in the plus direction and the rotation in the minus direction cause fluctuation in the height of the biological information detecting device. Therefore, although the fluctuation should be suppressed, it is undesirable to require the user to take an excessively accurate posture. Therefore, a certain degree of an angle range is determined as a proper posture. Concerning θx, unlike θy, there is no reason for giving more importance to the plus direction or the minus direction. Therefore, an angle range such as -12 degrees<θx<+12 degrees only has to be set.

Concerning the Z axis, usually, a value close to -1 G is taken. A range of angle fluctuation may be set for the Z axis as well in the same manner as the range setting for the X axis and the Y axis. However, in this explanation, the determination processing for a posture state is performed in the two axes of X and Y and a fine angle range is not set for the Z axis. However, when an acceleration value of Z is substantially different from -1 G (e.g., a value of Z is plus), a situation is possible in which an inappropriate motion such as a lift of the arm with all the might is performed or some error occurs. Therefore, it is determined that a posture is not a proper posture irrespective of values of the X axis and the Y axis. Alternatively, measures such as some error processing may be taken.

In this explanation, it is determined from θx and θy whether a posture is a proper posture. However, as explained above, actual sensor information is acceleration values of the X, Y, and Z axes. Therefore, rather than directly using the angle ranges of θx and θy, numerical value ranges of acceleration values of the X, Y, and Z axes corresponding to the angle ranges may be calculated in advance and the determination processing for a posture state may be performed using the numerical value ranges. For example, if a correspondence relation in which, for example, θy=+6 degrees corresponds to an acceleration value of the Y axis of about 0.10 G and θy=-18 degrees corresponds to an acceleration value of about -0.31 G is calculated in advance, a condition -18 degrees<θy<+6 degrees can be converted into a condition -0.31G<ay<0.10G concerning an acceleration value ay of the Y axis.

A posture state only has to be determined by the processing explained above. A result of the determination only has to be displayed as the posture state notification image shown in Figs. 17A and 17B. In the examples shown in Figs. 17A and the like, an allowable range in the X axis only has to be associated with a range (A3) in the abscissa direction of the first region. An allowable range in the Y axis only has to be associated with a range (A4) in the ordinate direction of the first region. For example, if the numerical range of -0.31G<ay<0.10G is set for ay as explained above, a numerical value change amount due to a difference in a display position of one dot is specified by dividing a numerical value range of the Y axis (in the above example, 0.41 G) by the number of dots corresponding to A4. Then, it is possible to change a display position of the object A1 in the ordinate direction and display the object A1 according to a posture change in the Y-axis direction. Concerning the X-axis direction, when considered the same way, it is possible to notify the user whether a posture state is appropriate according to the position of the object A1 in the posture state notification image. A region other than the first region only has to be considered the same way.

As shown in Figs. 17A and 17B, a display form of the object A1 may be changed according to whether the object A1 is present in the first region or present in the second region other than the first region. In Figs. 17A and 17B, a difference is that the object A1 is represented by a black circle (paint-out) or represented by a white circle (hollow). Consequently, it is possible to clearly present to the user whether the present posture state is appropriate. The change in the display form is not limited to the method shown in Figs. 17A and 17B and may be a change in a shape or a change in a color. Alternatively, blinking may be performed in one of the first region or the second region. Rather than changing the display form, a method of, for example, generating sound or vibration when the object is located in the first region may be used.

### 4.2 Normalized graph

When it is determined that the posture state is appropriate, pulse wave information in the present load state (pressing state) is measured. In measuring pulse wave information, since an absolute value of the pulse wave information has a large individual difference for each of the users as explained above, it is necessary to pay attention to scales of axes in graph representation. Specifically, it is undesirable that a displayable numerical value range is insufficient and a graph does not appropriately reflect numerical values or a numerical value range is too wide (an upper limit is too large) and therefore all graphs are displayed small and it is difficult to visually compare the graphs.

Therefore, in this explanation, normalization processing is performed using measurement results (levels in load states) acquired in the past and a graph after the normalization processing is displayed. Specific examples are shown in Figs. 20A to 20E. An absolute value of amplitude is used as a level of pulse wave information. However, the level of the pulse wave information is not limited to this.

First, it is assumed that a level in a first load state is 300. In this case, the normalization processing is performed using a value of 300, which is the level, in order to prevent a numerical value of 300 from being displayed excessively large (exceeding a displayable range) or being displayed excessively small. Specifically, an upper limit is set such that the height of a graph in the first load state is 80% of a display range. Specifically, since 300/0.8=375, a maximum value of an axis only has to be set to 375 (Fig. 20A).

Next, it is assumed that the first load state shifts to a second load state and amplitude of 800 is obtained in the state. In this case, 300 and 800 are compared and the normalization processing is performed using the larger value 800. Specifically, as in the first load state, 800/0.8=1000 only has to be set as a maximum value of an axis.

In this case, a value in the second load state is 80% of the display range. The value of 300 acquired in the first load state corresponds to a position of 30% with respect to the display range because the maximum value of the axis is 1000 (Fig. 20B). In the first embodiment, a relative relation among the levels in the load states only has to be visually presented (specific determination processing for the proper pressing force is explained below). Therefore, as it is seen when Figs. 20A and 20B are compared, even if absolute values are the same, the size of a displayed graph fluctuates according to a result of the normalization processing.

Subsequently, similarly, if a result in a third load state is 1300, the normalization processing is performed using 1300, which is a maximum value among three values. A graph is created with 1300/0.8=1625 set as a maximum value of an axis (Fig. 20C). Similarly, if a result in a fourth load state is 1400, the normalization processing is performed using 1400, which is a maximum value among four values. A graph is created with 1400/0.8=1750 set as a maximum of an axis (Fig. 20D).

If a result in a fifth load state is 1000, a maximum value among five values is 1300 corresponding to the fourth load state. Therefore, as the normalization processing, processing same as the processing in Fig. 20D is performed. A graph is created with 1300/0.8=1625 set as a maximum value of an axis (Fig. 20E).

Consequently, it is possible to absorb an individual difference of an absolute value of the pulse wave information. Irrespective of the pulse wave information of what kind of a value is obtained, it is possible to present a determination result (or an interim result of the determination) of the proper pressing force in a visually recognizable form.

Although the normalization processing is performed using the maximum value of the level in the example explained above, the normalization processing is not limited to this. Various modifications are possible to, for example, perform the normalization processing using both of an upper limit value and a lower limit value. When a graph is displayed on the display section 70, display of the ordinate or the abscissa or both the axes may be omitted. Consequently, visibility of a small screen is improved.

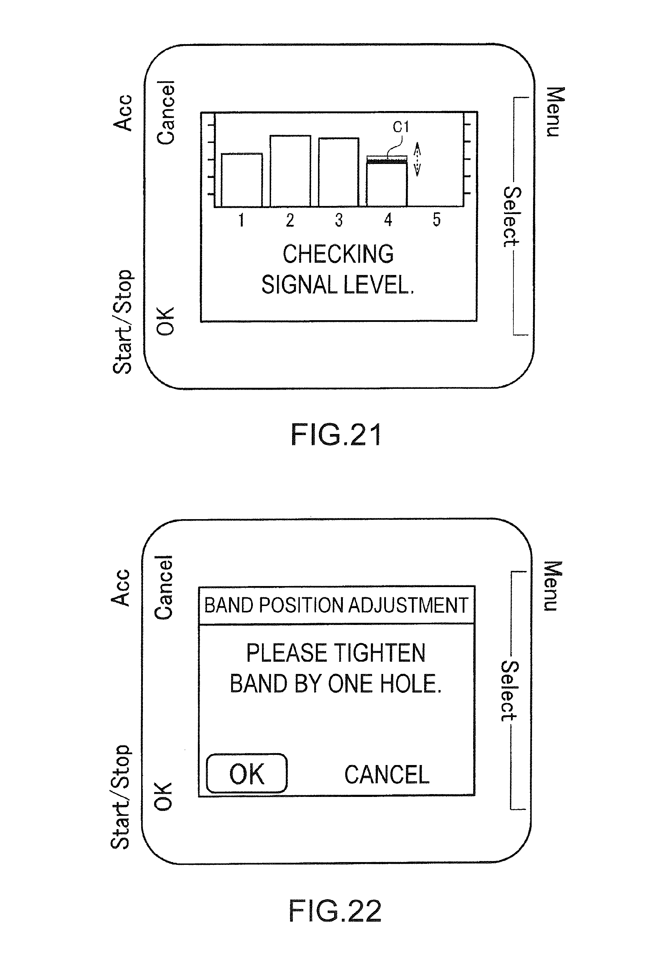

In Figs. 20A to 20E and the like, the pulse wave information being measured is not displayed. However, as shown in Fig. 21, a provisional result of a value currently being measured may be displayed.

An effect can be expected that the provisional result display notifies the user that measurement processing is also appropriately performed in the next load state to urge the user to wait or indicate that a failure or the like has not occurred. From this viewpoint, it is unnecessary to reflect a provisional result (e.g., a fluctuation state of an AC component signal) during measurement processing on display. Display unrelated to an actual signal value may be performed.

Further, since the processing section 100 executes measurement processing for pulse wave information, display may be performed using an actually acquired AC component signal. For example, graph representation moving up and down as indicated by C1 in Fig. 21 may be performed to correspond to an amplitude value of the pulse wave information that oscillates according to the elapse of time (arrows in the up down direction in Fig. 21 do not mean that such an image is displayed but indicate that a graph moves up and down in a C1 portion). Consequently, even before the end of the measurement processing, the user can estimate a rough measurement result by viewing a display image. Further, when a value clearly having low appropriateness (an extremely large value, an extremely small value, etc.) is displayed, for example, the user can doubt that an error has occurred and take measures.

### 4.3 Instruction image

In the first embodiment, the determination processing for the proper pressing force is performed by comparing measurement results of pulse wave information in a plurality of load states (pressing states). Therefore, when a measurement result in a given load state is obtained and an unmeasured load state is still present, it is necessary to shift to the unmeasured load state.

Therefore, in this explanation, when processing in the given load state ends, as shown in Fig. 22, an instruction image for urging the user to change a load state is displayed on the display section 70.

### 4.4 Final result image

When the measurement processing in all the load states ends or when an unmeasured load state is present but the measurement processing ends (the biological information detecting device may determine an end or may end the measurement processing according to an end instruction by the user), a determination result image of the proper pressing force is displayed.

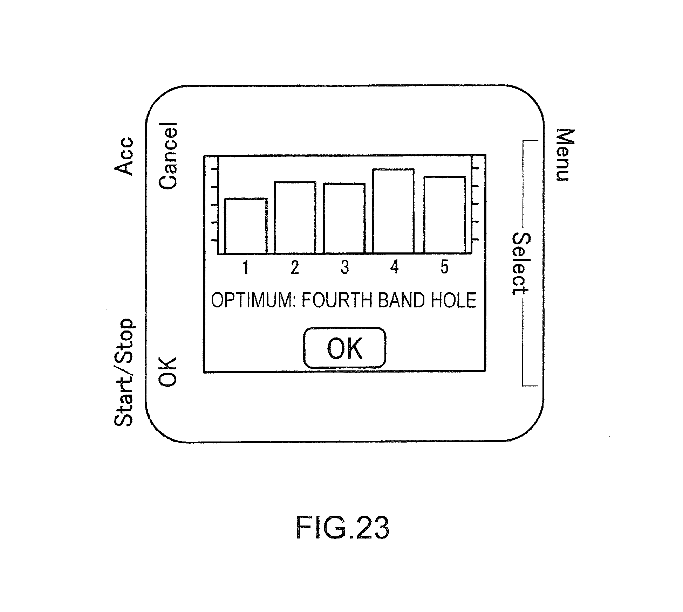

Specifically, an image shown in Fig. 23 or the like is displayed to inform the user of an optimum load state (band position). Like the graph shown in Fig. 20E, measurement results (levels) of pulse wave information in the load states may be displayed as a normalized graph. Consequently, it is possible to not only simply notify the optimum load state but also visually present a relative relation among the load states.

### 4.5 Example of screen transition

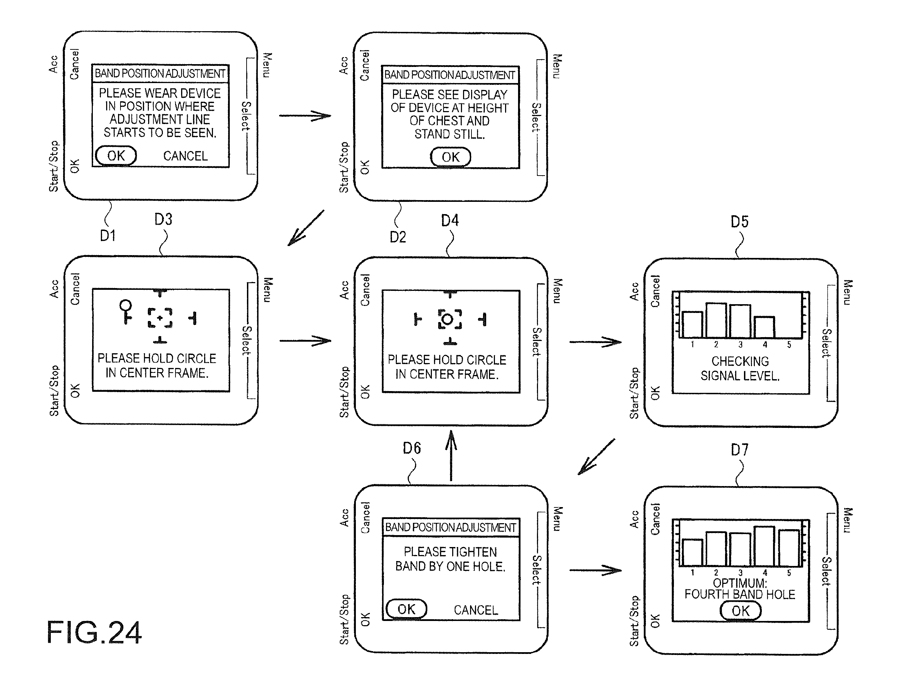

An example of screen transition is explained. When the determination processing for the proper pressing force is started, first, the biological information detecting device roughly adjusts a load state and a posture state. Specifically, the biological information detecting device displays a load state instruction image as indicated by D1 in Fig. 24. An adjustment line represents, for example, the display section 6135 shown in Fig. 11 or the positioning dies 6136. As explained above, even if the display section 6135 or the positioning dies 6136 are used, an individual difference of the proper pressing force cannot be dealt with. However, it is possible to determine a generally conceivable range of the proper pressing force. By displaying the image to instruct a load state, it is possible to skip processing in a state in which it is useless to perform the determination processing for the proper pressing force in the first place, for example, a state in which a pressing force is extremely small (e.g., the band is loose) and a state in which a pressing force is extremely large (e.g., the band is tight).

Subsequently, as indicated by D2 in Fig. 24, the biological information detecting device displays a posture state instruction image for instructing a rough posture state. If such an instruction is not given, the user can take an arbitrary posture. It is likely that it is difficult to effectively use the posture state notification image. Therefore, the biological information detecting device instructs the rough posture state using the posture state instruction image.

Subsequently, the biological information detecting device displays the posture state notification image. Specific explanation is omitted because the posture state notification image is explained above. When it is determined that a posture state indicated by D4 is appropriate, the biological information detecting device shifts to the measurement processing for the pulse wave information and displays an image shown in Fig. 21. In displaying the image, when it is determined that a posture state is an appropriate posture state (a state indicated by D4), the biological information detecting device may continue the display of the posture state notification image of D4 for a given wait time. If the processing section 100 determines that the posture state is appropriate, the biological information detecting device has shifted to the measurement processing for the pulse wave information. However, when the display of D4 ends instantaneously and shifts to display of D5, this is undesirable for the user because there is no time for sufficiently recognizing that the posture of the user is appropriate. Therefore, even if the posture state becomes appropriate the display section 70 may continue the display of the posture state notification image for the given wait time (e.g., 3 seconds) and notify the user that the present posture state is appropriate.

An image during the measurement processing for the pulse wave information is as explained above with reference to Fig. 21. However, it is also likely that the posture state becomes inappropriate during the measurement processing. In this case, even if the measurement processing is continued in the inappropriate posture state, accuracy of the determination processing for the proper pressing force is deteriorated. Therefore, in that case, it is desirable to once return to the display D4 of the posture state notification image and instruct the user to take the appropriate posture again. When the user returns to the appropriate posture state, the biological information detecting device resumes the measurement processing.

When the measurement processing ends, the biological information detecting device instructs a change in the load state as indicated by D6 and repeats the same processing in the load state after the change. Specifically, the biological information detecting device displays the posture state notification images D3 and D4 and, when a posture state is appropriate, displays an image of D5.

When the measurement processing ends, as explained above using Fig. 23, the biological information detecting device displays a final result image D7 including information concerning a load state for applying the proper pressing force. In an example of the displays D5 and D7, all measurement processing results for each of load states are displayed. However, the display of measurement processing results is not limited to this. For example, the biological information detecting device may be configured to, to clearly show a peak of pulse wave amplitude, display three measurement processing results having large amplitudes among changed load states on the displays D5 and D7 or display the immediately preceding measurement processing result and the measurement processing result of this time. That is, in the measurement processing carried out while changing the load state, the biological information detecting device only has to be capable of displaying an image including a measurement processing result having largest amplitude of a pulse wave.

### 5. Proper pressing force determination based on a pulse wave sensor signal

A specific example of proper pressing force determination performed in a proper posture when the user takes the proper posture according to the display of the posture state notification image is explained. It is assumed that an amplitude value of a below-mentioned AC component signal is displayed in the normalized graphs (Figs. 20A to 21).

### 5.1 Determination of the proper pressing force

As explained above, a signal value of a pulse wave sensor signal is different depending on a user. Therefore, a signal value in the case of the proper pressing force is relatively large for some users and is relatively small for other users. Therefore, even if only a signal value at the time of a given pressing force is acquired, it is difficult, based on the signal value, to determine whether the pressing force is the proper pressing force. For example, even if it is attempted to determine whether the pressing force is the proper pressing force according to comparison processing for a signal value and a given threshold, it is difficult to set a threshold that can be generally used for a plurality of users.

Therefore, in the first embodiment, a pressing force on a living organism is changed. The proper pressing force is determined based on a change characteristic of a pulse wave sensor signal with respect to the pressing force change. The magnitude of a signal value has an individual difference. This is because a change characteristic of the pulse wave sensor signal has the same tendency in any user. For example, when a discrete pressing force change is examined, first to Nth pressing forces different from one another are sequentially applied to a living organism and pulse wave sensor signals at the respective pressing forces (in a narrow sense, since one pulse wave sensor signal corresponds to one pressing force, first to Nth pulse wave sensor signals) are acquired. It only has to be determined on the basis of the acquired first to Nth pulse wave sensor signals which of the first to Nth pressing forces is the proper pressing force.

The proper pressing force has a certain degree of a range (a range satisfying V1<V<V2 explained above) in view of characteristics of the living organism. Therefore, a pressing force determined as the proper pressing force according to the first embodiment is not limited to one pressure value. The pressing force may have a plurality of values or may be represented by a given range. Consequently, the user can select a tightening position comfortable for the user within the range of the proper pressing force. Therefore, it is possible to reduce a sense of load on the user even when the user wears the biological information detecting device for a long time.

A pressing force is changed by the user. However, in order to realize the pressing force change, display control for, for example, displaying an instruction image (e.g., Fig. 22) on the display section 70 is performed. In this explanation, on the premise that the pressing force change is appropriately performed, determination processing based on an acquired pulse wave sensor signal is explained.

As explained above, in the first embodiment, processing is performed with the proper pressing force associated with a holding state of the load mechanism 300 rather than a physical quantity of a unit such as kPa. Therefore, in the following explanation, to simplify description, a pressing force is changed to determine the proper pressing force. However, the change in the pressing force is equivalent to a change in a load state (a holding state) in the load mechanism 300. The determination of the proper pressing force is equivalent to determination of a holding state for realizing the proper pressing force.

### 5.2 Proper pressing force determination based on an AC component signal

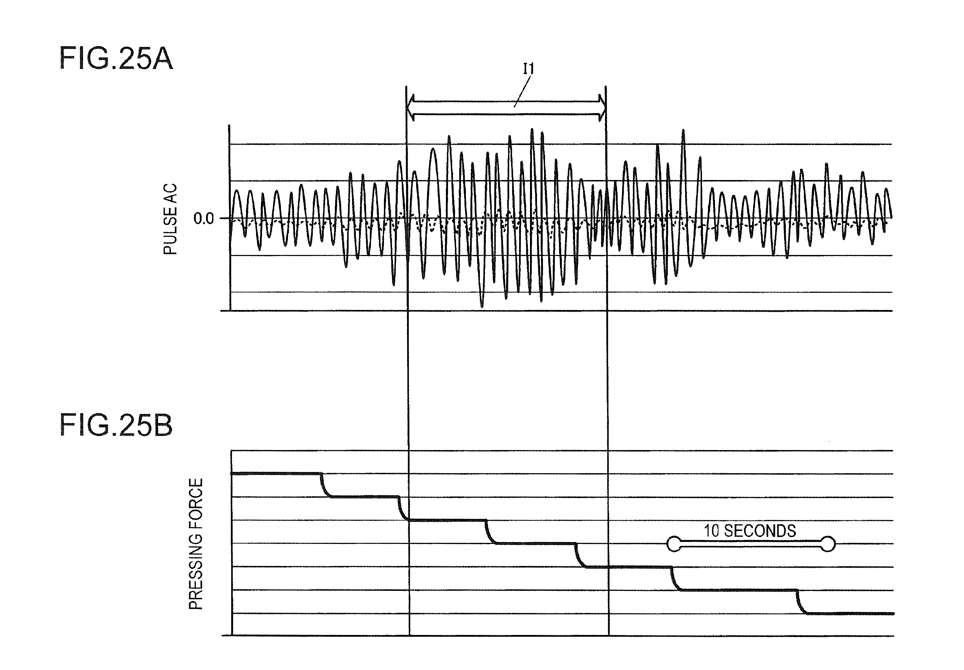

As specific determination processing, determination based on an AC component signal corresponding to an AC component of a pulse wave sensor signal is explained. A change characteristic of a signal value of the AC component signal with respect to a pressing force change is shown in Figs. 25A and 25B. Fig. 25A is a diagram for explaining a general change tendency of the AC component signal with respect to the pressing force. The abscissas of Figs. 25A and 25B represent time. As it is evident from a temporal change in the pressing force, Fig. 25B is a graph in a depressurizing direction in which the pressing force is reduced according to time.

As shown in Fig. 25A, the amplitude of the AC component signal is a small value when the pressing force is large. However, the amplitude value increases as the pressing force is reduced. When the pressing force is smaller than a given value, the amplitude value changes to a decreasing tendency. When it is taken into account that the AC component signal is a signal due to a heartbeat and is used for calculation of pulsation information, a pressing force having a large amplitude value of the AC component signal only has to be set as the proper pressing force. For example, in Fig. 25A, a range indicated by I1 is the proper pressing force.

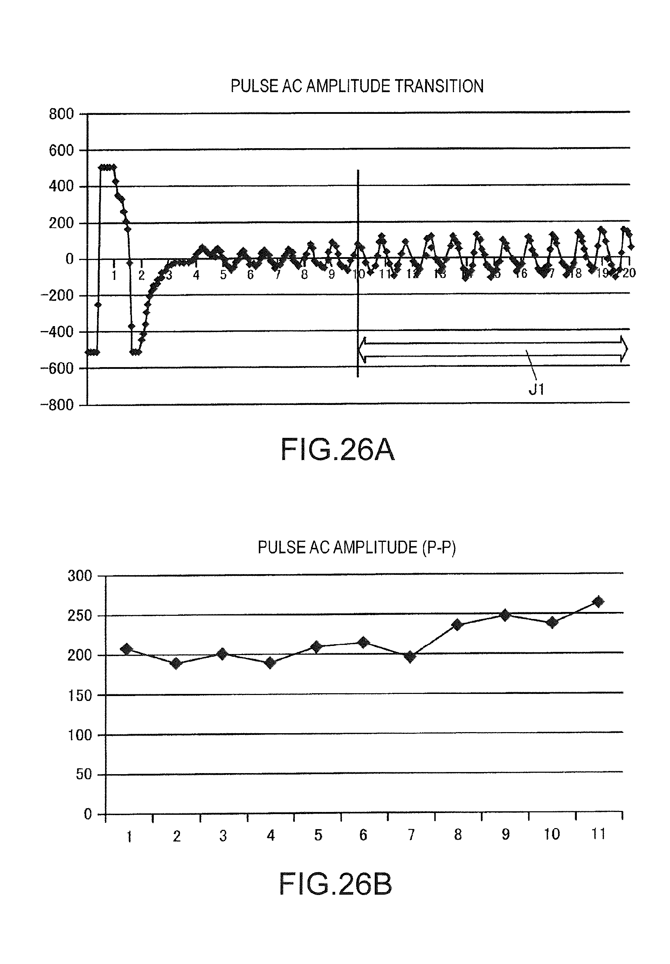

That is, when the AC component signal is used, it is sufficient that amplitude values at the pressing forces are calculated and a pressing force having a large calculated amplitude value is set as the proper pressing force. A specific example is shown in Figs. 26A and 26B. Fig. 26A shows a temporal change in the AC component signal that occurs when a band hole position is set in a given position. A unit of the abscissa is second. A former half portion (a period of 0 second to about 10 seconds) of Fig. 26A is timing when the band hole position is set and a period in which an elapsed time from the timing is short. Since a signal value of the AC component signal is unstable in this period, an amplitude value is not calculated. That is, the amplitude value is calculated on the basis of a signal value after a given time elapses after the band is mounted (e.g., a signal value in a period of J1 of Fig. 26A).

The amplitude value only has to be calculated on the basis of a peak. One of an upper peak and a lower peak may be used. However, in this explanation, the amplitude value is calculated from both of the upper peak and the lower peak. Specifically, a maximum value (the upper peak) and a minimum value (the lower peak) in one cycle of the AC component signal (corresponding to a motion of one beat of the heart) are detected. A difference value (peak to peak) between the maximum value and the minimum value is set as an amplitude value in the cycle. As indicated by J1 in Fig. 26A, it is assumed that a calculation period for the amplitude value is longer than one cycle of the AC component signal. Therefore, a plurality of difference values are acquired in the calculation period. Fig. 26B is an example of a temporal change in the difference values. Difference values acquired once at every one cycle of a pulsation are arranged in order of the acquisition (the abscissa represents acquisition order and is not a unit such as second). In the first embodiment, an average of the plurality of difference values in the amplitude value calculation period J1 only has to be set as an amplitude value in the set band hole position (and a pressing force corresponding to the band hole position).

The average may be a simple average or may be a trimmed mean calculated by excluding extremely large (or small) data. If the trimmed mean is used, an excluded range only has to be set from a standard deviation σ or the like. For example, 3σ only has to be used.

According to the processing explained above, an amplitude value of the AC component signal at a given pressing force (band hole position) can be calculated. In the determination of the proper pressing force, it is sufficient that amplitude values at the pressing forces are respectively calculated and a pressing force having a maximum amplitude value is set as the proper pressing force.

### 6. Details of the processing

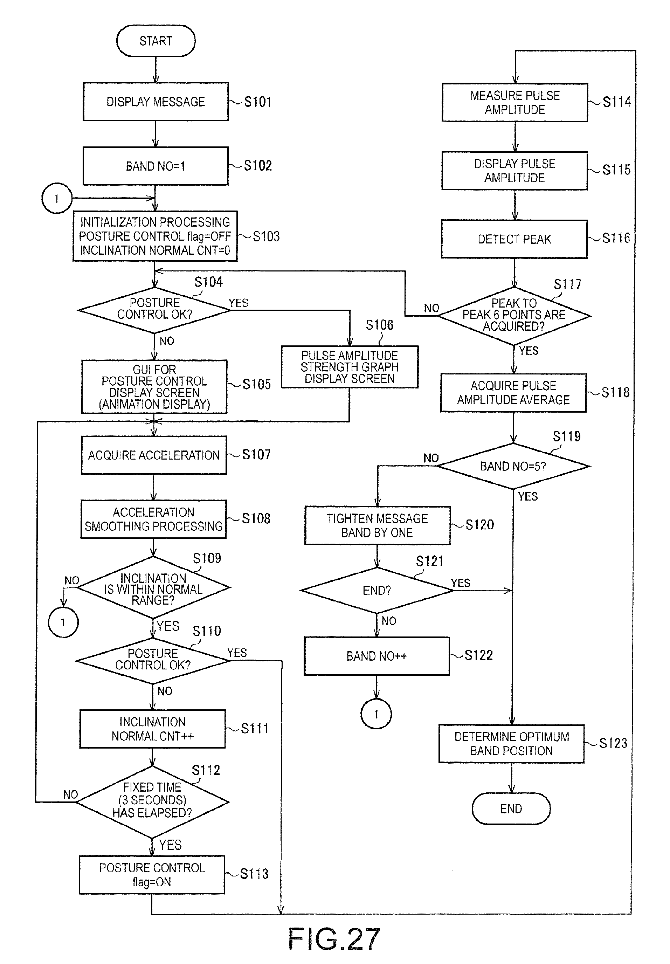

A flow of the determination processing for the proper pressing force in the first embodiment is explained with reference to a flowchart of Fig. 27. In Fig. 27, a biological information detecting device that can take five positions as load states (band positions) is assumed. However, the biological information detecting device is not limited to this.

When the processing is started, first, the biological information detecting device displays, on the display section 70, a message indicating that the determination processing for the proper pressing force is started and notifies the user of the processing start (S101). Alternatively, the display is not limited to display for simply notifying the start of the processing and may be display of an image for performing an instruction to limit a load state to some degree using the display section 6135 or the like as indicated by D1 in Fig. 24 or may be display of an image for performing an instruction to limit a posture state to some degree as indicated by D2 in Fig. 24. When the image of D1 is displayed and a load state is limited, it is likely that the measurement processing is not performed in all load states that the load mechanism 300 could take. However, that point is not taken into account in the flowchart of Fig. 27.

Subsequently, the biological information detecting device displays an instruction screen for setting a band position to 1 (the first load state) on the display section 70 (S102) and performs initialization processing (S103). The initialization processing is processing for resetting a posture control flag representing whether a posture state is appropriately maintained for a fixed period (setting the posture control flag to OFF) and processing for setting an inclination normal counter, which corresponds to time in which an appropriate posture is maintained, to 0.

The biological information detecting device determines a measurable posture change using the posture control flag (S104). If the posture control flag is OFF, the biological information detecting device displays the posture state notification image shown in Figs. 17A and 17B or the like (S105). When the posture control flag is ON in S104, this corresponds to a state in which an appropriate posture is maintained for the fixed period and a measurement result (and a provisional result) is acquired by processing in S114 and subsequent steps explained later. Therefore, the biological information detecting device displays a result screen shown in Fig. 21 (S106).

After the processing in S105 or S106, the biological information detecting device acquires an acceleration signal as information for determining a posture state (S107). The biological information detecting device performs smoothing processing (e.g., processing for calculating a moving average) for the acquired acceleration (S108) and determines whether the present posture state is appropriate on the basis of a result of the smoothing processing (S109). Specifically, the biological information detecting device only has to perform the processing using θx and θy or an acceleration value or the like acquired on the basis of θx and θy.

When it is determined in S109 that the posture state is inappropriate, the biological information detecting device returns to S103. That is, the posture control flag is set to OFF and the inclination normal counter is set to 0. According to the processing shown in Fig. 27, the appropriate posture state is maintained and, even after a shift to measurement processing in S114 and subsequent steps, if it is determined by the posture state determination in S109 that the posture state is not the appropriate posture state, the flag and the counter are reset and the biological information detecting device returns to the processing for taking the appropriate posture state again.

Subsequently, the biological information detecting device determines whether the posture control flag is ON (S110). When the posture control flag is ON, the biological information detecting device shifts to S114 and performs the measurement processing. That is, when the posture control flag is set to ON and the appropriate posture state is maintained thereafter, the measurement processing is performed by repeating loops of S104 to S110 and S114 to S117.

When it is determined that the posture control flag is OFF (NO in S110), this corresponds to a state in which, although the present posture state is appropriate, a predetermined period has not elapsed after the posture changed to the appropriate posture earlier (time enough for pulse wave information to stabilize has not elapsed). Therefore, the biological information detecting device increments the inclination normal counter representing duration of the appropriate posture state (S111) and compares a value of the counter with a specified time (3 seconds) to determine whether the pulse wave information is considered to stabilize (S112).

When it is determined that the pulse wave information is not considered to stabilize (NO in S112), the biological information detecting device returns to S107 and determines a posture state at the next timing. If the posture state is appropriate, a loop of S107 to S112 is repeated, whereby a value of the inclination normal counter increases.

When it is determined that the pulse wave information is considered to stabilize (YES in S112), the appropriate posture state is maintained for the fixed period and the pulse wave information changes to a state suitable for measurement. Therefore, the biological information detecting device sets the posture control flag to ON (S113) and shifts to the measurement processing.

As specific measurement processing, the biological information detecting device performs measurement of an amplitude value of the pulse wave signal (S114), display of a provisional result using the measured amplitude value (S115), and peak detection based on the amplitude value (S116). The biological information detecting device determines whether six upper peaks and six lower peaks are acquired (S117). When six upper peaks and six lower peaks are not acquired, the biological information detecting device returns to S104 and checks whether the appropriate posture state is maintained. As explained above, if the appropriate posture state is maintained, the biological information detecting device performs the graph representation in S106, determines that the posture control flag is ON (YES in S110), and returns to S114.

When six peak to peaks are acquired in S117, the biological information detecting device acquires an amplitude average from an average of the peak to peaks and ends the measurement processing in the load state (the band position) (S118).

Subsequently, the biological information detecting device determines whether the band position is 5 (the measurement ends in all the band positions) (S119). When it is determined that the band position is not 5 (NO in S119), the biological information detecting device displays the instruction screen shown in Fig. 22 and urges the user to update the band position (S120). Further, since it is likely that the processing is forcibly ended by the user, the biological information detecting device determines whether the user ends the processing (e.g., displays "end the processing?") on the display section 70 and determines on the basis of an input of the user responding to the display whether the user ends the processing) (S121). When the user ends the processing, the biological information detecting device determines an optimum band position (a load state for applying the proper pressing force) using results obtained to the end of the processing (S123). In determining whether the user ends the processing, it is desirable to display the image shown in Fig. 23 as well. When it is determined that the band position is 5 (the measurement ends in all the band positions) (YES in S119), the biological information detecting device also shifts to S123 and determines the optimum band position using all the results.

When it is determined that the user does not end the processing (NO in S121), the biological information detecting device updates the band state (S122), returns to S103, and continues the processing. S122 is update processing for band position information retained by the biological information detecting device and does not indicate whether the band position is actually manually changed by the user.

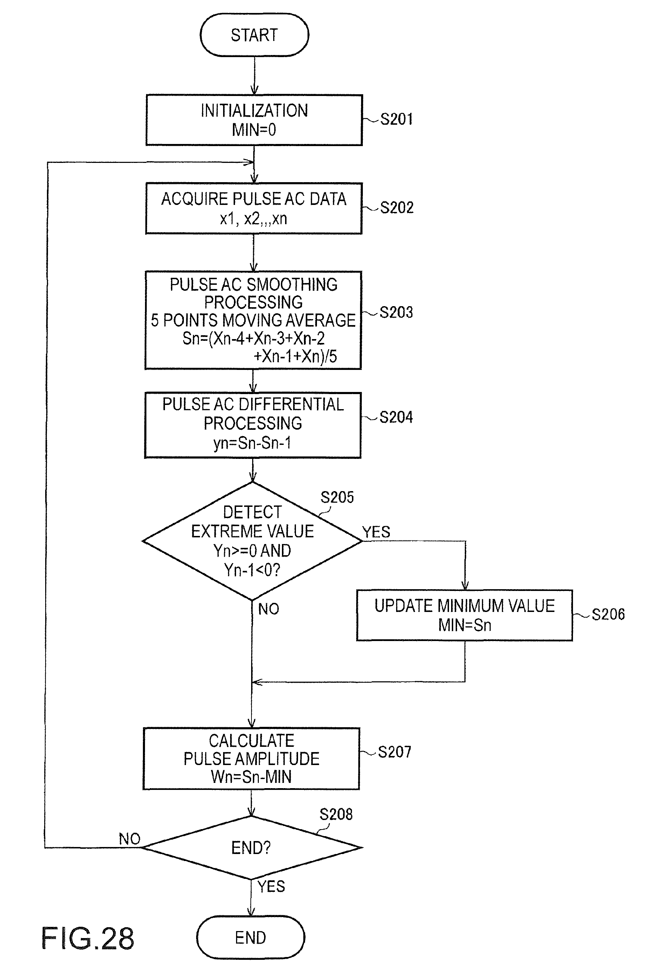

A specific example of the measurement processing in S114 to S118 is explained with reference to a flowchart of Fig. 28. When the processing is started, first, the biological information detecting device initializes a variable MIN representing a lower peak value to 0 (S201). The biological information detecting device acquires pulse wave information (specifically, AC component signals) at respective timings (S202) and calculates a moving average of latest five points to smooth the pulse wave information (S203).

The biological information detecting device applies differential processing to a result of the moving average (S204). The biological information detecting device performs processing for calculating a difference value of a moving average of adjacent two points. The biological information detecting device detects an extreme value using a result of the differential processing (S205). Specifically, the biological information detecting device detects a lower peak of the extreme value and performs processing with the lower peak set at a point where a differential value changes from negative to positive (including 0). When the extreme value is detected, the biological information detecting device performs processing with a value of the moving average set as a lower peak value (S206).

When the lower peak is not detected in S205 or after the update processing in S206, the biological information detecting device calculates a difference value Wn between the variable MIN at that point and the present value of the moving average and sets the difference value Wn as a value of pulse amplitude (S207). That is, a latest lower peak value is always retained and a difference between the lower peak value and the present value is set as the pulse amplitude.

Thereafter, the biological information detecting device determines whether the user ends the processing (S208). When it is determined that the user ends the processing (YES in S208), the biological information detecting device ends the processing. When the user does not end the processing (NO in S208), the biological information detecting device returns to S202.

### 7. Modifications

In the first embodiment, the biological information detecting device is configured to display the display image shown in Fig. 24 on the display section 70 of the device main body 2. However, the biological information detecting device is not limited to this. For example, the biological information detecting device may be configured to cause an external electronic device having an image display function such as a smart phone or a tablet electronic device to perform data communication with the device main body 2 on a real time basis and display the display image. By adopting such a configuration, it is possible to perform pressing force adjustment while viewing a larger screen. It is possible to improve convenience for the user. Since the external electronic device including the screen larger than the display section 70 of the device main body 2 is caused to display the display image, it is also possible to cause the external electronic device to display additional information as well. It is possible to feed back information useful for the user.

The device main body 2 is configured to include the display section 70. However, the device main body 2 is not limited to this and may be configured not to include the display section 70. In this case, as in the modification example 1 explained above, the biological information detecting device may be configured to perform data communication between the external electronic device and the device main body 2 on a real time basis and perform, on the external electronic device, display same as the display on the display section 70. The biological information detecting device may be configured to use a plurality of optical elements such as LEDs (Light Emitting Diodes) to notify the user of various kinds of information necessary for adjustment of a pressing force. For example, the biological information detecting device may be configured to give meanings to LEDs having specific colors and numbers or LEDs in specific positions to correspond to D1 to D7 shown in Fig. 24 and light the LEDs corresponding to a posture state of the user or a pulse wave measurement state. The biological information detecting device may be configured to change the luminance of the LEDs stepwise in order to display D3 and D4. For example, it is also possible to configure the biological information detecting device to gradually increase the luminance of the LEDs from the state of D3 to the state of D4. By configuring the biological information detecting device in this way, the size of the device main body 2 can be substantially reduced. Therefore, a sense of wearing of the user is remarkably improved. Even in an environment of use in which the user always wears the electronic device SP, it is possible to substantially reduce a load on the user.

In the first embodiment, the biological information detecting device includes, as shown in Fig. 5, the pulse-wave-information detecting section 10 configured to detect pulse wave information of the test subject, the display section 70, and the processing section 100 configured to perform the measurement processing for the pulse wave information and the discrimination processing for a posture state of the test subject. The display section 70 displays the posture state notification image that dynamically changes according to a change in a posture state. The processing section 100 performs, as the discrimination processing, processing for determining whether a posture state of the test subject is appropriate as a posture for performing the measurement processing for the pulse wave information and performs the measurement processing for the pulse wave information when it is determined that the posture state is appropriate.

Consequently, the biological information detecting device can display the posture state notification image and urge the user to take an appropriate posture. As a result, it is possible to improve accuracy of the measurement processing for the pulse wave information. The appropriate posture state may be, for example, the posture shown in Fig. 19A. As an example, a posture in which a water head pressure is a value close to a desired value, a stable posture in which fluctuation in pulse wave information due to an exercise or the like is suppressed, and the like are conceivable.

The display section 70 may display, as the posture state notification image, an image in which a display position of an object representing a posture state changes according to the posture state.

Consequently, since the change in the posture state is reflected on the change in the display position of the object, it is possible to give an instruction concerning a posture state to the user in an intuitively understandable form. A specific posture state display image may be the image shown in Figs. 17A and 17B or the like.

When the processing section 100 determines that the posture state is appropriate, the display section 70 may display, as the posture state notification image, an image in which the object is displayed in the first region of the image.

Consequently, it is possible to notify the user whether the present posture state is appropriate in an intuitively understandable form indicating whether the object is present in a given region. The position and the size of the first region in the display section 70 are arbitrary. However, the first region may be, for example, a region indicated by A2 in Fig. 17A.

The biological information detecting device is mounted on the wrist of the test subject as shown in Fig. 18A. A direction from the forearm to the hand of the test subject is represented as X axis. A direction orthogonal to the screen of the display section 70 and directed from the wrist of the test subject to the display section 70 side is represented as Z axis. An axis orthogonal to the X axis and the Z axis is represented as Y axis. When an angle representing rotation around the Y axis of the X axis during the discrimination processing with respect to a horizontal plane orthogonal to the gravity direction as shown in Figs. 18B and 18C is represented as first angle θx and an angle representing rotation around the X axis of the Y axis during the discrimination processing with respect to the horizontal plane is represented as second angle θy, the processing section 100 may determine that the posture state is appropriate when it is determined that the first angle θx is within a first angle range and the second angle θy is within a second angle range. In a narrow sense, the X axis, the Y axis, and the Z axis form a left-hand system as shown in Fig. 18A.

Consequently, it is possible to determine whether a posture state is appropriate on the basis of the rotation angles of the axes set as shown in Fig. 18A. By using the rotations of the axes, it is possible to specify the position (the height) in the vertical direction of the biological information detecting device, a twist of a mounting position (the arm), and the like. It is possible to set an appropriate posture state taking into account these elements. In actual determination processing, rather than directly using the angle values, as explained above, the angle ranges may be converted into numerical value ranges of sensor values of the acceleration sensor 21.

Concerning the second angle θy, when an angle representing rotation on the gravity direction side with respect to the horizontal plane is set as a positive value and an angle representing rotation on the opposite side of the gravity direction is set as a negative value as shown in Fig. 18B, the processing section 100 may determine that the posture state is appropriate when it is determined that the second angle θy is within the second angle range in which a median is a negative value.

Consequently, it is possible to increase possibility that a state in which θy is a negative value is determined as an appropriate posture state. As explained above, the rotation of the arm in a direction in which θy is a positive value is a motion for applying a twist in an ergonomically unnatural direction. Fluctuation is caused in a blood flow state by the twist. Therefore, by setting the angle range of θy wide in the negative direction, it is possible to reduce possibility of occurrence of a twist (reduce a degree of the twist even if the twist occurs) and accurately measure pulse wave information.

When the processing section 100 determines that the posture state is appropriate, the display section 70 may continuously display, as the posture state notification image, for a given wait time, an image in which the object is displayed in the first region.

Consequently, even when the posture state is appropriate, it is possible to continue the image display shown in Fig. 17B for a certain degree of time rather than immediately shifting to a display image shown in Fig. 21. Therefore, it is possible to clearly inform the user that the present posture state is appropriate. As a result, it is possible to smoothly perform maintenance of the appropriate posture state or subsequent adjustment of the posture state.

The display section 70 may display, after the elapse of the weight time, a measurement state notification image representing that the measurement processing for the pulse wave information is performed in the processing section 100.

Consequently, after the elapse of the wait time, it is possible to display the display image shown in Fig. 21 or the like and notify the user that the measurement processing for the pulse wave information is normally in progress.

When the processing section 100 determines during the display of the measurement state notification image after the elapse of the wait time that the posture state is inappropriate, the processing section 100 may end the measurement processing for the pulse wave information. The display section 70 may end the display of the measurement state notification image and perform display of the posture state notification image.

Consequently, even if the posture state is determined as appropriate and the measurement processing and the display of the measurement state notification image are started once, it is possible to determine whether the appropriate posture state is maintained. If the posture state is not the appropriate posture state any more, naturally, the state is not suitable for measurement of pulse wave information. Therefore, accurate processing cannot be performed even if the measurement processing is continued. Accordingly, the determination processing for a posture state is continued even during the measurement processing (this is equivalent to, for example, a flow for performing the processing in S104 to S110 after S117 in Fig. 27).

When the processing section 100 determines that the posture state is inappropriate, the display section 70 may display, as the posture state notification image, an image in which the object is displayed in the second region different from the first region in the image and may display, as the posture state notification image, an image in which a display form of the object is different when the object is displayed in the first region and when the object is displayed in the second region.

Consequently, as shown in Figs. 17A and 17B, it is possible to notify the user whether the posture state is the appropriate posture state according to the display position of the object and notify the user whether the posture state is the appropriate posture state according to a change in the display form of the object. Therefore, it is possible to notify the user of the posture state in a visually recognizable form.

The biological information detecting device may include the load mechanism 300 configured to generate a pressing force of the pulse-wave-information detecting section 10 on the test subject. A load state of the load mechanism 300 may be to any one of first to Nth (N is an integer equal to or larger than 2) load states having different states of pressing forces. The processing section 100 may perform the discrimination processing for a posture state in an ith (i is an integer satisfying 1≤i≤N) load state of the load mechanism 300. The display section 70 may display a posture state notification image that dynamically changes according to a change in the posture state in the ith load state.

Consequently, it is possible to generate a plurality of load states with the load mechanism (in a narrow sense, the band structure shown in Fig. 7) 300 and perform the processing in the respective load states.

When the processing section 100 determines that the posture state is appropriate as a result of the discrimination processing, the processing section 100 may perform the measurement processing for the pulse wave information in the ith load state. After the end of the measurement processing by the processing section 100, the display section 70 may display an instruction image for performing an instruction to change the load mechanism 300 to a jth (j is an integer satisfying 1≤j≤N, i≠j) load state different from the ith load state.

Consequently, when the measurement processing in a given load state ends, it is possible to display an image for instructing the user to change the load state as shown in Fig. 22. A pressing state changes according to the load state and, for example, accuracy of the measurement processing for the pulse wave information changes according to the change in the pressing state. Therefore, when the biological information detecting device can take a plurality of load states, it is useful to change the load state.

The processing section 100 may perform, on the basis of a result of the measurement processing for the pulse wave information acquired in at least two load states among the first to Nth load states, proper pressing force information acquisition processing for calculating proper pressing force information representing a proper value of a pressing force on the test subject.

Consequently, it is possible to estimate the proper pressing force from measurement results in a plurality of pressing states. Whereas since the estimation processing is performed using the proper pressing force having a large individual difference, actual pulse wave information of individuals, it is possible to obtain a result corresponding to the individual difference. A blood flow state changes because of various causes such as a water head pressure, an exercise state, a twist of the arm, and the like. However, since the posture state notification image is displayed to instruct the user to take a posture, it is possible to accurately perform the proper pressing force information acquisition processing.

The first embodiment can be applied to a biological information detecting device including the pulse-wave-information detecting section 10 configured to detect pulse wave information of a test subject, a presenting section configured to perform presentation of the information, and the processing section 100 configured to perform measurement processing of the pulse wave information and discrimination processing for a posture state of the test subject. The presenting section takes a different presentation form according to a change in the posture state to present information concerning the posture state. The processing section 100 performs processing for determining whether the posture state of the test subject is appropriate as a posture for performing the measurement processing for the pulse wave information and, when determining that the posture state is appropriate, performs the measurement processing for the pulse wave information.

The presenting section is not limited to the display section 70. For example, as explained in the modification, the presenting section may be configured by one or a plurality of optical elements (the optical elements are LEDs in a narrow sense but are not limited to the LEDs). In this case, it is conceivable that the presenting section takes a presentation form for emitting light in a first color (e.g., green) when the posture state is appropriate and takes a presentation form for emitting light in a second color (e.g., red) when the posture state is inappropriate. Consequently, when the presenting section is emitting light in red, the user adjusts the posture state such that the red light emission changes to green light emission. However, when convenience for the user is taken into account, it is preferable that it is possible to discriminate whether immediately preceding adjustment (an operation for changing the posture state) is a change in an appropriate direction or a change in a deteriorating direction. Therefore, the posture state may be divided into three or more states rather than two states and different colors may be respectively allocated to the states to improve a response of a change in the presentation form to a change in the posture state. A presentation state is not limited to the light emission color of the optical elements. The presentation state may be determined according to which of the plurality of light-emitting elements provided in different positions is emitting light. The presenting section is not limited to the presenting section configured by the optical elements. The presenting section may be a presenting section configured to generate sound or a presenting section configured to vibrate the device. In this case, it is conceivable that the change in the presentation state is performed according to a change in the volume of sound, a change in the pitch of sound or a change in vibration strength and vibration cycle, or the like. Other various modifications are possible for the presenting section.

Consequently, a method of urging the user to take an appropriate posture is not limited to the display section 70 configured to display the posture state notification image. It is possible to urge the user to take an appropriate posture using various methods. As a result, it is possible to improve accuracy of the measurement processing for the pulse wave information. Unlike when the display section 70 is used, when the LEDs or the like is used, even if the size of the presenting section is small, it is possible to improve visibility of a change in the presentation form corresponding to a change in the posture state. When sound or vibration is used, visibility is not a problem in the first place. Therefore, although a presentation mechanism corresponding to the presentation form such as the light-emitting elements or a device that generates sound or vibration is necessary, in general, it is possible to reduce the size of the biological information detecting device compared with the biological information detecting device including the display section 70. It is possible to reduce a wearing load on the user. In particular, since it is assumed that the biological information detecting device according to the first embodiment is continuously worn for a certain degree of time, the advantage of the reduction in the load on the user is significant.

When results of the measurement processing in the first to an ith (i is an integer satisfying 2≤i≤N) pressing states are acquired, results of the measurement processing in an i+1th pressing state to the Nth pressing state are not acquired, and the processing section 100 determines that the posture state is appropriate in the i+1th pressing state, the processing section 100 starts the measurement processing in the i+1th pressing state. Further, the display section 70 may display a first measurement result to a jth (j is an integer satisfying j≤i) measurement result included in the results of the measurement processing in the first pressing state to the ith pressing state and an provisional measurement result of the level of the pulse wave information in the i+1th pressing state during the measurement processing.

Consequently, after the update of the pressing state, it is possible to perform, after determining whether the posture state is appropriate, the measurement processing and the display of a provisional result when the posture state is the appropriate posture state. Therefore, even when the pressing state is updated, it is possible to perform the measurement processing using the appropriate posture state in the respective pressing states. Therefore, it is possible to improve accuracy of the measurement processing.

In the i+1th pressing state, when the processing section 100 determines that the posture state is appropriate, the display section 70 may continuously display, as the posture state notification image, for a given wait time, an image in which the object is displayed in a first region in the image corresponding to a state in which the posture state is appropriate.

When the processing section 100 acquires a result of the measurement processing in the i+1th pressing state, the display section 70 may display an instruction screen for performing an instruction to change the pressing state to an i+2th pressing state and, when the pressing state is changed to the i+2th pressing state according to the instruction screen, display the posture state notification image in the i+2th pressing state.

Consequently, after performing a change instruction for the pressing state using the image shown in Fig. 22 or the like, it is possible to start processing after the change in the pressing state. It is possible to, for example, smoothly update the pressing state.

The processing section 100 may perform, on the basis of the results of the measurement processing for the pulse wave information acquired in at least two pressing states among the first pressing state to the Nth pressing state, the proper pressing force information acquisition processing for calculating proper pressing force information representing a proper value of a pressing force on the test subject.

The first measurement result to the Mth measurement result may include at least a measurement result in which the level is the maximum among results of the measurement processing for the level of the pulse wave information in the first pressing state to the Nth pressing state.

Consequently, even when a part of the acquired measurement results is set as a display target, it is possible to perform effective display. In particular, in the first embodiment, the determination processing for the proper pressing force is assumed. Therefore, it is necessary to search for a pressing state in which the level of the pulse wave information is large (in a narrow sense, the maximum). That is, when a part of the acquired measurement results is used, it is possible to appropriately notify the user of a determination state of the proper pressing force by using a maximum value of the measurement results.

In the first embodiment explained above, the biological information detecting device includes, as shown in Fig. 5, the pulse-wave-information detecting section 10 configured to detect pulse wave information of a test subject, the processing section 100 configured to perform the measurement processing for the level of the pulse wave information at the time when a pressing force of the pulse-wave-information detecting section 10 on the test subject is in each of the first pressing state to the Nth (N is an integer equal to or larger than 2) pressing state, and the display section 70 configured to display the first measurement result to the Mth (M is an integer satisfying M≤N) measurement result among results of the measurement processing for the level of the pulse wave information in the first pressing state to the Nth pressing state.

The level of pulse wave information may be an amplitude value of pulse wave information or may be, for example, power at a frequency subjected to the Fourier transform or the like.

In the explanation in the first embodiment, typically, all the load states that the load mechanism 300 can take are selected one by one, measurement results are acquired in the pressing states corresponding to the load states, and all the measurement results are displayed. That is, when the load mechanism 300 takes the first to the Nth load states, whereby the pressing force of the pulse-wave-information detecting section 10 on the test subject is in the first pressing state to the Nth pressing state to correspond to the first to the Nth load states, the first to the Nth measurement results are acquired and all the measurement results are set as display targets on the display section 70. However, the display targets are not limited to this. A part of the acquired first to Nth measurement results may be set as the display targets. The first to the Mth measurement results set as the display targets do not mean that the order of acquisition of results of the measurement processing is not first to Mth and mean that arbitrary M results among results acquired in N pressing states can be set as the display targets. In the following explanation of this specification, as explained above, the first measurement result to the Mth measurement result among results of the measurement processing in the first pressing state to the Nth pressing state are displayed. However, when M=N, it goes without saying that the above explanation applies.

Consequently, in the biological information detecting device that can take a plurality of pressing states, it is possible to display a plurality of measurement results corresponding to the plurality of pressing states. In the processing for calculating the proper pressing force, it is necessary to compare pulse wave information actually measured in the plurality of pressing states in order to deal with the individual difference. Even in the case other than the determination of the proper pressing force, it is useful to display a plurality of measurement results if it is taken into account that a pressing state affects, for example, detection accuracy of pulse wave information. Therefore, in the first embodiment, the first measurement result to the Mth measurement result are displayed.

The display section 70 may display the first measurement result to the Mth measurement result subjected to normalization processing for the level of the pulse wave information.

Consequently, it is possible to display, for example, the image shown in the upper part of Fig. 23. An absolute value of the pulse wave information is likely to be substantially different for each of the users. It is undesirable to determine a scale (e.g., an upper limit and a lower limit of an axis of a graph) as a general value. Specifically, differences among a plurality of measurement results are not clarified because a value of measured pulse wave information exceeds a displayable maximum value or a value of pulse wave information is extremely small compared with a range of a graph. Therefore, in the first embodiment, the normalization processing shown in Figs. 20A to 20E or the like is performed to enable appropriate display of a measurement result of pulse wave information having a large individual difference.

The display section 70 may display the first measurement result to the Mth measurement result subjected to the normalization processing according to a maximum value of the level among the first measurement result to the Mth measurement result.

Consequently, it is possible to perform the normalization processing using the maximum value of the level. When a measurement result of the pulse wave information exceeds a displayable value, a displayed value does not reflect the measurement result. It is highly likely that the user is caused to misrecognize the measurement result. Therefore, it is necessary to prevent the measurement result of the pulse wave information from exceeding the displayable value. In particular, when processing for calculating the proper pressing force using the method in the first embodiment is performed, it is assumed that a determination method for determining that a pressing state having a large level is the proper pressing force. Therefore, it is useful to normalize the measurement result with the maximum value of the level and appropriately display the maximum value. However, the use of a minimum value and other values in the normalization processing is not prevented.

When results of the measurement processing for the level of the pulse wave information in the first pressing state to the ith (i is an integer satisfying 2≤i≤N) pressing state are acquired and results of the measurement processing for the level of the pulse wave information in the i+1th pressing state to the Nth pressing state are not acquired, the display section 70 may perform the normalization processing with a maximum value of a level among the first measurement result to the jth (j is an integer satisfying j≤i) measurement result included in results of the measurement processing for the level of the pulse wave information in the first pressing state to the ith pressing state and display the first measurement result to the jth measurement result subjected to the normalization processing.

Consequently, even when the measurement processing ends only for a part of a plurality of pressing states, it is possible to display results only for the part of the processing states. Since a certain degree of time is required for the measurement processing for the pulse wave information, it is assumed that, for example, the user sometimes desires to end the processing halfway because of convenience of time or the like. For example, when the user desires to learn a pressing state that gives the maximum value of the level (e.g., calculate the proper pressing force), if a result that the level rises until a given pressing state and thereafter changes to a decrease is obtained, it is possible to estimate at that point in time that a point that gives an extreme value is the pressing state to be calculated. That is, by displaying an interim result as appropriate, it is possible to perform, for example, determination by the user based on the displayed result and realize a more user-friendly device. The interim result is not limited to display of all measurement results acquired to that point. A part of the measurement results may be set as display targets.

The display section 70 may display an instruction screen for performing an instruction to change the pressing state to the i+1th pressing state. When the processing section 100 starts the measurement processing for the level of the pulse wave information in the i+1th pressing state, the display section 70 may display a provisional measurement result of the level of the pulse wave information in the i+1th pressing state during the measurement processing together with the first measurement result to the jth measurement result.

When displaying the provisional measurement result, the display section 70 may perform, as the display of the provisional measurement result, animation display that fluctuates in response to fluctuation in the pulse wave information in the i+1th pressing state.

Consequently, the instruction screen can be displayed. Therefore, it is possible to, for example, update the pressing state and acquire measurement results in a large number of pressing states and display a provisional measurement result corresponding to the pressing state being measured. By displaying the provisional measurement result, it is possible to clearly indicate to the user that the measurement processing is executed in the present pressing state. When displaying the provisional measurement result, as shown in Fig. 21, the display is changed using fluctuation in actual pulse wave information. Consequently, even before the end of the measurement processing, it is possible to estimate, to a certain degree, a measurement result corresponding to the pressing state at that point. When an unexpected value (an extremely large value, an extremely small value, etc.) is acquired, it is possible to, for example, take possibility of an error or the like into account.

The processing section 100 may perform, in each of the first pressing state to the Nth pressing state, the discrimination processing for determining whether the posture state of the test subject is appropriate as a posture for performing the measurement processing for the pulse wave information. The display section 70 may display, in each of the first pressing state to the Nth pressing state, the posture state notification image that dynamically changes according to a change in the posture state.

### Second Embodiment

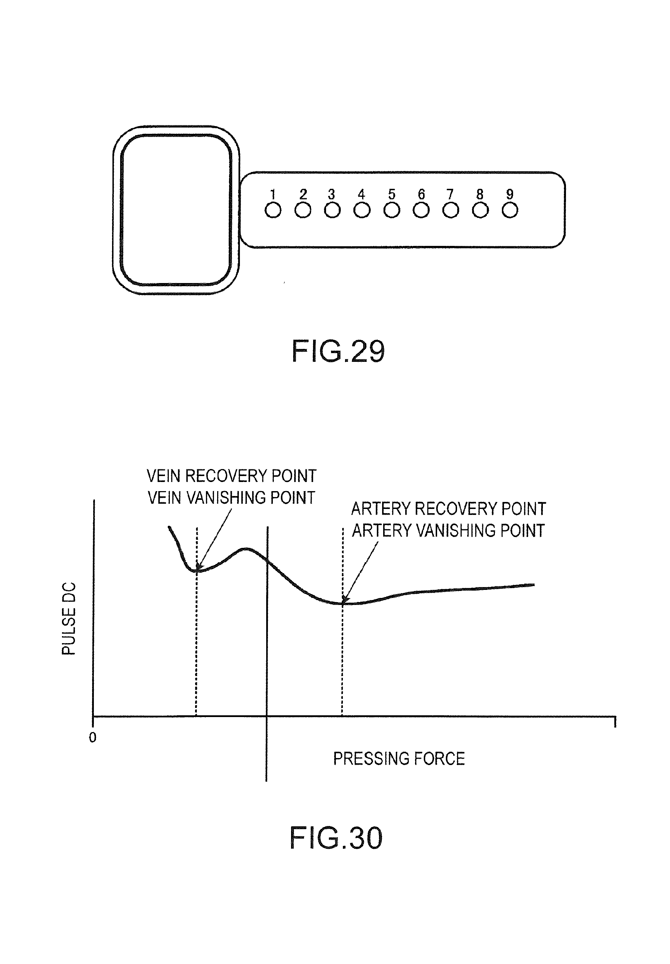

A second embodiment of the invention is explained with reference to Figs. 29 to 42. In the following explanation, components same as the components explained above are denoted by the same reference numerals and signs and explanation of the components is omitted.

In the second embodiment, a heart rate meter is a watch-type device including a band functioning as the holding mechanism 300 shown in Fig. 4A. A plurality of holes are provided in the band as shown in Fig. 29. It is assumed that a pressing force can be changed according to which of the holes is used to hold the heart rate meter. However, the configuration of the holding mechanism 300 is not limited to this.

A pressing force is changed by the user. However, in order to realize the pressing force change, display control for, for example, displaying an instruction screen on the display section 70 is performed. A specific interaction based on the display control or the like is explained below. In this explanation, on the premise that the pressing force change is appropriately performed, determination processing based on an acquired pulse wave sensor signal is explained. For improvement of determination accuracy, it is necessary to set an appropriate environment for determination. The environment setting is also performed by the display control for the instruction screen or the like. Details of the environment setting are explained below. Like the pressing force change, it is assumed that appropriate environment for determination is set.

As explained above, in the second embodiment, processing is performed with the proper pressing force associated with a holding state of the load mechanism 300 rather than a physical quantity of a unit such as kPa. Therefore, in the following explanation, to simplify description, a pressing force is changed to determine the proper pressing force. However, the change in the pressing force is equivalent to a change in a holding state in the load mechanism 300. The determination of the proper pressing force is equivalent to determination of a holding state for realizing the proper pressing force.

### 8. 1 Proper pressing force determination based on a DC component signal

In the second embodiment, determination based on a DC component signal corresponding to a DC component of a pulse wave sensor signal is explained.

A change characteristic of a signal value of the DC component signal with respect to a pressing force change is shown in Fig. 30. Fig. 30 is a diagram for explaining a general change tendency of the DC component signal with respect to a pressing force. Unlike Figs. 25A and 25B, the abscissa of Fig. 30 represents the pressing force.

As shown in Fig. 30, in a change characteristic curve of the DC component signal with respect to the pressing force change, both of a pressing force corresponding to a vein vanishing point and a pressing force corresponding to an artery vanishing point are inflection points. That is, by detecting the inflection points of the DC component signal, it is possible to detect the pressing force corresponding to the vein vanishing point and the pressing force corresponding to the artery vanishing point. As explained above, the proper pressing force only has to be pressure larger than the pressing force at the vein vanishing point and smaller than the pressing force at the artery vanishing point. Therefore, the proper pressing force only has to be determined in a range satisfying this condition. In the pressure range satisfying the condition, one pressure value may be set as the proper pressing force. For example, an average of the pressing force at the vein vanishing point and the pressing force at the artery vanishing point may be set as the proper pressing force.

Various detecting methods for the inflection points of the change characteristic curve of the DC component signal are conceivable. For example, as shown in Fig. 25B, when a pressing force changes as time elapses, a temporal change curve of the DC component signal corresponding to the pressing force change (i.e., a graph with the abscissa representing time and the ordinate representing a signal value of the DC component signal) also represents a change characteristic of the DC component signal corresponding to the pressing force change. Therefore, inflection points of the curve may be detected. With this method, since signal values of the DC component signal acquired at respective times can be directly used, preprocessing or the like is unnecessary.

On the other hand, in the temporal change curve, since the abscissa of the graph represents the time and does not represent the pressing force, when a change in the abscissa direction is considered, the change does not correspond to a linear change in the pressing force. For example, when a pressing force in a period of time T1 to time T2 is considered, it is less likely that the pressing force changes with a fixed gradient. It is conceivable that the pressing force in the period is invariable or the pressing force excessively changes to be a first pressing force in T1 to T3 (T3<T2) and a second pressing force in T3 to T2. That is, it is expected that the change characteristic to the pressing force markedly appears when a pressing force change curve shown in Fig. 30 (a graph with the abscissa representing the pressing force and the ordinate representing the signal value of the DC component signal) is used rather than the temporal change curve. As explained concerning the AC component signal, it is conceivable that a signal value does not stabilize for a given time after the band is worn. However, in the temporal change curve, since such an unstable signal value directly appears, it is likely that processing is adversely affected.

Therefore, in the second embodiment, when a given pressing force is set, one representative value is calculated on the basis of the DC component signal at the pressing force. By performing the processing at a plurality of pressing forces, it is possible to acquire a graph (the pressing force change curve) representing a corresponding relation between the pressing force and the DC component signal. Therefore, inflection points in the acquired graph only have to be detected.

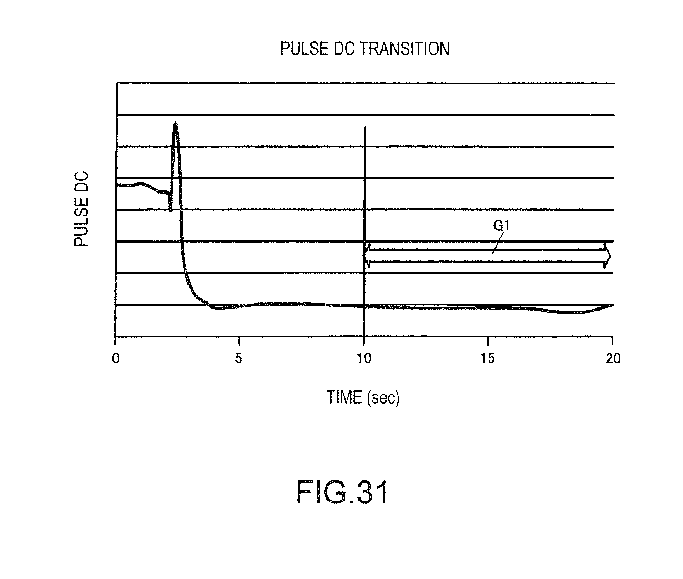

A method of calculating a representative value when the given pressing force is set is explained with reference to Fig. 31. Fig. 31 represents a temporal change in the DC component signal at the time when a band hole position is set in a given position. As in Fig. 26A, a former half portion of Fig. 31 is timing when the band hole position is set and a period in which an elapsed time from the timing is short. Since a signal value of the DC component signal is unstable in this period, a representative value is not calculated. The subsequent period indicated by G1 is used. Various method of calculating a representative value are conceivable. For example, an average of signal values of the DC component signal in a calculation section (Gl in Fig. 31) only has to be calculated. As in the processing in the AC component signal, the average may be a simple average or a trimmed mean calculated by excluding extremely large (or small) data in the average calculation. By calculating representative values at the respective pressing forces in this way, it is possible to calculate the pressing force change curve of the DC component signal.

Detection of inflection points from a curve is a mathematically important field. There are many existing arts concerning inflection point detection in a computer system and the like. In the second embodiment, an arbitrary method among the related arts can be used. Therefore, detailed explanation concerning a detection method for inflection points is omitted.

### 8.2 Proper pressing force determination based on the AC component signal and the DC component signal

Proper pressing force determination in the second embodiment is not limited to processing based on one of the AC component signal and the DC component signal and may be processing performed using both of the signals.

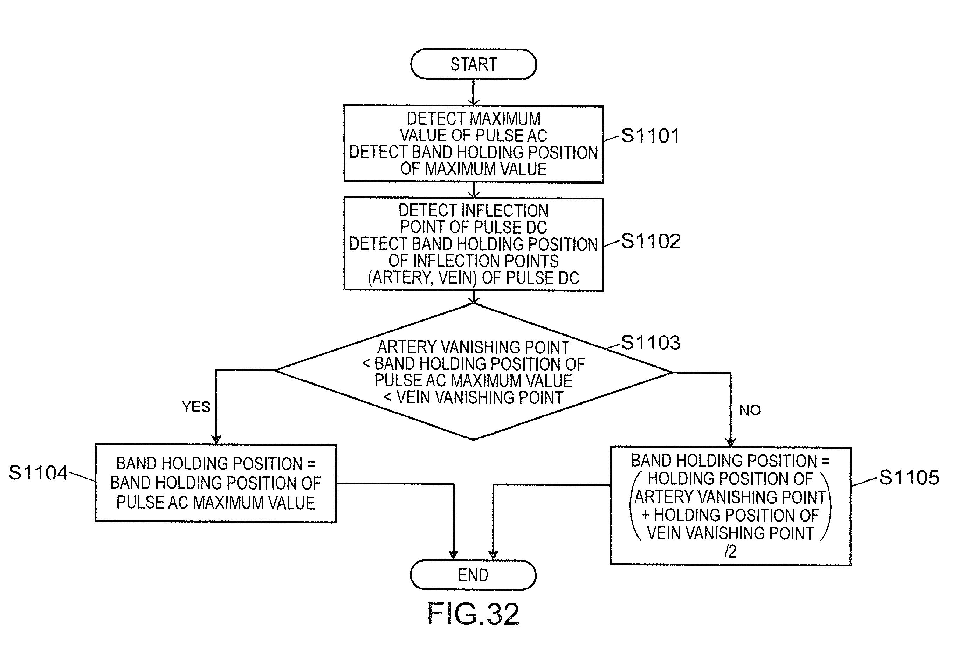

A specific example is shown in a flowchart of Fig. 32. When this processing is started, first, the heart rate meter calculates a pressing force VA having a maximum amplitude value on the basis of the AC component signal (S1101). The heart rate meter performs inflection point detection for the DC component signal and calculates a pressing force VD1 at a vein vanishing point and a pressing force VD2 at an artery vanishing point (S1102). Specific processing in S1101 and S1102 is as explained above.

Thereafter, the heart rate meter determines whether a relation of VD1<VA<VD2 holds (S1103). When it is determined that the relation holds (YES in S1103), since the pressing force VA is included in a proper range calculated from the DC component signal, the heart rate meter sets the pressing force VA as the proper pressing force assuming that reliability of the pressing force VA is secured (S1104). On the other hand, when it is determined that the relation does not hold (NO in S1103), since the reliability of the pressing force VA is unconvincing, the heart rate meter determines the proper pressing force on the basis of the pressing force VD1 and the pressing force VD2 calculated from the DC component signal (S1105 Specifically, an average of the pressing force VD1 and the pressing force VD2 only has to be set as the proper pressing force.

Consequently, it is possible to perform proper pressing force determination based on both of the AC component signal and the DC component signal. For example, improvement of accuracy of the determined proper pressing force can be expected. The processing based on both of the AC component signal and the DC component signal is not limited to the processing shown in the flowchart of Fig. 32. Other processing may be performed.

### 8. 3 Display control method

The user changes the pressing force in the proper pressing force determination by adjusting the holding mechanism 300 (e.g., adjusting the band hole position). However, it is undesirable for the user to grasp all procedures of the proper pressing force determination because a load on the user is large. Therefore, in the second embodiment, the heart rate meter and the user interact with each other using an interface such as the display section 70. A system (the heart rate meter) gives an appropriate instruction to the user to attain a reduction in the load on the user.

In the proper pressing force determination, fluctuation in a signal value due to a cause other than a pressing force change is undesirable because determination accuracy is deteriorated. Therefore, in order to suppress the undesirable fluctuation in the signal value, it is determined whether given conditions for an environment for determination is satisfied. The proper pressing force determination is performed when it is determined that the condition is satisfied. When the condition is not satisfied, some instruction for the user is necessary. Therefore, it is desirable to use the interface such as the display section 70.

The interaction between the user and the system in the second embodiment and display control and the like performed in the interaction are explained with reference to a flow of typical processing in the proper pressing force determination.

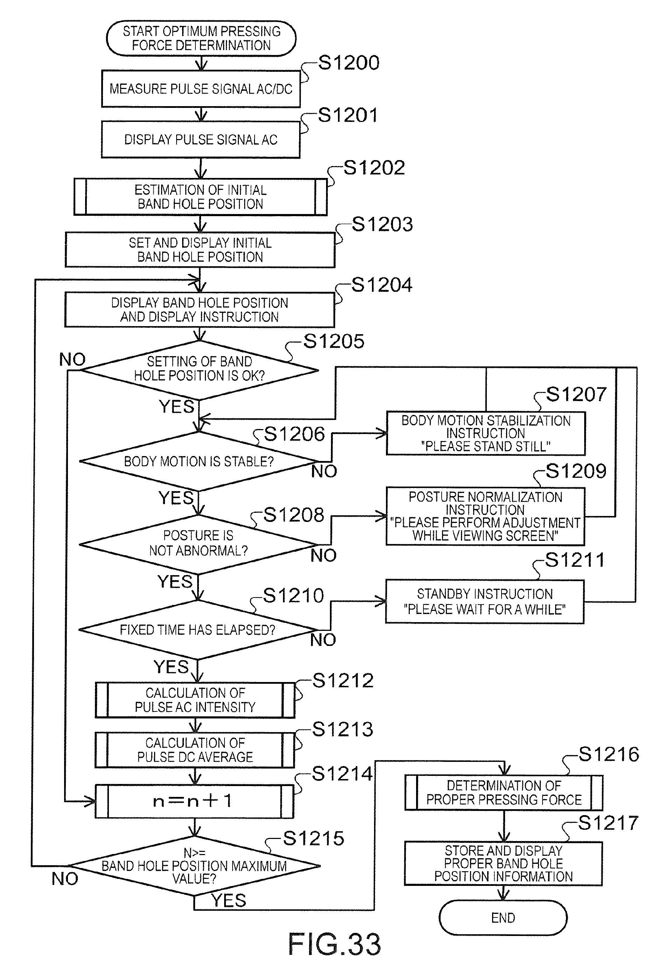

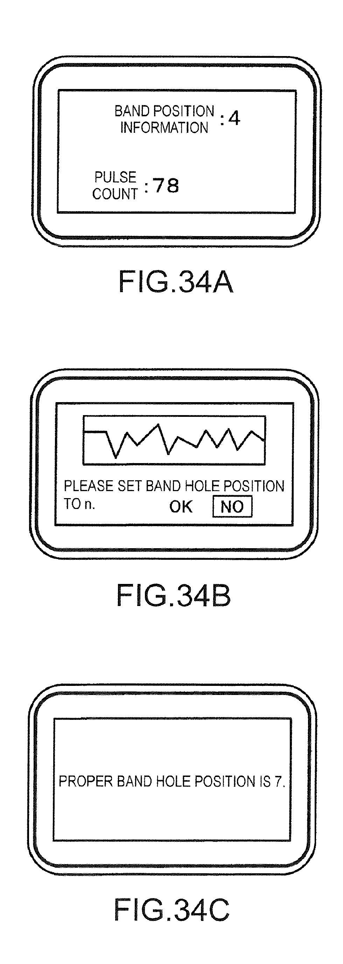

A flowchart for explaining the processing of the proper pressing force determination is shown in Fig. 33. When the processing is started, first, the heart rate meter measures the AC component signal and the DC component signal (S1200). As shown in an upper part of Fig. 34B, the heart rate meter displays a waveform or the like of the AC component signal on the display section 70 (S1201). The display in S1201 is not essential. However, it is possible to notify the user that a signal can be appropriately acquired by the pulse wave sensor 11. Therefore, it is possible to give a sense of security to the user to assure that there is no failure or the like in the device.

Subsequently, the heart rate meter estimates an initial value of the band hole position suitable for the user (S1202). Specific contents of processing for the estimation are explained below.

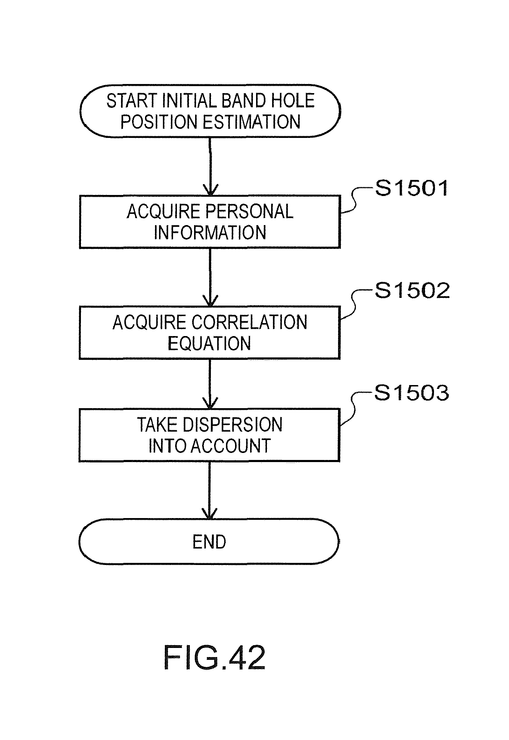

Fig. 42 is a flowchart for explaining processing for initial holding state estimation. In the processing, first, the heart rate meter acquires personal information input by the external I/F section 80 and stored in the storing section 90 and sets the personal information as a constant (S1501). In an example explained below, three constants are used: X1=BMI (Body Mass Index) calculated from height and weight, X2=sex, and X3=age. A calculation method for BMI is not explained because the calculation method is a method by publicly-known means.

Subsequently, the heart rate meter acquires a correlation equation stored in the storing section 90. The heart rate meter calculates an initial band hole position α optimum for each individual by performing calculation of the correlation equation using the three constants of BMI, sex, and age (S1502).

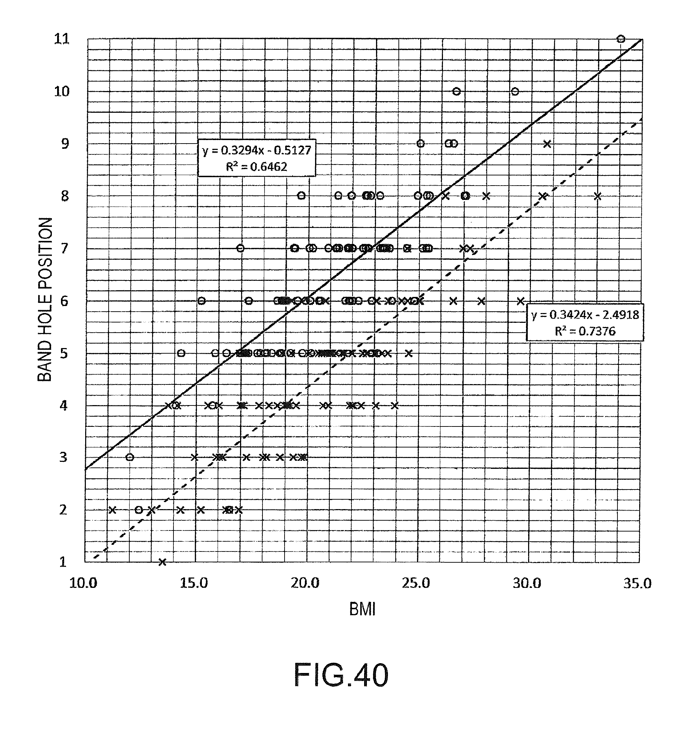

Fig. 40 is a diagram showing a correlation between the personal information (BMI, sex, and age) of the user and the band hole position. In the figure, circles indicate a relation between BMIs of males in the age of 20 to 24 and the optimum band hole position and crosses indicate a relation between BMIs of females in the age of 20 to 24 and the optimum band hole position. In the figure, a solid line is obtained by linearly approximating the relation between the BMIs of males in the age of 20 to 24 and the optimum band hole position. A dotted line is obtained by linearly approximating the relation between the BMIs of females in the age of 20 to 24 and the optimum band hole position. A correlation between the three constants of BMI, sex, and age obtained by performing, for example, a statistical survey beforehand and a proper band hole position obtained by determination of an optimum pressing force is calculated and the correlation is subjected to polynomial (a function having a first-order or higher-order variable) approximation to obtain the correlation equation. The correlation equation is stored in the storing section 90. The statistical survey or the like is necessary beforehand to obtain the correlation shown in Fig. 40. However, in this explanation, Fig. 40 is a simulation result obtained by generating random numbers on the basis of data of human body dimension averages of the Ministry of Economy, Trade and Industry.

In processing of optimum pressing force determination explained below, when second to Nth pressing force adjustment directions are a depressurizing direction (i.e., a direction in which the band hole position is loosened), by instructing the user to set the initial band hole position α tight in advance, possibility of adjustment to an optimum pressing force is improved. As a result, it is possible to improve convenience for the user.

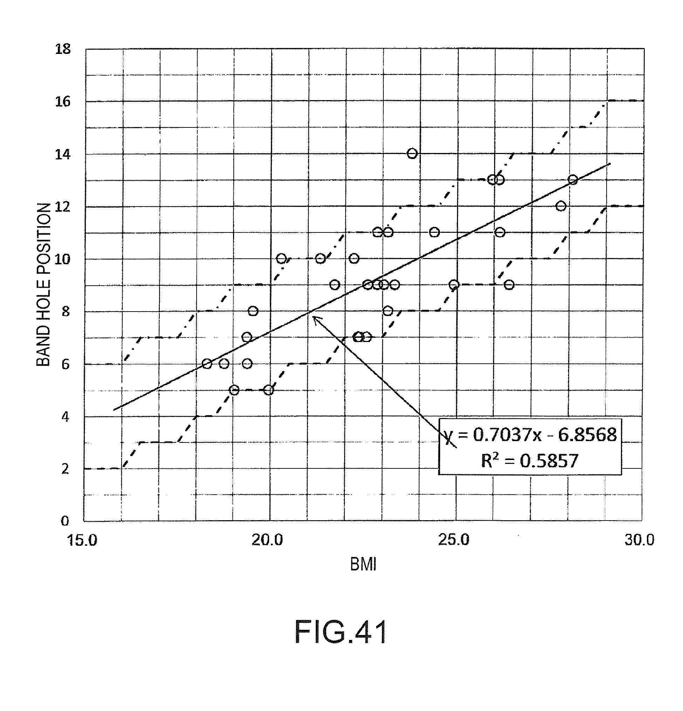

Fig. 41 is an example of deviation (dispersion) between estimated band hole positions and an actual optimum band hole position. In the figure, circles indicate a relation between BMIs of males in the age of 21 to 54 and the optimum band hole position. A solid line is obtained by linearly approximating the relation between the BMIs of males in the age of 21 to 54 and the optimum band hole position. In the figure, an alternate long and short dash line indicates an example in which the estimated initial band hole position α is incremented by 2 (the band is loosened by two band holes). A dotted line indicates an example in which the estimated initial band hole position α is decremented by 2 (the band is tightened by two band holes). Fig. 41 is actual measurement data obtained by using the heart rate meter in the second embodiment in thirty samples and males in the age of 21 to 45.

A scale instructed to the user to set tight in advance as explained above can be determined from the correlation equation, a scale σ of deviation of an actual value obtained by statistics, and a hole interval d of band holes (a band hole pitch). For example, an optimum pressing force can be obtained in most people (3σ) by instructing the user to set the scale tight by the number of band holes obtained by conversion into an integer of 3σ/d. As a calculation method for the scale σ of the deviation of the actual value obtained by statistics, there are a plurality of methods by publicly-known statistical methods. An arbitrary method only has to be used. Therefore, explanation of the calculation method is omitted.

Consequently, the user can determine the proper pressing force with the number of times of determination smaller than the number of times of determination for determining whether pressing forces are the proper pressing force concerning all the holding states. Therefore, convenience for the user is improved.

However, the personal information used in calculating the initial band hole position α does not need to be limited to the three variable constants of BMI, sex, and age and may be, for example, the circumference of a region where the device is held or body fat percentage.

The correlation equation may be obtained by subjecting data by an external large database extremely larger than the data by the statistical survey or the like to the polynomial approximation and stored in the storing section 90 through communication means such as radio communication.

The correlation equation may be obtained by subjecting data by an external large database extremely larger than the data by the statistical survey or the like to the polynomial approximation. The personal information stored in the storing section 90 may be uploaded by communication means such as radio communication. The large database may perform calculation using the correlation equation to thereby calculate the initial band hole position α optimum for each individual. The device may acquire the initial band hole position α.

In the processing of optimum pressing force determination explained below, when second to Nth pressing force adjustment directions are a pressurizing direction (i.e., a direction in which the band hole position is tightened), the user may be instructed to set the initial band hole position α loose in advance.

Thereafter, the heart rate meter sets a variable n representing a band hole position to n=α (S1203). The variable n represents a band hole position recognized by the heart rate meter side and does not guarantee that an actual holding state of the holding mechanism 30 is a holding state by the band hole position corresponding to n. However, when the user follows an instruction on an instruction screen as explained below, the band hole position recognized by the system and the actual holding position correspond to (coincide) each other.

Subsequently, as shown in a lower part of Fig. 34B, the heart rate meter performs control for displaying an instruction screen for an instruction to the user such that the band hole position recognized on the system side and the actual holding state coincide with each other and, at the same time, receives an input from the user (S1204). In an example shown in Fig. 34B, the user attempts to hold the heart rate meter in the instructed band hole position. If the band can be tightened, the user inputs OK. If, for example, the band is too tight and cannot be tightened or the band is too loose and cannot be sufficiently fixed, the user inputs NG. However, the interaction with the user is not limited to this.

The heart rate meter receives the input of the user responding to the instruction in S1204 and determines whether OK is input (S1205). When it is determined that the OK is input (Yes in S1205), considering that the heart rate meter can be mounted in the band hole position instructed by the instruction screen, the heart rate meter shifts to processing based on the AC component signal and the DC component signal in that holding state (and a pressing force corresponding to the holding state). Specifically, the heart rate meter determines in S1206 to S1211 whether conditions for an environment for determination are satisfied. When the conditions for the environment for determination are satisfied, the heart rate meter performs actual processing in S1212 and S1213. The processing is specifically explained below.

First, as a condition for the environment for determination, the heart rate meter determines whether a body motion is stable (S1206). This is because, when the body motion is unstable, a component due to the body motion (body motion noise) is included in the AC component signal and proper pressing force determination is hindered. The processing in S1206 only has to be performed based on a body motion detection signal output from a body motion sensor. The body motion sensor is, for example, the acceleration sensor 21. The body motion detection signal is an acceleration detection value.

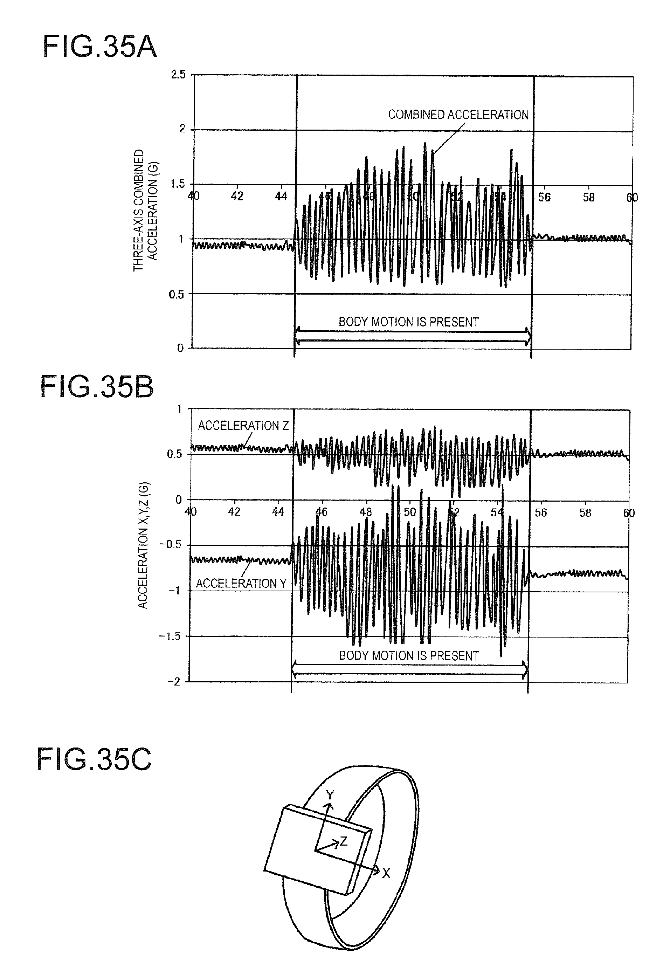

A specific example of the acceleration detection value is shown in Fig. 35B. As it is evident from Fig. 35B, compared with an acceleration detection value obtained when the body motion is absent, the magnitude of the acceleration detection value is extremely large when the body motion is present. Therefore, the heart rate meter only has to determine that the body motion is unstable when the acceleration detection value is large and determine that the body motion is stable when the acceleration detection value is small.

The acceleration sensor 21 is a three-axis acceleration sensor. Directions of axes of the acceleration sensor 21 are as shown in Fig. 35C. As shown in Fig. 35C, when a plane including a dial portion of a watch (in the heart rate meter, equivalent to the display section 70) is considered, a 12 o'clock direction of the watch included in the plane is a Y axis and a 3 o'clock direction of the watch included in the plane is an X axis. A Z axis is an axis in a direction orthogonal to the plane including the X axis and the Y axis and directed toward the wrist side of the test subject with respect to the dial portion. However, the directions of the axes are not limited to this.

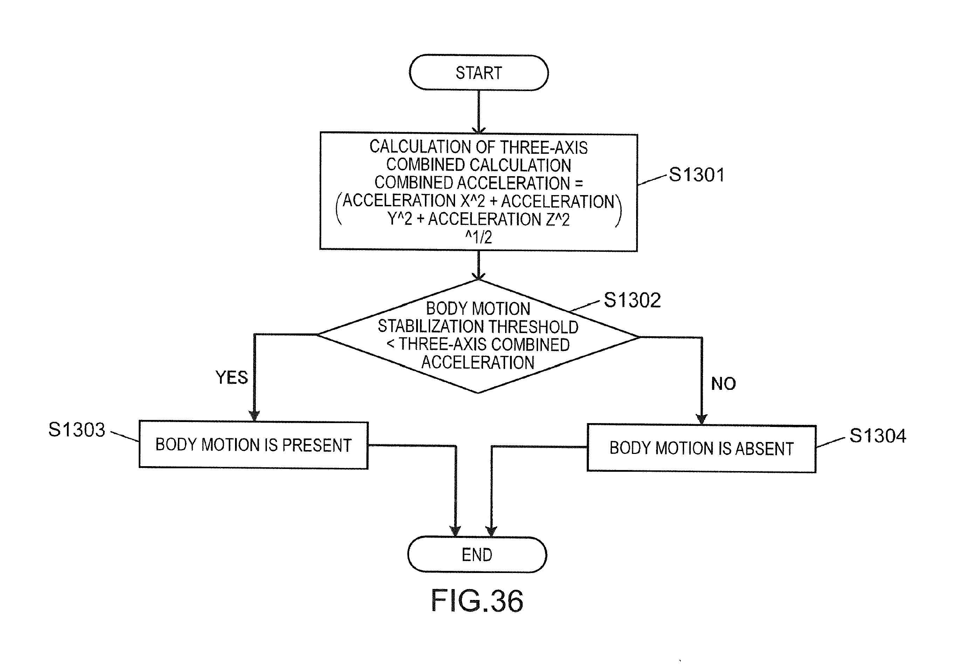

The heart rate meter calculates combined acceleration of the three axes shown in Fig. 35A and determines whether the body motion is stable on the basis of comparison processing for the magnitude of the calculated combined acceleration and a given threshold. A flowchart is shown in Fig. 36. The heart rate meter calculates a square root of a sum of squares of the axes as three-axis combined acceleration (S1301) and performs comparison processing for the calculated three-axis combined acceleration and a body motion stability threshold (S1302). When the three-axis combined acceleration is larger than the body motion stability threshold, the heart rate meter determines that the body motion is present (the body motion is unstable) (S1303). Otherwise, the heart rate meter determines that the body motion is absent (the body motion is stable) (S1304).

When it is determined by the processing that the body motion is unstable (NO in S1206), the heart rate meter waits for the body motion to stabilize. By instructing the user to stabilize the body motion from the system side, it is considered possible to efficiently realize the conditions for the environment for determination. Therefore, the heart rate meter performs control for displaying, on the display section 70, an instruction screen for instructing stabilization of the body motion (S1207).

When it is determined that the body motion is stable (Yes in S1206), as a second condition for the environment for determination, the heart rate meter determines whether a posture of the user is not abnormal (S1208). The posture indicates, in a narrow sense, a posture determined by a height relation between a mounted region (assumed to be the wrist) of the pulse wave sensor 11 and the heart. That is, since the magnitude of a water head pressure applied to a blood vessel changes when the posture changes, a blood flow rate changes. Therefore, the posture determination is performed as a condition for suppressing deterioration in accuracy of the proper pressing force determination due to the change in the blood flow rate.

As an example, a typical posture of the user in viewing the dial portion of the watch only has to be set as a reference posture. On the other hand, a posture in which the height relation between the wrist and the heart is substantially different only has to be set as an abnormal posture.

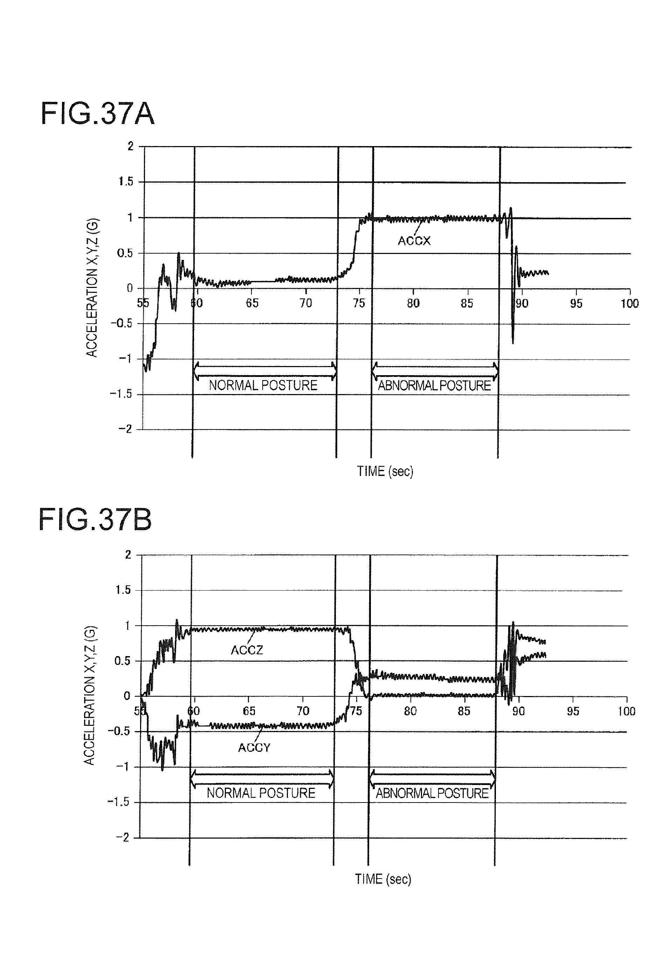

An example of the acceleration detection value in this case is shown in Figs. 37A and 37B. Figs. 37A and 37B are only shown as separate figures because of a problem of visibility of a graph. Values of the X axis, the Y axis, and the Z axis are acquired from the same acceleration sensor 21. Axes of the acceleration sensor 21 are the axes shown in Fig. 35C. Therefore, a typical range in the reference posture of acceleration detection values of the axes (which are mainly gravitational acceleration components acting on the axes because the acceleration detection values are based on the premise that the body motion is small) can be specified to a certain degree. For example, it is conceivable that the plane including the dial portion is close to the horizontal plane and the Z axis and the gravity direction are close to each other. In this case, values of the X axis and the Y axis are close to 0 and a value of the Z axis is close to 1 G. Therefore, proper ranges of values of the axes in the reference posture are set on the basis of these values. A posture is determined according to whether the acceleration detection values are within the proper ranges. As shown in Figs. 37A and 37B, in the abnormal posture, values of the axes deviate from the proper ranges.

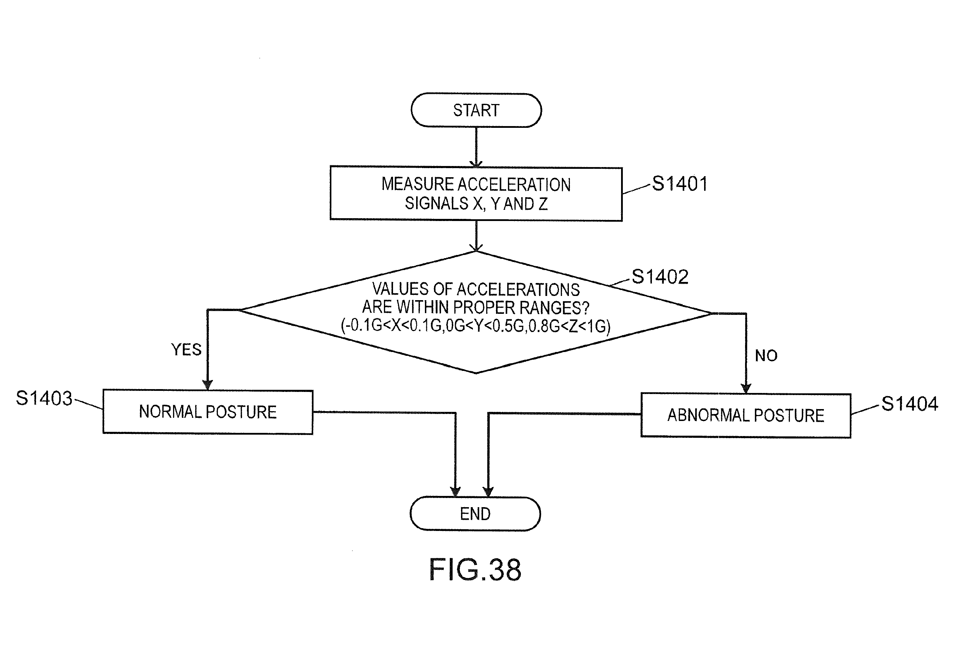

A flowchart is shown in Fig. 38. When this processing is started, first, the heart rate meter measures acceleration detection values in the X, Y, and Z axes (S1401). The heart rate meter performs comparison processing for the proper ranges of the axes set in advance and the acceleration detection values measured in S1401 (S1402). When all the acceleration detection values of the axes are within the proper ranges, the heart rate meter determines that the posture is the normal posture (S1403). Otherwise, the heart rate meter determines that the posture is the abnormal posture (S1404).

It is assumed that a portion above the elbow of the arm is kept in a state close to the horizontal. Therefore, the proper range of the X axis may be a narrow range including 0 G (e.g., as shown in Fig. 38, -0.1 to +0.1 G). On the other hand, it is also highly likely that an angle of the wrist is different depending on a person. It is desirable to set proper ranges wider than the proper range of the X axis as the proper ranges of the Y axis and the Z axis while setting 0 G and 1 G respectively as references for the Y axis and the Z axis. The proper ranges are not limited to the numerical values shown in S1402 in Fig. 38.

When it is determined by the processing that the posture is the abnormal posture (NO in S1208), the heart rate meter waits for the posture to be normal. In waiting for the posture to be normal, as in the stabilization of the body motion, the heart rate meter desirably performs display control for an instruction screen for instructing the user to take the normal posture (S1209). In this case, when convenience for the user or the like is taken into account, it is desirable to display the instruction screen that clearly shows what posture is the normal posture. The heart rate meter may instruct the user to take the normal posture using a sentence shown in S1209 in Fig. 33 or may instruct the user to take the normal posture by displaying a figure if there is no problem in the resolution or the like of the display section 70.

When it is determined that the posture is not the abnormal posture (YES in S1208), as a third condition for the environment for determination, the heart rate meter determines whether a fixed time has elapsed from the wearing of the band (S1210). When the holding mechanism 300 is realized by a band or the like including holes, for wearing by a given band hole, it is necessary to once fasten the band more tightly than during the wearing by the band hole. Therefore, as shown in Fig. 29A or Fig. 31, the AC component signal and the DC component signal during the wearing greatly fluctuate undesirably for the proper pressing force determination. Besides, a signal value is unstable for a fixed time thereafter. Therefore, in order to perform appropriate proper pressing force determination, it is desirable to exclude such an unstable signal value from the processing. Specifically, the heart rate meter stays on standby without performing the processing for a fixed time after the wearing and starts the processing after the elapse of the fixed time.

In S1210, the heart rate meter only has to determine whether the given time has elapsed on the basis of time measurement information from the time measuring section 140 realized by a timer or the like. When it is determined that the given time has not elapsed (NO in S1210), the heart rate meter performs control for displaying an instruction screen for instructing the user to stay on standby (S1211).

When it is determined that the given time has elapsed (YES in S1210), since the condition for the environment for determination is satisfied, the heart rate meter performs processing based on the AC component signal and processing based on the DC component signal (S1212 and S1213). In S1212, the heart rate meter performs processing for calculating an amplitude value of the AC component signal. In S1213, the heart rate meter performs processing for calculating a representative value (an average) of the DC component signal. Since the specific processing is explained above, detailed explanation of the processing is omitted.

After the processing in S1213 or when it is determined that OK is not input (NO in S1205), the heart rate meter increments n (S1214) and determines whether the processing in all the band holes ends (S1215). When it is determined that the processing does not end (NO in S1215), it is necessary to perform the processing in a band hole position equivalent to n incremented in S1214. Therefore, the heart rate meter returns to S1204, performs control for displaying an instruction screen for giving an instruction to the user such that a band hole position recognized on the system side and an actual holding state coincide with each other, and, at the same time, receives an input from the user. Subsequent processing is the same as the processing explained above.

When it is determined that the processing ends (YES in S1215), since the processing in all the band hole positions ends, the heart rate meter determines the proper pressing force on the basis of amplitude values of the AC component signal and representative values of the DC component signal calculated in the band hole positions (S1216). Specifically, the heart rate meter only has to perform, for example, the processing shown in the flowchart of Fig. 32.

The heart rate meter stores a band hole position corresponding to the proper pressing force (in a broad sense, holding state specifying information) and performs control for displaying, on the display section 70, a screen for notifying the user of the proper band hole position as shown in Fig. 34C (S1217).

In the second embodiment explained above, the heart rate meter includes, as shown in Fig. 5, the pulse-wave detecting section 10 including the pulse wave sensor 11 configured to output a pulse wave sensor signal, the processing section 100 configured to calculate pulsation information on the basis of the signal output from the pulse-wave detecting section 10, the display section 70 configured to display a processing result in the processing section 100, the storing section 90 configured to store personal information of a test subject and the processing result in the processing section 100, and the holding mechanism 300 (shown in Fig. 24A) configured to hold the heart rate meter on the test subject. The processing section 100 estimates a holding state of the holding mechanism optimum for the test subject on the basis of the personal information of the test subject stored in the storing section and determines whether a pressing force on the test subject in the pulse-wave detecting section 10 is the proper pressing force. The storing section 90 stores the holding state specifying information at the time when it is determined that the pressing force on the test subject in the pulse-wave detecting section 10 is the proper pressing force. The processing section 100 performs control for displaying the holding state specifying information on the display section 70.

The holding state specifying information is information for specifying a holding state in the holding mechanism (the load mechanism) 300.

For example, if the heart rate meter is the device held by the band as shown in Fig. 4A, the holding state specifying information is information for specifying a state of the band. Specifically, when the band is fixed using the holes as shown in Fig. 29, information indicating which band hole position is used to hold the heart rate meter is the holding state specifying information. The band is not limited to the band held using the holes. A band of a Velcro type having a scale or a band of a ratchet type may be used. The holding mechanism 300 is not limited to the band. An arbitrary mechanism can be used as long as the mechanism can fix the heart rate meter to the test subject and does not prevent acquisition of sensor information in the pulse wave sensor 11.

Consequently, after determining the proper pressing force in the heart rate meter, it is possible to store and display the holding state specifying information corresponding to the proper pressing force. Even if the proper pressing force can be calculated in a unit of kPa, mmHg, or the like, it is not easy to determine in which state the holding mechanism 300 is set in order to realize the proper pressing force. In this regard, by associating the proper pressing force and the holding state specifying information, the user does not need to be aware of a numerical value or the like of the proper pressing force. The user can recognize whether the pressing force is the proper pressing force according to an intuitive method such as a tightening state of the band. Therefore, after the determination of the proper pressing force is performed once, it is possible to easily reproduce, even when the device is mounted again, a state in which the proper pressing force is applied. It is possible to expect, for example, improvement of convenience for the user.

As explained above, the proper pressing force is pressure in a range larger than the pressing force corresponding to the vein vanishing point (a vein recovery point) and smaller than the pressing force corresponding to the artery vanishing point (an artery recovery point). That is, in the second embodiment explained above, the heart rate meter may include the pulse-wave detecting section 10 including the pulse wave sensor 11 configured to output a pulse wave sensor signal, the processing section 100 configured to calculate pulsation information on the basis of the signal output from the pulse-wave detecting section 10, the display section 70 configured to display a processing result of the processing section 100, the storing section 90 configured to store personal information of a test subject and a processing result in the processing section 100, and the holding mechanism 300 configured to hold the heart rate meter on a test subject. The processing section 100 may estimate a holding state of the holding mechanism optimum for the test subject on the basis of the personal information of the test subject stored in the storing section and determine whether a pressing force on the test subject in the pulse-wave detecting section 10 is included in the range larger than the pressing force corresponding to the vein vanishing point (the vein recovery point) and smaller than the pressing force corresponding to the artery vanishing point (the artery recovery point). The storing section 90 may store the holding state specifying information for specifying a holding state of the holding mechanism 300 at the time when it is determined that the pressing force on the test subject in the pulse-wave detecting section 100 is included in the range. The processing section 100 may perform control for displaying the holding state specifying information on the display section 70.

The processing section 100 may determine whether the pressing force is the proper pressing force on the basis of the pulse wave sensor signal.

Consequently, it is possible to perform the proper pressing force determination taking into account an individual difference. In the method in the past, since processing based on physical information such as a pressure value is performed, the individual difference is not taken into account. However, by using the pulse wave sensor signal, which is a vital sign having a large individual difference, it is possible to appropriately determine the proper pressing force for each of the users. It is also possible to deal with fluctuation in the proper pressing force based on a physical condition change or the like of the same user. In a state in which the proper pressing force determined by the method in the first embodiment is applied, by performing, for example, calculation of pulsation information, it is possible to, for example, improve accuracy of the calculated pulsation information.

The processing section 100 may determine whether the pressing force is the proper pressing force on the basis of one of the AC component signal corresponding to the AC component of the pulse wave sensor signal and the DC component signal corresponding to the DC component of the pulse wave sensor signal.

The AC component signal corresponds to a high-frequency component and the DC component signal corresponds to a low-frequency component. However, a frequency set as a reference for determining a level of the AC component signal and the DC component signal may be determined on the basis of, for example, the frequency of a pulsation. If it is taken into account that the AC component signal is also used for, for example, calculation of pulsation information, the AC component signal is required to include a component due to a heartbeat. When biological characteristics of the test subject are considered, it is possible to set a typical lower limit value of a pulse count to some degree. For example, it does not often occur that the pulse count is smaller than 30 per minute.

Therefore, it is considered to be rare that a signal due to the heartbeat is included in a frequency band equal to or lower than a frequency (0.5 Hz) corresponding to the pulse count of 30 per minute. Therefore, a frequency higher than the frequency may be set as the high-frequency component and a frequency lower than the frequency may be set as the low-frequency component.

Consequently, it is possible to perform the proper pressing force determination using at least one of the AC component signal and the DC component signal. In the AC component signal, a characteristic appears in an amplitude value. In the DC component signal, a characteristic appears in an inflection point.

The proper pressing force can be determined from one of the AC component signal and the DC component signal. However, it is possible to improve determination accuracy by using both the signals as shown in the flowchart of Fig. 32.

The processing section 100 may determine whether the pressing force is the proper pressing force on the basis of a characteristic of a change in the AC component signal that occurs when the pressing force is changed. Specifically, the processing section 100 may determine whether the pressing force is the proper pressing force on the basis of the characteristic of the change in the amplitude of the AC component signal that occurs when the pressing force is changed.

Consequently, it is possible to perform determination based on the change characteristic of the AC component signal with respect to the pressing force. The pulse wave sensor signal is a vital sign having a large individual difference. The AC component signal corresponding to the AC component of the pulse wave sensor signal also has a large individual difference. Therefore, since there is an individual difference in the magnitude of a signal value during the proper pressing force as well, it is difficult to determine, only from a signal value at a given pressing force, whether the pressing force is the proper pressing force. In this regard, if the change characteristic with respect to the pressing force is used, it is possible to perform appropriate determination not depending on the individual difference. As shown in Fig. 25A, since it is known that the AC component signal has a relatively large amplitude value at the proper pressing force, specifically, the determination only has to be performed on the basis of a change characteristic of the amplitude value.

The processing section 100 may determine whether the pressing force is the proper pressing force on the basis of a characteristic of a change in the DC component signal that occurs when the pressing force is changed. Specifically, the processing section 100 may detect an inflection point of the DC component signal on the basis of the characteristic of the change in the DC component signal that occurs when the pressing force is changed and determine whether the pressing force is the proper pressing force on the basis of the detected inflection point.

Consequently, it is possible to perform determination based on the change characteristic of the DC component signal with respect to the pressing force. The DC component signal represents the volume of a blood flow. In a pressurization (depressurization) process, since inflection points appear at the vein vanishing point (the vein recovery point) and the artery vanishing point (the artery recovery point), it is possible to perform accurate proper pressing force determination. Further, when the proper pressing force is calculated as a range, it is possible to accurately determine an upper limit value and a lower limit value of the proper pressing force.

The processing section 100 may perform control for displaying, on the display section 70, an instruction screen for performing a setting instruction for an environment for determining whether the pressing force is the proper pressing force.

The environment for determination means an environment that satisfies a condition that, for example, a body motion is stable, a posture is not abnormal, and a fixed time has elapsed after a holding state in the holding mechanism 300 is determined. Conditions other than the above may be added to the environment for determination. A part or all (when the other conditions are added) of the conditions may be excluded. Various settings are possible for contents of the environment for determination.

Consequently, it is possible to perform the setting instruction for the environment for determination. Unlike the method of determining a pressing force using a pressure value or the like in the past, in the second embodiment, since the determination is performed on the basis of the pulse wave sensor signal, which is the vital sign, a signal value also fluctuates because of a cause other than a change in the pressing force. It is difficult to specify or isolate the fluctuation cause only from the signal value of the pulse wave sensor signal. Therefore, it is necessary to suppress the fluctuation in the signal value due to the cause other than the pressing force as much as possible. In this explanation, an environment satisfying such a condition is set as the environment for determination and the user is instructed to satisfy the environment for determination. Therefore, it is possible to accurately perform the proper pressing force determination.

The processing section 100 may perform control for displaying, as an instruction screen, on the display section 70, a screen for instructing stabilization of the body motion of the test subject.

Consequently, it is possible to perform a stabilization instruction for the body motion. When the body motion is large, a component due to the body motion (body motion noise) is mixed in the pulse wave sensor signal. The body motion noise is superimposed on the AC component signal and the DC component signal calculated from the pulse wave sensor signal. Therefore, in order to suppress the influence of the body motion noise and perform accurate proper pressing force determination, it is desirable to attain stabilization of the body motion. The body motion is performed by the user and it is difficult for the heart rate meter to physically suppress the body motion of the user. Therefore, it is a realistic and effective method to instruct, in a simple form, the user to stabilize the body motion.

The processing section 100 may perform control for displaying, as an instruction screen, on the display section 70, a screen for causing the test subject to take a given posture for determination.

The posture for determination indicates a posture specified on the basis of a relative positional relation between the heart of the test subject and the pulse-wave detecting section 10. When the relative positional relation (specifically, height relation) between the heart of the test subject and the pulse-wave detecting section 10 fluctuates, a water head pressure fluctuates. For example, in a state in which the arm is lifted, compared with a state in which the arm is lowered, a blood flow rate decreases because of a change in the water head pressure. That is, the posture for determination is a posture set for suppressing the blood flow rate from fluctuating because of the water head pressure (independently from a pressing force change).

Consequently, it is possible to suppress the abnormal posture from being taken during the proper pressing force determination. Therefore, it is possible to suppress deterioration in accuracy of the proper pressing force determination due to the water head pressure. In the second embodiment based on the premise that the interaction on the display section 70 is performed, the posture for determination is preferably a posture for enabling the user to naturally view the display section 70 (e.g., a posture in viewing the dial portion of the wrist watch). However, the posture for determination is not limited to this. As the instruction to the user, as in the stabilization of the body motion, a method of displaying the instruction on the display section 70 is simple and effective.

The processing section 100 may perform control for displaying, as an instruction screen, on the display section 70, a screen for instructing the test subject to stay on standby for a given time.

Consequently, it is possible to accurately perform the proper pressing force determination by causing the user to stay on standby for the fixed time. This is because a certain degree of time is required when band adjustment is actually manually performed and a fixed time (about 10 seconds) is necessary until the signal value itself stabilizes as shown in Figs. 26A and 31.

The processing section 100 may determine whether the condition for the environment for determination displayed on the instruction screen is satisfied and, when it is determined that the condition is satisfied, determine whether the pressing force is the proper pressing force. Specifically, the processing section 100 determines whether the conditions for the environment for determination displayed on the instruction screen is satisfied on the basis of a body motion detection signal output from the body motion sensor or time measurement information output from the time measuring section 140.

Consequently, when the condition for the environment for determination is satisfied, it is possible to perform the proper pressing force determination. By displaying the instruction screen on the display section 70, it is possible to send an instruction to realize the environment for determination to the user. However, it is not guaranteed whether the user actually follows the instruction. Therefore, it is possible to attain, for example, improvement of determination accuracy by determining on the system side whether the condition is satisfied and, when the condition is satisfied, performing the processing. Concerning the stabilization of the body motion and the exclusion of the abnormal posture, the body motion sensor such as the acceleration sensor 21 only has to be used as shown in Figs. 35A, 37A, and 37B. Concerning the elapse of time, time measurement information output from the time measuring section 140 realized by a timer or the like only has to be used.

The processing section 100 may include, as processing modes, a holding state specifying information acquisition mode and a pulsation information calculation mode for calculating pulsation information. When the processing section 100 is set in the holding state specifying information acquisition mode, the processing section 100 acquires holding state specifying information on the basis of a determination result concerning whether the pressing force is the proper pressing force and stores the acquired holding state specifying information in the storing section 90. When the processing section 100 is set in the pulsation information calculation mode after the acquisition of the holding state specifying information, the processing section 100 may perform control for reading out the holding state specifying information stored in the storing section 90 and displaying the read-out holding state specifying information on the display section 70.

Consequently, after performing the proper pressing force determination and acquiring the holding state specifying information in the holding state specifying information acquisition mode, it is possible to display the acquired holding state specifying information on the display section 70 in the pulsation information calculation mode. Therefore, during calculation of pulsation information, it is possible to easily realize a holding state suitable for the calculation. Even if the proper pressing force is determined and the holding state specifying information at the proper pressing force is calculated, it is ineffective if a result of the calculation cannot be used for the calculation of the pulsation information, which is main processing of the heart rate meter. Therefore, if even the acquisition of the holding state specifying information, non-continuation of the calculation of the pulsation information, and the like are taken into account, it is necessary to once store the acquired holding state specifying information in the storing section 90. If the processing section 100 enters the pulsation information calculation mode, it is necessary to present the holding state specifying information to the user in a simple form.

The holding mechanism 300 may take, as the holding state, first to Nth (N is an integer equal to or larger than 2) states in which pressing forces on the test subject in the pulse-wave detecting section 10 are different. The processing section 100 acquires, based on a determination result concerning whether the pressing force in each of the first to Nth states is the proper pressing force, as the holding state specifying information, information corresponding to at least one of the first to Nth states.

Consequently, in the holding mechanism 300 (e.g., a band including holes) that can take a plurality of discrete holding states, it is possible to perform the processing and acquire, as the holding state specifying information of a result of the processing, at least one state (e.g., in which band hole position the band is held) among the plurality of holding states.

After determining whether a pressing force in an ith (i is an integer satisfying 1≤i≤N) state is the proper pressing force, the processing section 100 may perform control for displaying, on the display section 70, an instruction screen for performing an instruction to change the holding state in the holding mechanism 300 to a jth (j is an integer satisfying 1≤j≤N, j≠i) in which a pressing force is smaller than the pressing force in the ith state.

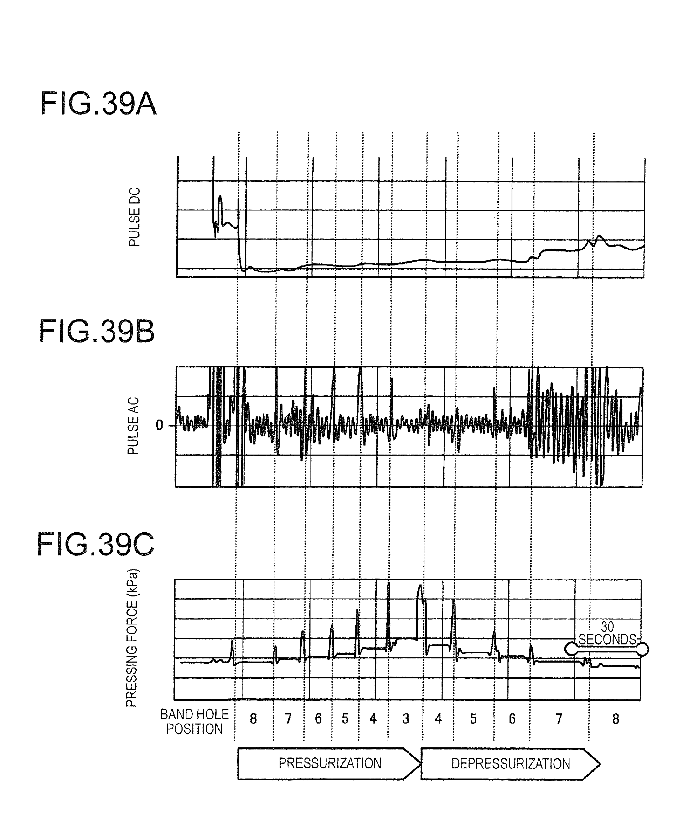

Consequently, it is possible to perform the proper pressing force determination in the depressurization process. An example is shown in Figs. 39A to 39C. As shown in Fig. 39C, pressurization is performed in the former half and depressurization is performed in the latter half. An extremely large pressing force appears during a change of a band hole position. A large signal value appears in the AC component signal corresponding to the pressing force. However, as shown in Fig. 26A and the like, the pressing force and the signal value are excluded in the proper pressing force determination.

As shown in Fig. 39B, in the AC component signal, large amplitude appears before and after a band hole position 7 in both of the pressurization process and the depressurization process. Therefore, the determination of the proper pressing force is possible. However, since an amplitude value is larger in the depressurization process, the determination is considered to be easy.

On the other hand, as shown in Fig. 39A, whereas an inflection point of the DC component signal is clear in the depressurization process, the inflection point is unclear in the pressurization process. If even small fluctuation of a signal value is taken into account, it is possible to detect the inflection point of the DC component signal in the pressurization process. However, the determination is easier in the depressurization process as in the AC component signal.

Consequently, by performing the proper pressing force determination in the depressurization process, it is possible to accurately perform the determination compared with the determination performed using the pressurization process.

The second embodiment explained above can be applied to a heart rate meter including the pulse-wave detecting section 10 including the pulse wave sensor 11, the processing section 100 configured to determine whether a pressing force on a test subject in the pulse-wave detecting section 10 is a proper pressing force and calculate pulsation information of the test subject on the basis of a signal output from the pulse-wave detecting section 10, the display section 70 configured to display a processing result in the processing section 100, and the storing section 90 configured to store the processing result in the processing section 100. The processing section 100 performs control for displaying, on the display section 70, an instruction screen for performing a setting instruction for an environment for determining whether the pressing force is the proper pressing force.

Consequently, even when the pressing force is changed by a method other than holding by the holding mechanism 300, by displaying the instruction screen for the environment for determination, it is possible to perform the pressing force determination in an appropriate situation. Irrespective of what kind of method is adopted as a method of a pressing force change, since it is undesirable that a signal value used for the proper pressing force determination fluctuates because of a cause other than the pressing force change, setting of the environment for determination is important. There is a significant advantage in displaying, on the display section 70, the instruction screen for performing the setting instruction for the environment for determination.

The processing section 100 may determine whether a condition for the environment for determination displayed on the instruction surface is satisfied and determine whether the pressing force is the proper pressing force on the basis of a signal output from the pulse-wave detecting section 10 when it is determined when the condition is satisfied.

Consequently, it is possible to perform the proper pressing force determination based on a signal (in a narrow sense, a pulse wave sensor signal and, in a narrower sense, at least one of an AC component signal and a DC component signal) output when the condition for the environment for determination is satisfied. Advantages in determining on the system side whether the condition for the environment for determination is satisfied and using the signal output from the pulse-wave detecting section 10 for the proper pressing force determination are as explained above.

A part or most of the processing of the biological information detecting devices (or the electronic device SP and the server system SE shown in Fig. 16) in the first embodiment and the second embodiment may be realized by a computer program. In this case, a processor such as a CPU executes the computer program, whereby the biological information detecting device and the like in the first embodiment are realized. Specifically, the computer program stored in a non-transitory information storage medium is read out and the processor such as the CPU executes the read-out computer program. The information storage medium (a computer-readable medium) is a medium for storing computer programs, data, and the like. The function of the information storage medium can be realized by an optical disk (a DVD, a CD, etc.), an HDD (hard disk drive), a memory (a card type memory, a ROM, etc.), or the like. The processor such as the CPU can perform the various kinds of processing in the first embodiment on the basis of a computer program (data) stored in the information storage medium. That is, in the information storage medium, a computer program for causing a computer (an apparatus including an operation section, a processing section, a storing section, and an output section) to function as the sections in the first embodiment (a computer program for causing the computer to execute the processing of the sections) is stored.

The first embodiment and the second embodiment are explained in detail above. However, those skilled in the art could easily understand that various modifications are possible without substantively departing from the new matters and the effects of the invention. Therefore, all such modifications are regarded as being included in the scope of the invention. For example, the terms described at least once together with broader or synonymous different terms in the specification or the drawings can be replaced with the different terms in any place in the specification or the drawings. The configurations and the operations of the biological information detecting device and the like are not limited to those explained in the first embodiment. Various modifications of the configurations and the operations are possible.

## Claims

1. A biological information detecting device comprising:
a pulse-wave-information detecting section configured to detect pulse wave information of a test subject;
a display section; and
a processing section configured to perform measurement processing for the pulse wave information and discrimination processing for a posture state of the test subject, wherein
the display section displays a posture state notification image that dynamically changes according to a change in the posture state, and
the processing section performs processing for determining whether the posture state of the test subject is appropriate as a posture for performing the measurement processing for the pulse wave information and, when determining that the posture state is appropriate, performs the measurement processing for the pulse wave information.

2. The biological information detecting device according to claim 1, wherein the display section displays, as the posture state notification information, an image in which a display position of an object representing the posture state changes according to the posture state.

3. The biological information detecting device according to claim 1 or 2, wherein, when the processing section determines that the posture state is appropriate, the display section displays, as the posture state notification image, an image in which the object is displayed in a first region in the image.

4. The biological information detecting device according to any one of the preceding claims, wherein, when the biological information detecting device is mounted on the test subject, a direction from the forearm to the hand of the test subject is represented as X axis, a direction orthogonal to the X axis and orthogonal to a screen of the display section is represented as Z axis, an axis orthogonal to the X axis and the Z axis is represented as Y axis, an angle representing rotation of the X axis around the Y axis during the discrimination processing with respect to a horizontal plane orthogonal to a gravity direction is represented as first angle θx, and an angle representing rotation of the Y axis around the X axis during the discrimination processing with respect to the horizontal plane is represented as second angle θy, the processing section determines that the posture state is appropriate when it is determined that the first angle θx is within a first angle range and the second angle θy is within a second angle range.

5. The biological information detecting device according to claim 4, wherein, concerning the second angle θy, when an angle representing rotation on the gravity direction side with respect to the horizontal plane is set as a positive value and an angle representing rotation on an opposite side to the gravity direction is set as a negative value, the processing section determines that the posture state is appropriate when it is determined that the second angle θy is within the second angle range in which a median is a negative value.

6. The biological information detecting device according to claim 3, 4 or 5, wherein, when the processing section determines that the posture state is appropriate, the display section continuously displays, as the posture state notification information, for a given wait time, an image in which the object is displayed in the first region.

7. The biological information detecting device according to claim 6, wherein the display section displays, after elapse of the wait time, a measurement state notification image representing that the processing section is performing the measurement processing for the pulse wave information.

8. The biological information detecting device according to claim 6 or 7, wherein
when the processing section determines that the posture state is inappropriate during the display of the measurement state notification image after the elapse of the wait time,
the processing section ends the measurement processing for the pulse wave information, and
the display section ends the display of the measurement state notification image and performs the display of the posture state notification image.

9. The biological information detecting device according to any one of claims 3 to 8, wherein, when the processing section determines that the posture state is inappropriate, the display section displays, as the posture state notification image, an image in which the object is displayed in a second region different from the first region in the image and displays, as the posture state notification image, an image in which a display form of the object is different when the object is displayed in the first region and when the object is displayed in the second region.

10. The biological information detecting device according to any one of the preceding claims, further comprising a load mechanism configured to generate a pressing force for pressing the pulse-wave-information detecting section against the test subject, wherein
a load state of the load mechanism is set to any one of first to Nth (N is an integer equal to or larger than 2) load states in which states of the pressing force are different,
the processing section performs the discrimination processing for the posture state in an ith (i is an integer satisfying 1≤i≤N) load state of the load mechanism, and
the display section displays the posture state notification image that dynamically changes according to a change in the posture state in the ith load state.

11. The biological information detecting device according to claim 10, wherein,
when the processing section determines that the posture state is appropriate as a result of the discrimination processing,
the processing section performs the measurement processing for the pulse wave information in the ith load state, and
the display section displays, after an end of the measurement processing in the processing section, an instruction image for performing an instruction to change the load mechanism to a jth (j is an integer satisfying 1≤j≤N, i≠j) load state different from the ith load state.

12. The biological information detecting device according to claim 10 or 11, wherein the processing section performs, on the basis of results of the measurement processing for the pulse wave information acquired in at least two load states among the first to Nth load states, proper pressing force information acquisition processing for calculating proper pressing force information representing a proper value of the pressing force on the test subject.

13. The biological information detecting device according to any one of the preceding claims, wherein
the processing section performs measurement processing for a level of the pulse wave information at time when a pressing force of the pulse-wave-information detecting section on the test subject is each of a first pressing state to an Nth (N is an integer equal to or larger than 2) pressing state, and
the display section displays a first measurement result to an Mth (M is an integer satisfying M≤N) measurement result among results of the measurement processing for the level of the pulse wave information in the first pressing state to the Nth pressing state.

14. The biological information detecting device according to claim 13, wherein the display section displays the first measurement result to the Mth measurement result subjected to normalization processing for the level of the pulse wave information.

15. The biological information detecting device according to claim 13 or 14, wherein the display section displays, with a maximum value of the level among the first measurement result to the Mth measurement result, the first measurement result to the Mth measurement result subjected to the normalization processing.

16. The biological information detecting device according to any one of claims 13 to 15, wherein
when results of the measurement processing for the level of the pulse wave information in the first pressing state to an ith (i is an integer satisfying 2≤i≤N) pressing state are acquired and results of the measurement processing for the level of the pulse wave information in an i+1th pressing state to the Nth pressing state are not acquired yet, the display section performs the normalization processing with a maximum value of the level among the first measurement result to a jth (j is an integer satisfying j≤i) measurement result included in the results of the measurement processing in the first pressing state to the ith pressing state and displays the first measurement result to the jth measurement result subjected to the normalization processing.

17. The biological information detecting device according to any one of claims 13 to 16, wherein the display section displays an instruction screen for performing an instruction to change a pressing state to the i+1th pressing state and, when the processing section starts the measurement processing for the level of the pulse wave information in the i+1th pressing state, displays a provisional measurement result of the level of the pulse wave information in the i+1th pressing state during the measurement processing together with the first measurement result to the jth measurement result.

18. The biological information detecting device according to claim 17, wherein the display section performs, as the display of the provisional measurement result, animation display that fluctuates in response to fluctuation in the pulse wave information in the i+1th pressing state.

19. The biological information detecting device according to any one of claims 13 to 18, wherein
the processing section performs, in each of the first pressing state to the Nth pressing state, discrimination processing for determining whether the posture state of the test subject is appropriate as a posture for performing the measurement processing for the pulse wave information, and
the display section displays, in each of the first pressing state to the Nth pressing state, the posture state notification image that dynamically changes according to a change in the posture state.

20. The biological information detecting device according to claim 19, wherein the display section displays, as the posture state notification image, an image in which a display position of an object representing the posture state changes according to the posture state.

21. The biological information detecting device according to any one of claims 13 to 20, wherein
when results of the measurement processing in the first pressing state to an ith (i is an integer satisfying 2≤i≤N) pressing state are acquired, results of the measurement processing in an i+1th pressing state to the Nth pressing state are not acquired yet, and the processing section determines that the posture state is appropriate in the i+1th pressing state,
the processing section starts the measurement processing for the level of the pulse wave information in the i+1th pressing state, and
the display section displays the first measurement result to a jth (j is an integer satisfying j≤i) measurement result included in results of the measurement processing in the first pressing state to the ith pressing state and a provisional measurement result of the level of the pulse wave information in the i+1th pressing state during the measurement processing.

22. The biological information detecting device according to any one of claims 13 to 21, wherein, when the processing section determines that the posture state is appropriate in the i+1th pressing state, the display section continuously displays, as the posture state notification image, for a given wait time, an image in which the object is displayed in a first region in the image corresponding to a state in which the posture state is appropriate.

23. The biological information detecting device according to any one of claims 13 to 22, wherein the display section displays, after elapse of the wait time, the first measurement result to the jth measurement result and the provisional measurement result in the i+1th pressing state.

24. The biological information detecting device according to claim 21, wherein
when the processing section acquires a result of the measurement processing in the i+1th pressing state, the display section displays an instruction screen for performing an instruction to change the pressing state to an i+2th pressing state, and
when the pressing state is changed to the i+2th pressing state according to the instruction screen, the display section displays the posture state notification image in the i+2th pressing state.

25. The biological information detecting device according to any one of claims 13 to 24, wherein the processing section performs, on the basis of results of the measurement processing of the pulse wave information acquired in at least two pressing states among the first pressing state to the Nth pressing state, proper pressing force information acquisition processing for calculating proper pressing force information representing a proper value of the pressing force on the test subject.

26. The biological information detecting device according to any one of the preceding claims, wherein the first measurement result to the Mth measurement result include at least a measurement result in which the level is a maximum among results of the measurement processing for the level of the pulse wave information in the first pressing state to the Nth pressing state.

27. A computer program for causing a computer to function as:
a pulse-wave-information detecting section configured to acquire pulse wave information of a test subject;
a processing section configured to perform measurement processing for the pulse wave information and discrimination processing for a posture state of the test subject; and
a display control section configured to perform control for displaying, on a display section, a posture state notification image that dynamically changes according to a change in the posture state, wherein
the processing section performs processing for determining whether the posture state of the test subject is appropriate as a posture for performing the measurement processing for the pulse wave information and, when determining that the posture state is appropriate, performs the measurement processing for the pulse wave information.

28. A computer program for causing a computer to function as:
a pulse-wave-information detecting section configured to acquire pulse wave information of a test subject;
a processing section configured to perform measurement processing for a level of the pulse wave information at time when a pressing force of the pulse-wave-information detecting section on the test subject is in each of a first pressing state to an Nth (N is an integer equal to or larger than 2) pressing state; and
a display control section configured to perform control for displaying, on a display section, a first measurement result to a Mth (M is an integer satisfying M≤N) measurement result among results of the measurement processing for the level of the pulse wave information in the first pressing state to the Nth pressing state.

29. A biological information detecting device comprising:
a pulse-wave-information detecting section configured to detect pulse wave information of a test subject;
a presenting section configured to present information; and
a processing section configured to perform measurement processing for the pulse wave information and discrimination processing for a posture state of the test subject, wherein
the presenting section presents the information concerning the posture state by taking a different presentation form according to a change in the posture state, and
the processing section performs processing for determining whether the posture state of the test subject is appropriate as a posture for performing the measurement processing for the pulse wave information and, when determining that the posture state is appropriate, performs the measurement processing for the pulse wave information.

30. A heart rate meter comprising:
a pulse-wave detecting section including a pulse wave sensor configured to output a pulse wave sensor signal;
a processing section configured to calculate pulsation information on the basis of the signal output from the pulse-wave detecting section;
a display section configured to display a processing result in the processing section;
a storing section configured to store individual information of a test subject and the processing result in the processing section; and
a holding mechanism for holding the heart rate meter on the test subject, wherein
the processing section estimates a holding state of the holding mechanism optimum for the test subject on the basis of the individual information of the test subject stored in the storing section and determines whether a pressing force on the test subject in the pulse-wave detecting section is a proper pressing force,
the storing section stores holding state specifying information for specifying a holding state of the holding mechanism at time when the processing section determines that the pressing force on the test subject in the pulse-wave detecting section is the proper pressing force, and
the processing section performs control for displaying the holding state specifying information on the display section.

31. The heart rate meter according to claim 30, wherein the processing section determines whether the pressing force is the proper pressing force on the basis of the pulse wave sensor signal.

32. The heart rate meter according to claim 30 or 31, wherein the processing section determines whether the pressing force is the proper pressing force on the basis of at least one of an AC component signal corresponding to an AC component of the pulse wave sensor signal and a DC component signal corresponding to a DC component of the pulse wave sensor signal.

33. A heart rate meter comprising:
a pulse-wave detecting section including a pulse wave sensor;
a processing section configured to determine whether a pressing force on a test subject in the pulse-wave detecting section is a proper pressing force and calculate pulsation information of the test subject on the basis of a signal output from the pulse-wave detecting section;
a display section configured to display a processing result in the processing section; and
a storing section configured to store individual information of the test subject and the processing result in the processing section, wherein
the processing section estimates a holding state of a holding mechanism optimum for the test subject on the basis of the individual information of the test subject stored in the storing section and performs control for displaying, on the display section, an instruction screen for performing a setting instruction for an environment for determining whether the pressing force is the proper pressing force.

34. A computer program for causing a computer to function as:
a pulse-wave detecting section including a pulse wave sensor configured to output a pulse wave sensor signal;
a processing section configured to calculate pulsation information on the basis of the signal output from the pulse-wave detecting section;
a display section configured to display a processing result in the processing section; and
a storing section configured to store individual information of a test subject and the processing result in the processing section, wherein
the processing section estimates a holding state of a holding mechanism optimum for the test subject on the basis of the individual information of the test subject stored in the storing section and determines whether a pressing force on the test subject in the pulse-wave detecting section is a proper pressing force,
the storing section stores holding state specifying information for specifying a holding state of the holding mechanism, which holds a heart rate meter on the test subject, at time when the processing section determines that the pressing force on the test subject in the pulse-wave detecting section is the proper pressing force, and
the processing section performs control for displaying the holding state specifying information on the display section.

35. A computer program for causing a computer to function as:
a pulse-wave detecting section including a pulse wave sensor;
a processing section configured to determine whether a pressing force on a test subject in the pulse-wave detecting section is a proper pressing force and calculate pulsation information of the test subject on the basis of a signal output from the pulse-wave detecting section;
a display section configured to display a processing result in the processing section; and
a storing section configured to store individual information of the test subject and the processing result in the processing section, wherein
the processing section estimates a holding state of a holding mechanism optimum for the test subject on the basis of the individual information of the test subject stored in the storing section and performs control for displaying, on the display section, an instruction screen for performing a setting instruction for an environment for determining whether the pressing force is the proper pressing force.
